(19) Europäisches Patentamt
European Patent Office
Office européen des brevets

(11) Veröffentlichungsnummer: **0 288 810 B1**

(12) **EUROPÄISCHE PATENTSCHRIFT**

(45) Veröffentlichungstag der Patentschrift: **02.12.92**

(51) Int. Cl.[5]: **C07D 213/64**, C07D 213/61, C07D 213/65, C07D 213/70, C07D 239/34, C07D 237/14, C07D 237/18, C07D 241/18, A01N 43/40, A01N 43/54, A01N 43/58

(21) Anmeldenummer: **88105711.1**

(22) Anmeldetag: **11.04.88**

Die Akte enthält technische Angaben, die nach dem Eingang der Anmeldung eingereicht wurden und die nicht in dieser Patentschrift enthalten sind.

(54) **Heterocyclische Neophananaloga, Verfahren zu ihrer Herstellung und ihre Verwendung als Schädlingsbekämpfungsmittel.**

(30) Priorität: **15.04.87 DE 3712752**

(43) Veröffentlichungstag der Anmeldung:
**02.11.88 Patentblatt 88/44**

(45) Bekanntmachung des Hinweises auf die Patenterteilung:
**02.12.92 Patentblatt 92/49**

(84) Benannte Vertragsstaaten:
**AT BE CH DE ES FR GB GR IT LI NL**

(56) Entgegenhaltungen:
**GB-A- 2 130 208**
**GB-A- 2 140 010**
**US-A- 3 188 315**

(73) Patentinhaber: **HOECHST AKTIENGESELL-SCHAFT**
**Postfach 80 03 20**
**W-6230 Frankfurt am Main 80(DE)**

(72) Erfinder: **Schubert, Hans Herbert, Dr.**
**Geisenheimer Strasse 95**
**W-6000 Frankfurt am Main 71(DE)**
Erfinder: **Salbeck, Gerhard, Dr.**
**Frankfurter Strasse 34**
**W-6238 Hofheim am Taunus(DE)**
Erfinder: **Krause, Hans-Peter, Dr.**
**Berliner Strasse 10**
**W-6238 Hofheim am Taunus(DE)**
Erfinder: **Lüders, Walter, Dr.**
**Feldbergstrasse 16**
**W-6056 Heusenstamm(DE)**
Erfinder: **Knauf, Werner, Dr.**
**Im Kirschgarten 24**
**W-6239 Eppstein/Taunus(DE)**
Erfinder: **Waltersdorfer, Anna, Dr.**
**Rauenthaler Weg 28**
**W-6000 Frankfurt am Main 71(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

Erfinder: **Kern, Manfred, Dr.**
**Im Traminer Weg 8**
**W-6501 Lörzweiler(DE)**

## Beschreibung

Die insektizide Wirkung von bestimmten Benzyl-neophylether-Verbindungen und deren Kohlenwasserstoffanaloga ist aus DE-A-31 17 510 und DE-A-33 17 908 bekannt.

Es wurden neue heterocyclische Neophan-Verbindungen mit hervorragenden insektiziden, akariziden und nematoziden Eigenschaften gefunden, die in ihren Anwendungseigenschaften verschiedenen herkömmlichen Präparaten deutlich überlegen sind.

Gegenstand der vorliegenden Erfindung sind daher die Verbindungen der Formel (I) und deren Stereoisomere

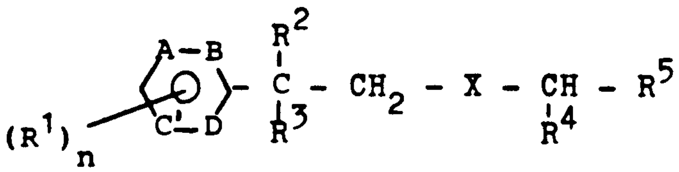 (I),

worin

A,B,C',D = unabhängig voneinander CH oder N, wobei mindestens eines der Symbole A,B,C',D einem Stickstoffatom entsprechen muß,

X = $CH_2$ oder Sauerstoff,

$R^1$ = ein an ein C-Atom gebundener Rest, der H,($C_1$-$C_4$)-Alkyl, Tri($C_1$-$C_4$)alkylsilyl, Halogen, Nitro, Cyano, ($C_2$-$C_6$)Alkenyl, ($C_2$-$C_6$)-Alkinyl, Amino, ($C_3$-$C_7$)Cycloalkyl, Phenyl, Phenoxy, ($C_1$-$C_5$)Alkoxy, ($C_2$-$C_4$)-Alkenyloxy, ($C_2$-$C_4$)Alkinyloxy, Hydroxycarbonyl, ($C_1$-$C_4$)-Alkylthio, ($C_3$-$C_7$)Cycloalkyloxy, ($C_1$-$C_6$)Alkylcarbonyl, ($C_1$-$C_4$)Alkoxycarbonyl, ($C_2$-$C_4$)-Alkenyloxycarbonyl, ($C_3$-$C_5$)Alkinyloxycarbonyl, ($C_1$-$C_4$)-Halogenalkyl, ($C_1$-$C_4$)Alkoxy($C_1$-$C_4$)alkyl, ($C_1$-$C_3$)Halogenalkoxy, ($C_1$-$C_3$)Halogenalkylthio, Halogen($C_1$-$C_4$)alkoxy($C_1$-$C_4$)-alkyl, ($C_1$-$C_4$)-Alkylthio($C_1$-$C_4$)alkyl, ($C_1$-$C_4$)Alkoxy($C_1$-$C_4$)alkoxy, Halogen($C_1$-$C_4$)alkoxy-($C_1$-$C_4$)alkoxy, ($C_2$-$C_4$)Alkenyloxy($C_1$-$C_4$)alkoxy, Halogen($C_2$-$C_4$)alkenyloxy, ($C_1$-$C_4$)-Alkoxy($C_1$-$C_4$)alkylthio, ($C_1$-$C_4$)Alkylthio($C_1$-$C_4$)alkoxy, ($C_1$-$C_4$)Alkylthio($C_1$-$C_4$)alkylthio, Halogen($C_1$-$C_4$)alkoxycarbonyl, Halogen($C_2$-$C_4$)alkenyloxycarbonyl oder Di($C_1$-$C_6$alkyl)-amino oder zwei Reste $R^1$ wenn sie ortho ständig zueinander orientiert sind zusammen einen Methylendioxy-, Ethylendioxy- oder ($C_3$-$C_5$)Alkylenrest,

$R^2$,$R^3$ = unabhängig voneinander ($C_1$-$C_3$)Alkyl, ($C_2$-$C_8$)Alkenyl, Phenyl oder $R^2$ und $R^3$ eine Alkylenkette, die - zusammen mit dem quartären Kohlenstoffatom einen unsubstituierten oder fluor-substituierten Ring mit drei bis sechs Ringgliedern ergibt,

$R^4$ = -H, F, $-CCl_3$, -C≡CH, ($C_1$-$C_4$)Alkyl ,

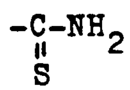,

$R^5$ = 2-Pyridy, Thienyl, die alle substituiert sind, oder substituiertes Phenyl.

$R^1$ bedeutet bevorzugt Wasserstoff, Halogen, ($C_1$-$C_4$)Alkyl, ($C_2$-$C_6$)-Alkenyl, ($C_3$-$C_7$)Cycloalkyl, ($C_1$-$C_5$)-Alkoxy, ($C_2$-$C_4$)Alkenyloxy, ($C_1$-$C_4$)Alkylthio, ($C_1$-$C_4$)Halogenalkyl, ($C_1$-$C_3$)Halogenalkoxy oder - 2 Reste $R^1$ zusammen- Methylendioxy, wobei $R^1$ insbesondere in 3- oder 4-Stellung des heterocyclischen Restes orientiert ist.

$R^2$ und $R^3$ stehen bevorzugt für einen ($C_1$-$C_3$)Alkylrest wie Methyl, Ethyl, i-Propyl und n-Propyl oder bedeuten bevorzugt zusammen mit dem sie verknüpfenden Kohlenstoffatom einen unsubstituierten oder mono- oder difluorierten Cyclopropylring.

$R^4$ steht bevorzugt für Wasserstoff, Fluor oder ($C_1$-$C_4$)-Alkyl, besonders bevorzugt für Wasserstoff.

Als substituiertes Phenyl $R^5$ steht ein Phenylrest der Formel (A),

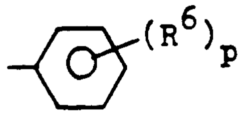

(A)

worin $R^6$ unabhängig voneinander H, Halogen, $(C_1$-$C_4)$Alkyl, $(C_1$-$C_4)$Alkoxy, $(C_1$-$C_4)$Halogenalkyl, Phenyl oder N-Pyrrolyl oder einen Rest der Formel (B) bedeutet,

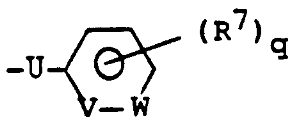

(B)

worin $R^7$ unabhängig voneinander H, Halogen, $(C_1$-$C_4)$Alkyl, $(C_1$-$C_4)$Alkoxy oder $(C_1$-$C_4)$Halogenalkyl; U = $-CH_2-$, $>C=O$, -O- oder -S-, bevorzugt -O-; V, W = CH oder N, wobei für V = N, W = CH bedeuten muß und vice versa,

p = eine ganze Zahl von 1 bis 5, insbesondere 1, 2 oder im Falle $R^6$ = Fluor 5 und falls $R^6$ der Gruppierung (B) entspricht, insbesondere 1 oder 2 und

q = 1 oder 2 bedeutet.

Von diesen Resten für $R^5$ sind von besonderer Bedeutung Reste der Formel (A), worin $R^6$ = H oder 4-Fluor und zusätzlich einen Rest der Formel (B) bedeutet, der in 3-Stellung des Phenylrestes von (A) orientiert ist.

Für substituiertes Pyridyl $R^5$ steht eine mono- oder disubstituierte Pyridyl-Gruppe der allgemeinen Formel (C),

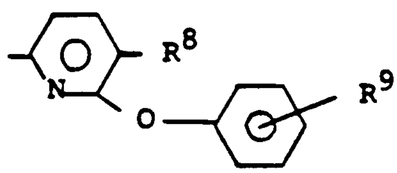

(C)

worin $R^8$ Halogen, insbesondere Fluor, oder H und $R^9$ = Wasserstoff, Halogen außer Jod, $(C_1$-$C_4)$Alkyl, $(C_1$-$C_4)$Alkoxy oder $(C_1$-$C_4)$Halogenalkyl bedeutet.

Für substituiertes Thienyl $R^5$ steht ein Heterocyclus der allgemeinen Formel (D),

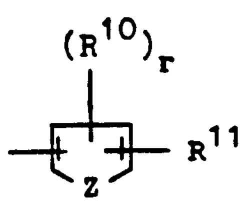

(D)

worin

Z = S,

$R^{10}$ = H, Halogen, $(C_1$-$C_4)$Alkyl, $(C_1$-$C_4)$Alkoxy, $(C_1$-$C_4)$Halogenalkyl, CN oder $NO_2$,

$R^{11}$ = gegebenenfalls substituiertes Benzyl, Propargyl, Allyl oder Phenoxy und

r = 1 oder 2

bedeutet.

Substituierte Phenylreste für $R^5$ sind für die Erfindung von besonderer Wichtigkeit.

Als typische Beispiele für die Gruppe $R^5$ werden folgende Reste angegeben:
Pentafluorphenyl, 5-Benzyl-3-furyl, 4-Phenoxyphenyl, 3-Phenoxyphenyl, 3-(4-Fluorphenoxy)phenyl, 3-(4-Chlorphenoxy)phenyl, 3-(4-Bromphenoxy)phenyl, 3-(3-Fluorphenoxy)phenyl, 3-(3-Chlorphenoxy)phenyl, 3-(3-Bromphenoxy)phenyl, 3-(2-Fluorphenoxy)phenyl, 3-(2-Chlorphenoxy)phenyl, 3-(2-Bromphenoxy)phenyl, 3-(4-Methylphenoxy)phenyl, 3-(3-Methylphenoxy)phenyl, 3-(2-Methylphenoxy)phenyl, 3-(4-Methoxyphenoxy)-phenyl, 3-(3-Methoxyphenoxy)phenyl, 3-(2-Methoxyphenoxy)phenyl, 3-(4-Ethoxyphenoxy)phenyl, 3-(Phenylthio)phenyl, 3-(4-Fluorphenylthio)phenyl, 3-(3-Fluorphenylthio)phenyl, 3-Benzoylphenyl, 3-Benzylphenyl, 3-(4-Fluorbenzyl)phenyl, 3-(4-Chlorbenzyl)phenyl, 3-(3,5-Dichlorphenoxy)phenyl 3-(3,4-Dichlorphenoxy)-phenyl, 3-(4-Chlor-2-methylphenoxy)phenyl, 3-(2-Chlor-5-methylphenoxy)phenyl, 3-(4-Chlor-5-methylphenoxy)phenyl, 3-(4-Ethylphenoxy)phenyl, 3-(3-Chlor-5-methoxyphenoxy)phenyl, 3-(2,5-Dichlorphenoxy)-phenyl, 3-(3,5-Dichlorbenzoyl)phenyl, 3-(3,4-Dichlorbenzoyl)phenyl, 3-(4-Methylbenzyl)phenyl, 3-(4-Isopropoxyphenoxy)phenyl, 4-Fluor-3-phenoxyphenyl, 4-Chlor-3-phenoxyphenyl, 4-Brom-3-phenoxyphenyl, 4-Fluor-3-(4-fluorphenoxy)phenyl, 4-Fluor-3-'(4-chlorphenoxy) = phenyl, 4-Fluor-3-(4-bromphenoxy)phenyl, 4-Fluor-3-(4-methylphenoxy)phenyl, 4-Fluor-3-(4-methoxyphenoxy)phenyl, 4-Fluor-3-(3-fluorphenoxy)phenyl, 4-Fluor-3-(3-chlorphenoxy)phenyl, 4-Fluor-3-(3-brompheoxy)phenyl, 4-Fluor3-(3-methoxyphenoxy)phenyl, 4-Fluor-3-(4-ethoxyphenoxy) = phenyl, 4-Fluor-3-(2-fluorphenoxy)phenyl, 3-Methoxy-5-phenoxyphenyl, 2-Fluor-3-phenoxyphenyl, 2-Fluor-3-(4-fluorphenoxy)phenyl, 2-Fluor-3-(3-fluorphenoxy)phenyl, 2-Fluor-3-(2-fluorphenoxy)phenyl, 3-Fluor-5-(4-fluorphenoxy)phenyl, 3-Fluor-5-(3-fluorphenoxy)phenyl, 3-Fluor-5-(2-fluorphenoxy)phenyl, 4-Methyl-3-phenoxyphenyl, 3-Fluor-5-(4-methylphenoxy)phenyl, 3-Fluor-5-(3-methoxyphenoxy)phenyl, 2-Fluor-5-(4-fluorphenoxy)phenyl, 2-Fluor-5-(3-fluorphenoxy)phenyl, 2-Fluor-5-(2-fluorphenoxy)-phenyl, 2-Chlor-3-phenoxyphenyl, 3-Fluor-5-phenoxyphenyl, 2-Fluor-5-phenoxyphenyl, 2-Chlor-5-phenoxyphenyl, 2-Brom-5-phenoxyphenyl, 4-Chlor-3-(3-methylphenoxy)-phenyl, 4-Chlor-3-(4-fluorphenoxy)phenyl, 3-Chlor-5-phenoxyphenyl, 3-Brom-5-phenoxyphenyl, 4-Brom-3-phenoxyphenyl, 4-Trifluormethyl-3-phenoxyphenyl, 4-Fluor-3-phenylthiophenyl, 4-Fluor-3-benzylphenyl, 3-(2-Pyridyloxy)phenyl, 3-(3-Pyridyloxy)-phenyl, 4-Fluor-3-(2-pyridyloxy)phenyl, 4-Chlor-3-(2-pyridyloxy)phenyl, 4-Brom3-3-(2-pyridyloxy)phenyl, 4-Methyl-3-(2-pyridyloxy)phenyl, 4-Fluor-3-(3-pyridyloxy)phenyl, 4-Chlor-3-(3-pyridyloxy)phenyl, 4-Brom-3-(3-pyridyloxy)phenyl, 4-Methyl-3-(3-pyridylox)yphenyl , 2-Methyl-3-phenylphenyl, 2-Methyl-3-(N-pyrrolyl)-phenyl, 6-Phenoxy-2-pyridyl, 6-(4-Fluorphenoxy)-2-pyridyl, 6-(4-Chlorphenoxy)-2-pyridyl, 6-(4-Bromphenoxy)-2-pyridyl, 6-(4-Methylphenoxy)-2-pyridyl, 6-(4-Methoxyphenoxy)-2-pyridyl, 6-(4-Ethoxyphenoxy)-2-pyridyl, 6-(3-Fluorphenoxy)-2-pyridyl, 6-(3-Chlorphenoxy)-2-pyridyl, 6-(3-Bromphenoxy)-2-pyridyl, 6-(3-Methoxyphenoxy)-2-pyridyl, 6-(2-Fluorphenoxy)-2-pyridyl, 6-(2-Chlorphenoxy)-2-pyridyl, 6-(2-Bromphenoxy)-2-pyridyl, 4-t-Butylphenyl, 4-Methylphenyl, 4-Isopropylphenyl, 4-(2-Chlor-4-trifluormethyl-2-pyridyloxy)phenyl, 4-Biphenyl und 4-Phenoxy-2-thienyl.

Gegenstand der vorliegenden Erfindung sind auch Verfahren zur Herstellung der Verbindungen der allgemeinen Formel (I), dadurch gekennzeichnet, daß man

a) für Verbindungen mit X = O eine Verbindung der Formel (II) oder (III)

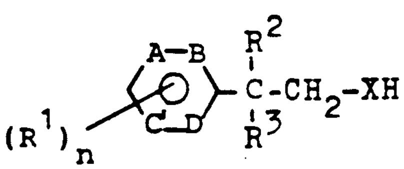 (II) 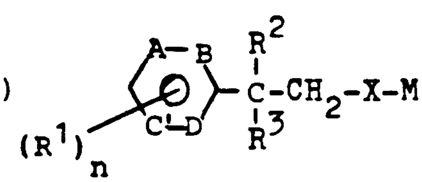 (III)

mit einem Alkylierungsmittel der Formel IV,

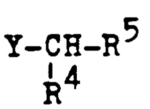 (IV),

worin Y eine nucleofuge Abgangsgruppe wie z. B. Halogen oder Sulfonat bedeutet und M einem Alkalimetall- oder Erdalkalimetall-Äquivalent, insbesondere Li, Na, K, Mg, entspricht,
gegebenenfalls in Gegenwart einer Base,
oder
b) für Verbindungen mit X = O eine Verbindung der Formel (V)

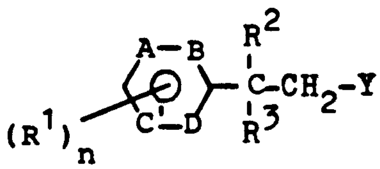

(V)

mit einer XH-aciden Verbindung des Typs (VI)

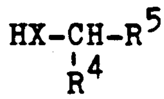

(VI)

in Gegenwart einer Base
oder mit einer metallorganischen Verbindung des Typs VII

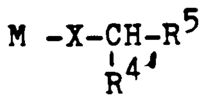

(VII),

mit $R^{4'}$ = H oder $(C_1-C_4)$Alkyl
oder
c) für Verbindungen mit X = $CH_2$ eine Verbindung der allgemeinen Formel VIII

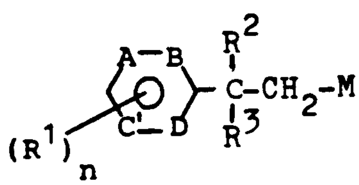

(VIII)

mit einer Verbindung des Typs (IX)

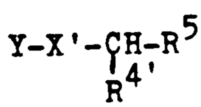

(IX)

mit X' = $CH_2$,
oder
d) eine Verbindung der Formel (X)

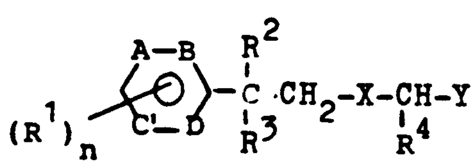

(X)

mit einem metallorganischen Reagenz des Typs (XI)

$M - R^5$ (XI)

oder
e) für Verbindungen mit $R^4$ = H und X = $CH_2$ einen Aldehyd der Formel (XII)

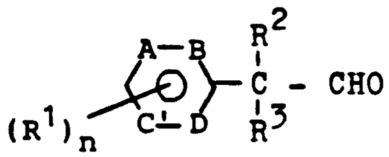

(XII)

mit einem Keton der Formel (XIII)

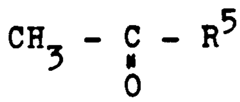

(XIII)

zu einer Zwischenstufe (XIV)

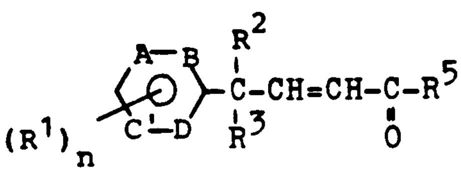

(XIV)

kondensiert und diese anschließend auf übliche Weise reduziert,
oder
f) für Verbindungen mit X = $CH_2$ einen Aldehyd der Formel (XV)

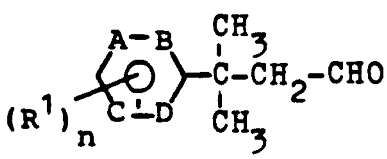

(XV)

mit einem Ylid der Formel (XVI) oder einem Phosphonester der Formel (XVII)

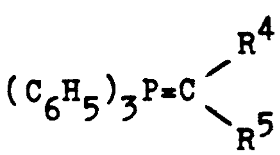

(XVI),

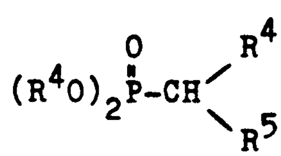

(XVII)

zu einer Zwischenstufe (XVIII)

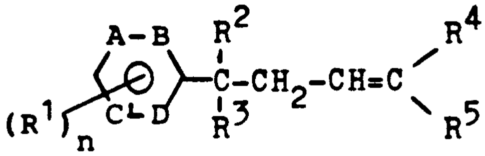

(XVIII)

kondensiert und diese anschließend mit einem Reduktionsmittel zum Endprodukt (I) mit X = $CH_2$ umsetzt.

Die als Ausgangsverbindungen beim Herstellverfahren a) zu verwendenden Substanzen (II) bzw. (III) lassen sich z.B. über eine mehrstufige Synthese aus den Heterocyclen (XIX) erhalten,

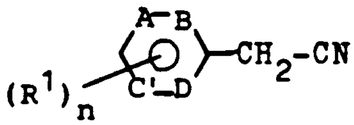

(XIX)

indem man zunächst schrittweise mit Hilfe starker Basen wie z.B. Natriumhydrid und Alkylierungsmitteln wie Alkyljodid oder Dialkylsulfat die beiden Reste $R^2$ und $R^3$ einführt, wobei die Zwischenstufe (XX) erhalten wird,

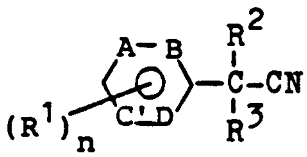

(XX)

diese dann zunächst zur entsprechenden Carbonsäure verseift und schließlich mit starken Reduktionsmitteln wie z.B. $LiAlH_4$ zum gewünschten Alkohol (II) reduziert.

Die als Ausgangsverbindungen beim Herstellverfahren b) zu verwendenden Heterocyclen des Typs V lassen sich durch eine nach Standardmethoden durchgeführte Halogenierung der Alkohole (II) (X = O)

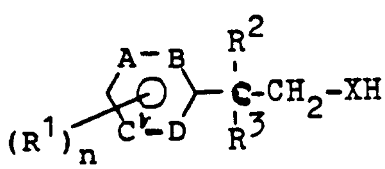

(II)

erhalten.

Die als Ausgangsverbindungen beim Herstellverfahren e) benötigten Aldehyde der allgemeinen Formel (XII) lassen sich auf einfache Weise durch Oxidation der entsprechenden Alkohole (II) (X = O), (Herstellung weiter oben beschrieben)

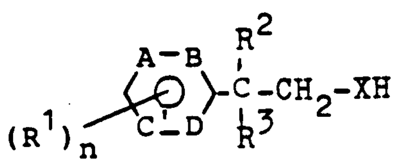

(II)

mit dem Oxidationsmittel Pyridin x $SO_3$ erhalten.

Die als Ausgangsverbindungen beim Herstellverfahren f) benötigten Aldehyde der allgemeinen Formel (XV) werden unter anderem durch Carbonylierung der aus den entsprechenden Halogeniden (V) erhältlichen Metallverbindungen (VIII)

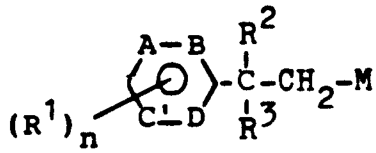

(VIII)

mit z.B. N,N-Dimethylformamid erhalten.

Die nicht näher beschriebenen Vor- und Zwischenstufen lassen sich nach üblichen Standardmethoden herstellen. So können alle erwähnten metallorganischen Zwischenstufen durch übliche Metallierungsreaktion wie Wasserstoff/Metall-Austausch oder - bevorzugt - Halogen/Metall-Austausch in allen seinen Varianten erhalten werden.

Die genannten Verfahrensvarianten c) und d) bevorzugt in einem Verdünnungsmittel durchgeführt, dessen Natur von der Art des eingesetzten Metallorganyls abhängt. Als Verdünnungsmittel eignen sich insbesondere aliphatische und aromatische Kohlenwasserstoffe wie z.B. Pentan, Hexan, Heptan, Cyclohexan, Petrolether, Benzin, Ligroin, Benzol, Toluol und Xylol, Ether wie z.B. Diethyl- und Dibutylether, Glykoldimethylether, Diglykoldimethylether, Tetrahydrofuran und Dioxan, und schließlich alle möglichen Gemische aus den zuvor genannten Lösungsmitteln.

Die Reaktionstemperatur liegt bei den obengenannten Verfahrensvarianten zwischen -75 °C und +150 °C, vorzugsweise zwischen -75 °C und +105 °C. Die Ausgangsstoffe werden gewöhnlich in äquimolaren Mengen eingesetzt. Ein Überschuß der einen oder anderen Reaktionskomponente ist jedoch möglich.

Für die weiter oben genannten Verfahrensvarianten a), b) e) und f) gilt im wesentlichen das gleiche wie für die Varianten c) und d). Bei Einsatz der Edukte vom Typ (II) und (VI) sowie der Carbonylverbindungen (XII) und (XIII) lassen sich jedoch noch weitere Verdünnungsmittel einsetzen. So eignen sich in diesen Fällen auch Ketone wie Aceton, Methylethyl-, Methylisopropyl-, und Methylisobutylketon, Ester wie Essigsäuremethylester und -ethylester, Nitrile wie z.B. Acetonitril und Propionitril, Amide wie z.B. Dimethylformamid, Dimethylacetamid und N-Methylpyrrolidon, sowie Dimethylsulfoxid, Tetramethylensulfon und Hexamethyl-phosphorsäuretriamid als Verdünnungsmittel. Als Basen finden anorganische Basen wie z.B. Alkali- oder Erdalkalimetallhydroxide, -hydride, -carbonate oder - hyrdrogencarbonate, aber auch organische Basen wie z.B. Pyridin, Triethylamin, N,N-Diisopropylethylamin oder Diazabicyclooctan Verwendung.

Die Isolierung und gegebenenfalls Reinigung der Verbindungen der Formel (I) erfolgt nach allgemein üblichen Methoden, z.B. durch Abdampfen des Lösungsmittels (gegebenenfalls unter vermindertem Druck) und anschließendes Destillieren oder Chromatographieren oder durch Verteilen des Rohproduktes zwischen zwei Phasen und die sich daran anschließende übliche Aufarbeitung

Die Verbindungen der allgemeinen Formel (I) sind in den meisten organischen Lösungsmitteln gut löslich.

Die Wirkstoffe eignen sich bei guter Pflanzenverträglichkeit und günstiger Warmblütertoxizität zur Bekämpfung von tierischen Schädlingen, insbesondere Insekten, Spinnentieren, und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blatella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp.,

Aus der Ordnung der Anoplura z.B. Phylloera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Bravicornyne brassicae, Cryptomyzus ribis, Doralis fabae, Doralis pomi, Eriosoma lanigerum Hyalopterus arundinis, Macrosiphium avenae, Myzus spp., Phorodon humuli, Rhopalosiphum padi, Empoasca spp., Euscelus bilobatus, Nephotettix cincticeps, Lecanium corni, Saissetia oleae, Laodelphax striatellus, Nilaparvata lugens, Aonidiella auroantii, Aspidiotus hederae, Pseudococcus spp., Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Buceulatrix thurberiella, Phyllocnistis citrella, Agrotis spp, Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami¸ Ceratitis capitata, Dacus oleae, Tipula paludosa.

Aus der Ordnung der Siphonaptera z.B. Xenopsylla cheopis, Ceratophyllus spp.

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Weiterhin haben die Verbindungen eine ausgezeichnete Wirkung gegen pflanzenschädigende Nematoden, beispielsweise solche der Gattungen Meloidogyne, Heterodera, Ditylenchus, Aphelenchoides, Radopholus, Globodera, Pratylenchus, Longidorus und Xiphinema. Gegenstand der Erfindung sind auch Mittel, die die Verbindungen der Formel I neben geeigneten Formulierungshilfsmitteln enthalten.

Die erfindungsgemäßen Mittel enthalten die Wirkstoffe der Formel I, im allgemeinen zu 1 - 95 Gew.-%. Sie können als Spritzpulver, emulgierbare Konzentrate, versprühbare Lösungen, Stäubemittel oder Granulate in den üblichen Zubereitungen angewendet werden.

Spritzpulver sind in Wasser gleichmäßig dispergierbare Präparate, die neben dem Wirkstoff außer einem Verdünnungs- oder Inertstoff noch Netzmittel, z.B. polyoxethylierte Alkylphenole, polyoxethylierte Fettalkohole, Alkyl- oder Alkylphenol-sulfonate und Dispergiermittel, z.B. ligninsulfonsaures Natrium, 2,2'-dinaphthylmethan6,6'-disulfonsaures Natrium, dibutylnaphthalinsulfonsaures Natrium oder auch oleylmethyltaurinsaures Natrium enthalten.

Emulgierbare Konzentrate werden durch Auflösen des Wirkstoffes in einem organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwandt werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanfettsäureester Polyoxyethylensorbitan-Fettsäureester oder Polyoxethylensorbitester.

Stäubemittel erhält man durch Vermahlen des Wirkstoffes mit fein verteilten festen Stoffen, z.B. Talkum, natürlichen Tonen wie Kaolin, Bentonit, Poryphillit oder Diatomeenerde. Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen auf die Oberfläche von Trägerstoffen wie Sand, Kaolinite, oder von granuliertem Inertmaterial. Auch können geeignete Wirkstoffe in der für die Herstellung von Düngemittelgranulaten üblichen Weise - gewünschtenfalls in Mischung mit Düngemitteln - herstellt werden.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen.

Zu den Insektiziden zählen beispielsweise Phosphorsäureester, Carbamate, Carbonsäureester, Formamidine, Zinnverbindungen, durch Mikroorganismen hergestellte Stoffe, Insektenwachstumsregulatoren u. a.

Bevorzugte Mischungspartner sind

1. aus der Gruppe der Phosphorverbindungen
Azinphos-ethyl, Azinphos-methyl, 1-(4-Chlorphenyl-4-(O-ethyl,S-propyl)phosphoryloxypyrazol (TIA 230), Chlorpyrifos, Coumaphos, Demeton, Demeton-S-methyl, Diazinon¸ Etrimfos, Fenitrothion, Fenthion, Parathion-methyl, Phosalon, Pirimiphos-ethyl, Pirimiphos-methyl, Profenofos, Prothiofos, Sulprofos, Triazophos, Trichlorphon.

2. aus der Gruppe der Carbamate
Aldicarb, Bendiocarb, BPMC (2-(1-Methylpropyl)phenylmethylcarbamat), Butocarboxim, Butoxicarboxim, Carbaryl, Carbofuran, Carbosulfan, Cloethocarb, Isoprocarb, Methomyl, Oxamyl, Piromicarb, Promecarb, Propoxur, Thiodicarb.

3. aus der Gruppe der Carbonsäureester
Allethrin, Alphametrin, Bioallethrin, Bioresmethrin, Cycloprothrin, Cyfluthrin, Cyhalothrin, Cypermethrin, Deltamethrin, 2,2-Dimethyl-3-(2-chlor-2-trifluormethylvinyl)cyclopropancarbonsäure(α-cyano-3-phenyl-2-methylbenzyl)ester (FMC 54800), Fenpropathrin, Fenfluthrin, Fenvalerate, Flucythrinate, Flumethrin, Fluvalinate, Permethrin, Resmethrin, Tralomethrin.

4. aus der Gruppe der Amidine
Amitraz, Chlordimeform;

5. aus der Gruppe der Zinnverbindungen
Azocyclotin, Cyhexatin, Fenbutatinoixid

6. aus der Gruppe der Wachstumsregulatoren
Fenoxycarb, Flufenoxuron, Buprofezin, Cyromazine, Flubenzimin, Diflubenzuron, Triflumuron, N-(((3,5-dichloro-4-(1,1,2,2,-tetrafluorethoxy)phenyl)aminocarbonyl)-2,6-difluorobenzamide (XRD 473), Tetraflubenzuron, Chlorfluazuron, Flucyclooxuron, Hydroprene, Methoprene, N-(2-Fluorbenzoyl)-N'-(4-(2-chlor-4-trifluormethyl-1-phenylmercapto)phenyl)harnstoff, N-(2-Chlorbenzoyl)-N'-(4-(2-chlor-4-trifluormethyl-1-phenylmercapto)phenyl)harnstoff, N-(2,6-Difluorbenzoyl)-N'-(4-(2-chlor-4-trifluormethyl-1-phenylmercapto)-phenyl)harnstoff, N-(N-(2-Chlor-4-trifluormethyl-1-phenylmercapto)phenyl)carbamoyl)-2-fluorbenzcarboximidsäureethylester, N-(N-(2-Chlor-4-trifluormethyl-1-phenylmercapto)phenyl)carbamoyl)-2-chlorbenzcarboximidsäureethylester, N-(N-(2-Chlor-4-trifluormethyl-1-phenylmercapto)phenyl)carbamoyl)-2,6-difuorbenzcarboximidsäureethylester.

7. Sonstige
α- und β-Acermectine, Bacillus thuringensis, Bensultap, Binapacryl, Bisclofentezin, Buprofezin, Cartap, Cyromacin, Dicofol, Endosulfan, Ethoproxyfen, Fenoxycarb, Hexythiazox, 3- 2-(4-Ethoxyphenyl)-2-methylpropoxymethyl-1,3-diphenylether (MTI-500), 5- 4-(4-Ethoxyphenyl)-4-methylpentyl -2-fluoro-1,3-diphenylether (MTI-800), 3-(2-Chlorphenyl)-3-hydroxy-2-(2-phenyl-4-thiazolyl)propennitril (SN 72129), Thiocyclam, Kernpolyeder-und Granuloseviren.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,00001 und 1 Gew.-% liegen.

Die Anwendung geschieht in einer den Anvendungsformen angepaßten üblichen Weise.

Die erfindungsgemäßen Wirkstoffe eignen sich auch zur Bekämpfung von Ekto- und Endoparasiten vorzugsweise von ektoparasitierenden Insekten auf dem veterinärmedizinischen Gebiet bzw. auf dem Gebiet der Tierhaltung.

Die Anwendung der erfindungsgemäßen Wirkstoffe geschieht hier in bekannter Weise, wie durch dermale Anwendung in Form beispielsweise des Tauchens (Dippen), Sprühens (Sprayen), Aufgießens (pouron and spot-on) und des Einpuderns.

A. Formulierungsbeispiele

a) Ein Stäubemittel wird erhalten, indem man 10 Gew.-Teile Wirkstoff und 90 Gew.-Teile Talkum als Inertstoff mischt und in einer Schlagmühle zerkleinert.

b) Ein in Wasser leicht dispergierbares, benetzbares Pulver wird erhalten, indem man 25 Gew.-Teile Wirkstoff, 65 Gew.-Teile kaolinhaltigen Quarz als Inertstoff, 10 Gew.-Teile ligninsulfonsaures Kalium und

1 Gew.-Teil oleoylmethyltaurinsaures Natrium als Netz- und Dispergiermittel mischt und in einer Stiftmühle mahlt.

c) Ein in Wasser leicht dispergierbares Dispersionskonzentrat stellt man her, indem man 20 Gew.-Teile Wirkstoff mit 6 Gew.-Teilen Alkylphenolpolyglykolether (Triton X 207), 3 Gew.-Teilen Isotridecanolpolyglykolether (8 AeO) und 71 Gew.-Teilen paraffinischem Mineralöl (Siedebereich z.B. ca. 255 bis über 377 ° C) mischt und in einer Reibkugelmühle auf eine Feinheit von unter 5 Mikron vermahlt.

d) Ein emulgierbares Konzentrat läßt sich herstellen aus 15 Gew.-Teilen Wirkstoff, 75 Gew.-Teilen Cyclohexanon als Lösungsmittel und 10 Gew.-Teilen oxethyliertes Nonylphenol (10 AeO) als Emulgator.

e) Ein Granulat läßt sich herstellen aus 2 bis 15 Gew.-Teilen Wirkstoff und einem inerten Granulatträgermaterial wie Attapulgit, Bimsgranulat und/oder Quarzsand.

## B. Chemische Beispiele

Beispiel 1: **2-Methyl-2-(6-chlor-pyrid-3-yl)-propyl-(3-phenoxy-benzyl)-ether (Verb. Nr. 18 Tab. 1)**

1.1) **6-Chlorpyridin-3-methanol**

Man trägt 69,5 g (0,5 Mol) 6-Hydroxynicotinsäure in ein Gemisch aus 200 g (1 Mol) Phosphorpentachlorid und 233 g (1,5 Mol) Phosphoroxidtrichlorid bei Raumtemperatur ein, rührt 5 h bei 95 ° C und entfernt danach überschüssige Phosphorchloride im Vakuum. Der kristalline Rückstand wird in eine Lösung von 75 g (1,98 Mol) Natriumborhydrid in 1 000 ml Wasser eingetragen, wobei man die Temperatur bei höchstens 30 ° C hält. Man rührt bei Raumtemperatur bis zur Beendigung der Gasentwicklung nach, saugt vom Festkörper ab und extrahiert das Filtrat mit Ether. Nach Entfernung des Lösungsmittels aus der organischen Phase erhält man 44,5 g (62 % d. Th.) öliges Rohprodukt. Man destilliert bei 0,4 mbar (Kp 110 - 115 ° C) 36,6 g (51 % d. Th.).

1.2) **2-Chlor-5-chlormethyl-pyridin**

14,4 g (0,1 Mol) 6-Chlor-pyridin-3-methanol werden vorsichtig mit 85 ml Thionylchlorid versetzt. Danach tropft man 8,1 g (0,1 Mol) Pyridin zu und erhitzt unter Rückfluß, bis die Gasentwicklung beendet ist. Überschüssiges Thionylchlorid wird im Vakuum entfernt, der Rückstand auf Eis gegeben. Man saugt den Niederschlag ab und trocknet. Ausbeute 10,6 g (65,5 % d. Th.), Fp.: 38 - 40 ° C.
Das Rohprodukt kann ohne Reinigung weiterverarbeitet werden.

1.3) **6-Chlor-pyridin-3-acetonitril**

Man löst 25,6 g Natriumcyanid (0,522 Mol) in 123 g Dimethylsulfoxid, erwärmt auf 90 ° C und trägt 68,9 g (0,43 Mol) 2-Chlor-5-chlormethyl-pyridin portionsweise ein. Dabei steigt die Temperatur auf 130 ° C an. Man läßt auf Raumtemperatur abkühlen, gibt auf Wasser und extrahiert mit Ether. Man entfernt das Lösungsmittel und destilliert das Rohprodukt bei 1,0 mbar (130 - 135 ° C). FP.: 80 ° C.
Ausbeute: 41,1 g (63 % d. Th.).

1.4) **2-(6-Chlor-pyrid-3-yl)-2-methyl-propannitril**

Man suspendiert 24 g (0,8 Mol) 80 %iges Natriumhydrid (in Weißöl) in 640 ml Tetrahydrofuran und tropft 56 g (0,37 Mol) 6-Chlor-pyridin-3-acetonitril, in 160 ml Tetrahydrofuran gelöst, zu. Nach Beendigung der Gasentwicklung wird bei 0 - 20 ° C 105,3 g (0,74 Mol) Methyliodid, in 160 ml Tetrahydrofuran gelöst, eingetropft. Man rührt bei Raumtemperatur noch 3 h und gießt dann auf 860 ml Wasser. Das Rohprodukt wird mit Ether extrahiert und nach Entfernen des Lösungsmittels bei 0,3 mbar (Kp. 110 - 120 ° C) destilliert. So werden 49,1 g (73,5 % d. Th.) eines gelblichen Öls erhalten, das beim Stehen kristallisiert. Fp.: 63 - 65 ° C.

1.5) **2-(6-Chlor-pyrid-3-yl)-2-methyl-propanol**

3,6 g (20 mMol) 2-(6-Chlor-pyrid-3-yl)-2-methylpropionitril werden in 50 ml Toluol vorgelegt, und 25 ml einer Diisobutylaluminiumhydrid-Lösung (1 M in Toluol) ohne Kühlung zugetropft, wobei die Innentemperatur auf 45 ° C steigt. Man rührt 2 h bei Raumtemperatur. Dann gibt man vorsichtig nacheinander 10 ml Methanol, 50 ml Wasser und 90 ml 2 N Schwefelsäure zu, rührt 2 h bei Raumtemperatur und schließlich 2

h bei 50 ° C. Die Toluolphase wird abgetrennt und das Lösungsmittel entfernt. Der Rückstand wird in 10 ml Methanol gelöst und mit einer Lösung von 0,1 g Natrium und 0,4 g (10,6 mMol) Natriumborhydrid in 10 ml Methanol versetzt. Man rührt 1 h bei Raumtemperatur, säuert mit 0,7 g Essigsäure an und entfernt das Lösungsmittel. Der Rückstand wird mit Essigsäureethylester ausgekocht, die Esterphase abgetrennt und im Vakuum das Lösungsmittel entfernt. Das ölige Rohprodukt wird im Kugelrohr destilliert; 0,15 mbar, Apparatetemperatur 170 ° C. Ausbeute 2,5 g (67 % d. Th.) eines farblosen Öls.

1.6) **2-(6-Chlor-pyrid-3-yl)-2-methyl-propyl-(3-phenoxy-benzyl)-ether**

Man löst 1,9 g (10,2 mMol) 2-(6-Chlor-pyrid-3-yl)-2-methyl-propanol in 15 ml Toluol, gibt 2,7 g (10,3 mMol) 3-Phenoxy-benzylbromid zu und versetzt mit einer Lösung von 0,6 g (1,8 mMol) Tetra-n-butylammonium-hydrogen-sulfat in 5 g 50 %iger Natronlauge. Es wird 2 h bei 70 - 80 ° C gerührt, mit einem Gemisch aus 2 ml Methanol und 2 ml konzentriertem Ammoniakwasser verrührt und schließlich alles zwischen Wasser und Toluol verteilt. Die organische Phase wird mit Ammoniumchloridlösung gewaschen, das Lösungsmittel entfernt und das Rohprodukt über eine Kieselgel-Säule gereinigt. Man erhält so 2,4 g (62,9 % d. Th.) eines schwach gelblichen Öls. Destilliert im Kugelrohr bei 0,03 mbar und 230 - 240 ° C Apparatetemperatur.

Beispiel 2: **1-(6-Chlor-pyrid-3-yl)-cyclopropyl)-methyl-(3-phenoxybenzyl)-ether (Verb. Nr. 513 in Tab. 2)**

2.1) **1-(6-Chlor-pyrid-3-yl)-cyclopropan-1-carbonitril** (entspricht Verfahren 1.4 in Beispiel 1).

6,5 g (0,217 Mol) 80 %iges Natriumhydrid in Weißöl werden in 170 ml Tetrahydrofuran suspendiert und die Lösung von 15,3 g (0,1 Mol) 6-Chlor-pyridin-3-acetonitril in 60 ml Tetrahydrofuran zugetropft. Man rührt bis zur Beendigung der Gasentwicklung, kühlt ab und läßt bei 5 - 20 ° C die Lösung von 14,3 g (0,1 Mol) 1-Brom-2-chlor-ethan in 15 ml Tetrahydrofuran zutropfen. Man rührt 2 h bei Raumtemperatur, gießt auf konzentrierte Kochsalzlösung und extrahiert mit Ether. Das vom Lösungsmittel befreite Rohprodukt wird in Essigsäureethylester aufgenommen und durch ein Bett aus 200 g Kieselgel filtriert. Man erhält so 14,3 g einer bräunlichen, halbkristallinen Masse, die durch Kugelrohrdestillation gereinigt wird. Bei 130 - 140 ° C Apparatetemperatur und 0,08 mbar destillieren 5,8 g (32,5 % d. Th.) eines beim Abkühlen erstarrenden Öls.

Diese Substanz läßt sich in Analogie zu den in Beispiel 1 angeführten Verfahren 1.5 und 1.6 in die Verbindung Nr. 513 (Tab. 2) überführen.

Beispiel 3: **(2-(6-Ethoxy-pyridazin-3-yl)-2-methyl-propyl)-(3-phenoxy-benzyl)-ether (Verb. Nr. 343 in Tab. 1)**

3.1) **2-(6-Hydroxy-pyridazin-3-yl)-propannitril**

60,4 g (2,02 Mol) 80 %iges Natriumhydrid in Weißöl werden in 2 l Tetrahydrofuran suspendiert und bei 20 - 30 ° C tropfenweise mit 311,8 g (2,02 Mol) 2-Cyanpropionsäuretert.-butylester versetzt. Innerhalb 45 Min. wird diese klare Lösung zu einer Lösung von 298 g (2,0 Mol) 3,6-Dichlorpyridazin getropft und alles 2 h unter Rückfluß erhitzt. Dann gibt man 317 ml Essigsäure zu und entfernt das Lösungsmittel im Vakuum. Der Rückstand wird in einer Mischung aus 6 400 ml Essigsäure, 250 ml Acetanhydrid und 32 g p-Toluolsulfonsäure-monohydrat aufgenommen, 3 h unter Rückfluß erhitzt und wiederum im Vakuum zum Trocknen gebracht. Man schlämmt in wenig Wasser auf, stellt mit Natronlauge pH 5 ein und dampft das Wasser ab. Der Rückstand wird mit Essigsäureethylester heiß extrahiert. So werden 99,7 g (33 % d. Th.) Rohprodukt erhalten, welches ohne weiteres zu der folgenden Reaktion eingesetzt werden kann.

3.2) **2-(6-Chlor-pyridazin-3-yl)-propannitril**

1,5 g (10 mMol) 2-(6-Hydroxy-pyridazin-3-yl)-propannitril werden mit 5 g Phosphoroxidtrichlorid vermischt und 45 min. bei 80 - 90 ° C gerührt. Man gibt auf 30 g Eis, stellt mit 2N NaOH pH 6 ein und ethert aus. Nach Entfernung des Lösungsmittels bleibt ein Öl zurück, das beim Stehen erstarrt.
Ausbeute: 1 g (59,7 % d. Th.); Fp.: 65 ° C aus Hexan.

3.3) **2-(6-Chlor-pyridazin-3-yl)-2-methyl-propannitril**

3,9 g (0,131 Mol) 80 %iges Natriumhydrid in Weißöl werden in 380 ml Tetrahydrofuran suspendiert und mit einer Lösung von 18,9 g (0,113 Mol) 2-(6-Chlor-pyridazin-3-yl)-propannitril in 80 ml Tetrahydrofuran bei 15 - 20 ° C versetzt. Nach Beendigung der Gasentwicklung tropft man die Lösung von 16,3 g (0,115 Mol) Methyliodid in 50 ml Tetrahydrofuran zu und rührt 1 h bei Raumtemperatur. Man gießt auf 1,4 l konzentrierte Kochsalzlösung und ethert aus. Das Rohprodukt, das nach dem Entfernen des Lösungsmittels zurückbleibt, wir durch Kugelrohrdestillation gereinigt. Apparatetemperatur: 180 - 190 ° C¸ 0,1 mbar.
Ausbeute 8,4 g (41 % d. Th.) Öl, das beim Stehen erstarrt. Fp.: 107 - 111 ° C.

In Analogie zu dem in Beispiel 1 angeführten Verfahren 1.5 läßt sich dieses Nitril in 2-(6-Chlor-pyridazin-3-yl)-2-methyl-propanol überführen.

3.4) **2-(6-Ethoxy-pyridazin-3-yl)-2-methyl-propanol**

1,6 g (0,053 Mol) 80 %iges Natriumhydrid in Weißöl werden in 50 ml Dimethylsulfoxid suspendiert und bei 15 - 20 ° C werden 2,5 g (0,055 Mol) Enthanol zugetropft. Nach Bendigung der Gasentwicklung läßt man die Lösung von 1,0 g (0,005 Mol) 2-(6-Chlor-pyridazin-3-yl)-2-methyl-propanol in 20 ml Dimethylsulfoxid zulaufen und erwärmt 1,5 h auf 90 - 100 ° C. Nach dem Abkühlen gibt man auf konzentrierte Kochsalzlösung und ethert aus. Nach Entfernen des Lösungsmittels bleibt ein gelbes Öl als Rohprodukt zurück. Ausbeute: 0,6 g (61 % d. Th.).

Dieses Rohprodukt kann direkt und in Analogie zu dem in Beispiel 1 angeführten Verfahren 1.6 in die Verbindung Nr. 343 der Tab. 1 überführt werden.

Die Herstellung der nachfolgenden Verbindungen der Formel I

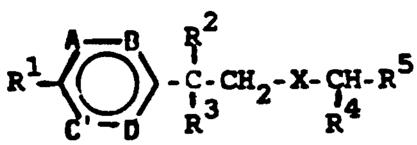

(I)

(Tabellen 1 und 2) mit X = O erfolgt analog zu den oben aufgezeigten Beispielen. Verbindungen mit X = $CH_2$ erhält man bevorzugt nach den Verfahren e) und f).

**Tabelle 1** $R^2$, $R^3$ = $CH_3$, A=N, CH oder C-$R^1$

| Verbdg. Nr. | A | $R^1$ | B | C' | D | X | $R^4$ | $R^5$ | physik. Daten |
|---|---|---|---|---|---|---|---|---|---|
| 1 | N | EtO | CH | CH | CH | $CH_2$ | H | 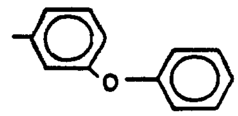 | $Kp_{0.01}$ = 225-230°C |
| 2 | N | EtO | CH | CH | CH | $CH_2$ | H | 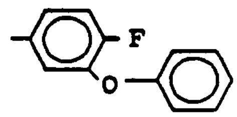 | $Kp_{0.05}$ = 240-245°C |
| 3 | N | EtO | CH | CH | CH | $CH_2$ | H | 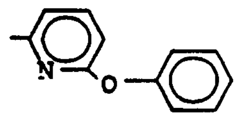 | |
| 4 | N | EtO | CH | CH | CH | $CH_2$ | H | 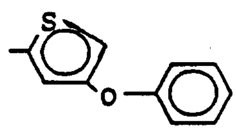 | |
| 5 | N | EtO | CH | CH | CH | $CH_2$ | H | 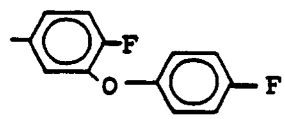 | $Kp_{0.02}$ = 230-240°C |
| 6 | N | EtO | CH | CH | CH | $CH_2$ | CN | 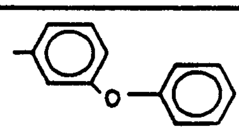 | |

**Fortsetzung Tabelle 1**

| Verbdg. Nr. | A | $R^1$ | B | C' | D | X | $R^4$ | $R^5$ | physik. Daten |
|---|---|---|---|---|---|---|---|---|---|
| 7 | N | EtO | CH | CH | CH | $CH_2$ | CN | 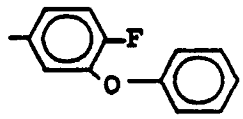 | |
| 8 | N | EtO | CH | CH | CH | O | H | 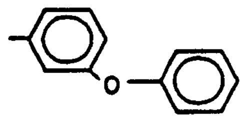 | $Kp_{0.005}$ = 210-220°C |
| 9 | N | EtO | CH | CH | CH | O | H | 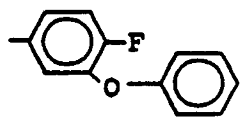 | $Kp_{0.1}$ = 230-235°C |
| 10 | N | EtO | CH | CH | CH | O | H | 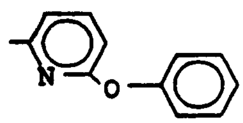 | |
| 11 | N | EtO | CH | CH | CH | O | CN | 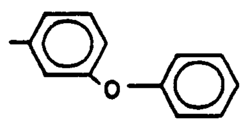 | |
| 12 | N | Cl | CH | CH | CH | $CH_2$ | H | 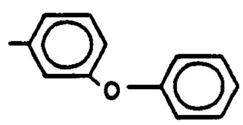 | |
| 13 | N | Cl | CH | CH | CH | $CH_2$ | H | 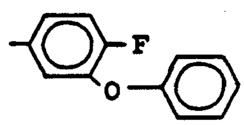 | |
| 14 | N | Cl | CH | CH | CH | $CH_2$ | H | 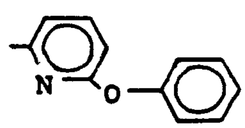 | |
| 15 | N | Cl | CH | CH | CH | $CH_2$ | H | 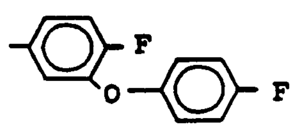 | |

**Fortsetzung Tabelle 1**

| Verbdg. Nr. | A | $R^1$ | B | C' | D | X | $R^4$ | $R^5$ | physik. Daten |
|---|---|---|---|---|---|---|---|---|---|
| 16 | N | Cl | CH | CH | CH | $CH_2$ | CN | 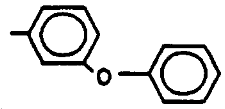 | |
| 17 | N | Cl | CH | CH | CH | $CH_2$ | CN | 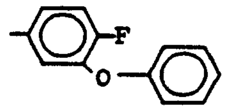 | |
| 18 | N | Cl | CH | CH | CH | O | H | 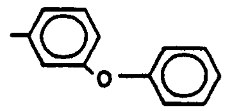 | $Kp_{0.03}$ = 230–240°C |
| 19 | N | Cl | CH | CH | CH | O | H | 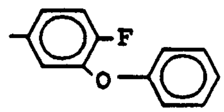 | $Kp_{0.01}$ = 225–230°C |
| 20 | N | Cl | CH | CH | CH | O | H | 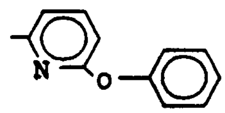 | |
| 21 | N | Cl | CH | CH | CH | O | CN | 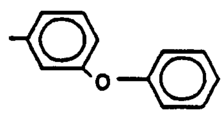 | |
| 22 | N | Cl | CH | CH | CH | O | $CH_3$ | 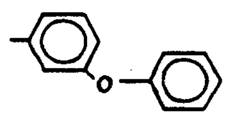 | |
| 23 | N | $H_3CO$ | CH | CH | CH | $CH_2$ | H | 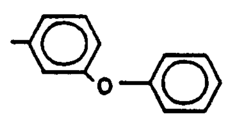 | |
| 24 | N | $H_3CO$ | CH | CH | CH | $CH_2$ | H | 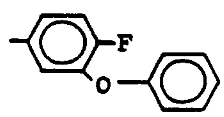 | |
| 25 | N | $H_3CO$ | CH | CH | CH | $CH_2$ | H | 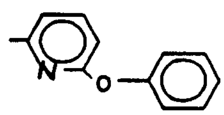 | |

Fortsetzung Tabelle 1

| Verbdg. Nr. | A | $R^1$ | B | C' | D | X | $R^4$ | $R^5$ | physik. Daten |
|---|---|---|---|---|---|---|---|---|---|
| 26 | N | $H_3CO$ | CH | CH | CH | $CH_2$ | H | 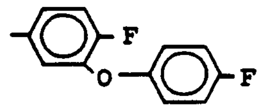 | |
| 27 | N | $H_3CO$ | CH | CH | CH | $CH_2$ | CN | 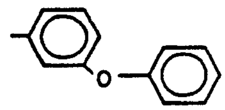 | |
| 28 | N | $H_3CO$ | CH | CH | CH | $CH_2$ | CN | 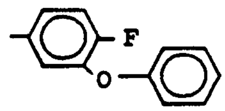 | |
| 29 | N | $H_3CO$ | CH | CH | CH | O | H | 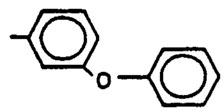 | |
| 30 | N | $H_3CO$ | CH | CH | CH | O | H | 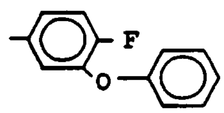 | $Kp_{0.07}$ = 220-240°C |
| 31 | N | $H_3CO$ | CH | CH | CH | O | H | 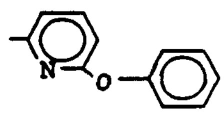 | |
| 32 | N | $H_3CO$ | CH | CH | CH | O | CN | 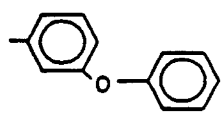 | |
| 33 | N | $H_3CO$ | CH | CH | CH | O | $CH_3$ | 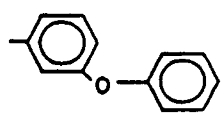 | |
| 34 | N | EtS | CH | CH | CH | $CH_2$ | H | 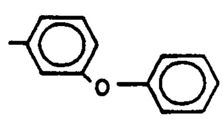 | |
| 35 | N | EtS | CH | CH | CH | $CH_2$ | H | 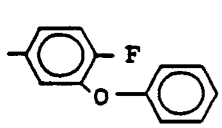 | |

**Fortsetzung Tabelle 1**

| Verbdg. Nr. | A | $R^1$ | B | C' | D | X | $R^4$ | $R^5$ | physik. Daten |
|---|---|---|---|---|---|---|---|---|---|
| 36 | N | EtS | CH | CH | CH | $CH_2$ | H | 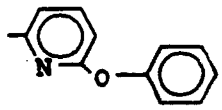 | |
| 37 | N | EtS | CH | CH | CH | $CH_2$ | H | 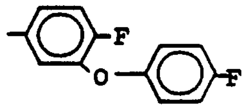 | |
| 38 | N | EtS | CH | CH | CH | $CH_2$ | CN | 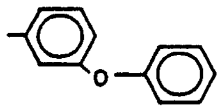 | |
| 39 | N | EtS | CH | CH | CH | $CH_2$ | CN | 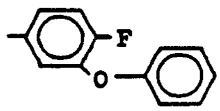 | |
| 40 | N | EtS | CH | CH | CH | O | H | 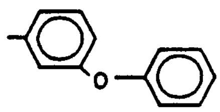 | |
| 41 | N | EtS | CH | CH | CH | O | H | 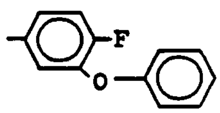 | $Kp_{0.01}$ = 220-230°C |
| 42 | N | EtS | CH | CH | CH | O | H | 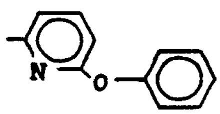 | |
| 43 | N | EtS | CH | CH | CH | O | CN | 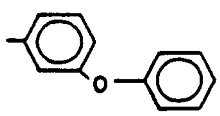 | |
| 44 | N | EtS | CH | CH | CH | O | $CH_3$ | 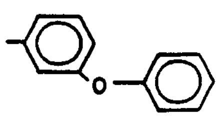 | |
| 45 | N | $H_3CS$ | CH | CH | CH | $CH_2$ | H | 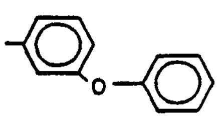 | |

Fortsetzung Tabelle 1

| Verbdg. Nr. | A | $R^1$ | B | C' | D | X | $R^4$ | $R^5$ | physik. Daten |
|---|---|---|---|---|---|---|---|---|---|
| 46 | N | $H_3CS$ | CH | CH | CH | $CH_2$ | H | 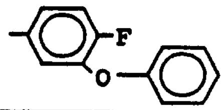 | |
| 47 | N | $H_3CS$ | CH | CH | CH | $CH_2$ | H | 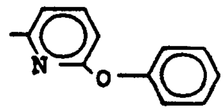 | |
| 48 | N | $H_3CS$ | CH | CH | CH | $CH_2$ | H | 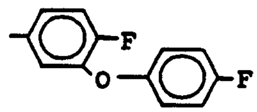 | |
| 49 | N | $H_3CS$ | CH | CH | CH | $CH_2$ | CN | 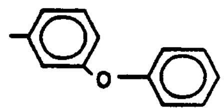 | |
| 50 | N | $H_3CS$ | CH | CH | CH | $CH_2$ | CN | 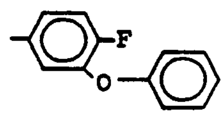 | |
| 51 | N | $H_3CS$ | CH | CH | CH | O | H | 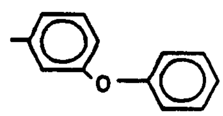 | |
| 52 | N | $H_3CS$ | CH | CH | CH | O | H | 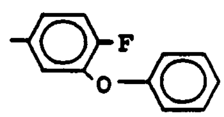 | |
| 53 | N | $H_3CS$ | CH | CH | CH | O | H | 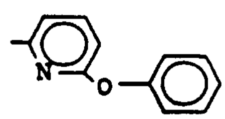 | |
| 54 | N | $H_3CS$ | CH | CH | CH | O | CN | 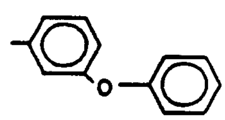 | |
| 55 | N | $H_3CS$ | CH | CH | CH | O | $CH_3$ | 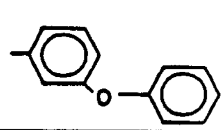 | |

Fortsetzung Tabelle 1

| Verbdg. Nr. | A | $R^1$ | B | C' | D | X | $R^4$ | $R^5$ | physik. Daten |
|---|---|---|---|---|---|---|---|---|---|
| 56 | CCl | Cl | CH | N | CH | $CH_2$ | H | 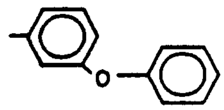 | |
| 57 | CCl | Cl | CH | N | CH | $CH_2$ | H | 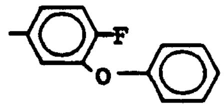 | |
| 58 | CCl | Cl | CH | N | CH | $CH_2$ | H | 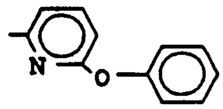 | |
| 59 | CCl | Cl | CH | N | CH | $CH_2$ | H | 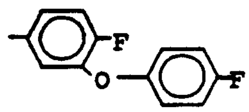 | |
| 60 | CCl | Cl | CH | N | CH | $CH_2$ | CN | 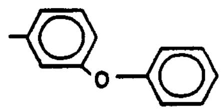 | |
| 61 | CCl | Cl | CH | N | CH | $CH_2$ | CN | 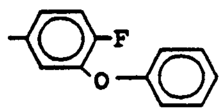 | |
| 62 | CCl | Cl | CH | N | CH | O | H | 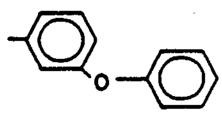 | |
| 63 | CCl | Cl | CH | N | CH | O | H | 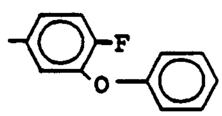 | |
| 64 | CCl | Cl | CH | N | CH | O | H | 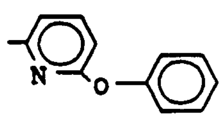 | |
| 65 | CCl | Cl | CH | N | CH | O | CN | 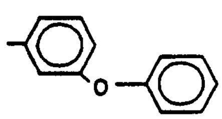 | |

**Fortsetzung Tabelle 1**

| Verbdg. Nr. | A | $R^1$ | B | C' | D | X | $R^4$ | $R^5$ | physik. Daten |
|---|---|---|---|---|---|---|---|---|---|
| 66 | CCl | Cl | CH | N | CH | O | $CH_3$ | 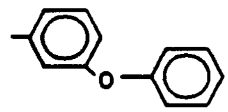 | |
| 67 | CCl | EtO | CH | N | CH | $CH_2$ | H | 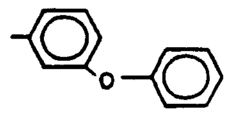 | |
| 68 | CCl | EtO | CH | N | CH | $CH_2$ | H | 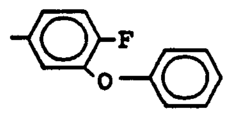 | |
| 69 | CCl | EtO | CH | N | CH | $CH_2$ | H | 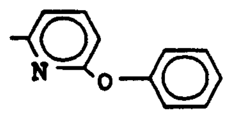 | |
| 70 | CCl | EtO | CH | N | CH | $CH_2$ | H | 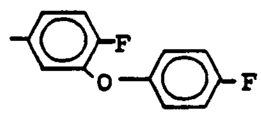 | |
| 71 | CCl | EtO | CH | N | CH | $CH_2$ | CN | 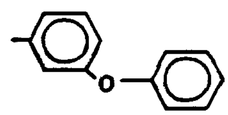 | |
| 72 | CCl | EtO | CH | N | CH | $CH_2$ | CN | 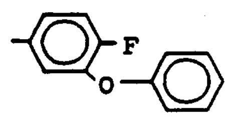 | |
| 73 | CCl | EtO | CH | N | CH | O | H | 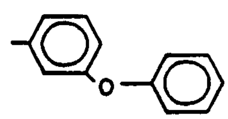 | |
| 74 | CCl | EtO | CH | N | CH | O | H | 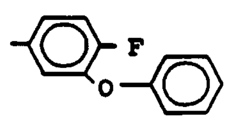 | |
| 75 | CCl | EtO | CH | N | CH | O | H | 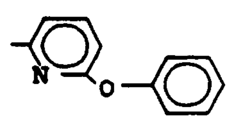 | |

Fortsetzung Tabelle 1

| Verbdg. Nr. | A | $R^1$ | B | C' | D | X | $R^4$ | $R^5$ | physik. Daten |
|---|---|---|---|---|---|---|---|---|---|
| 76 | CCl | EtO | CH | N | CH | O | CN | 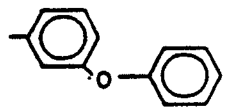 | |
| 77 | CCl | EtO | CH | N | CH | O | $CH_3$ | 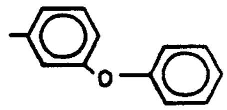 | |
| 78 | CF | EtO | CH | N | CH | $CH_2$ | H | 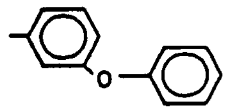 | |
| 79 | CF | EtO | CH | N | CH | $CH_2$ | H | 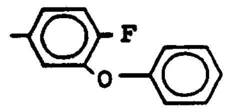 | |
| 80 | CF | EtO | CH | N | CH | $CH_2$ | H | 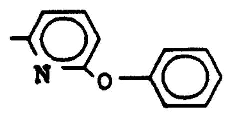 | |
| 81 | CF | EtO | CH | N | CH | $CH_2$ | H | 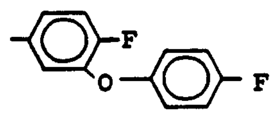 | |
| 82 | CF | EtO | CH | N | CH | $CH_2$ | CN | 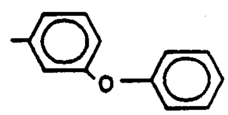 | |
| 83 | CF | EtO | CH | N | CH | $CH_2$ | CN | 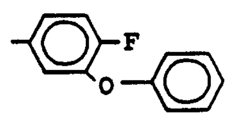 | |
| 84 | CF | EtO | CH | N | CH | O | H | 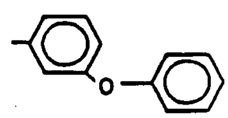 | |
| 85 | CF | EtO | CH | N | CH | O | H | 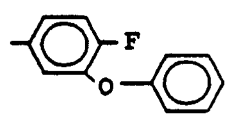 | |

Fortsetzung Tabelle 1

| Verbdg. Nr. | A | $R^1$ | B | C' | D | X | $R^4$ | $R^5$ | physik. Daten |
|---|---|---|---|---|---|---|---|---|---|
| 86 | CF | EtO | CH | N | CH | O | H | 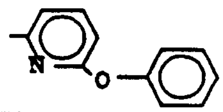 | |
| 87 | CF | EtO | CH | N | CH | O | CN | 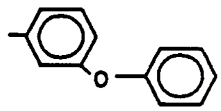 | |
| 88 | CF | EtO | CH | N | CH | O | $CH_3$ | 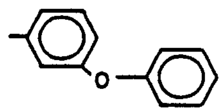 | |
| 89 | C-O-$CH_2$-O | | CH | N | CH | $CH_2$ | H | 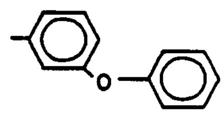 | |
| 90 | C-O-$CH_2$-O | | CH | N | CH | $CH_2$ | H | 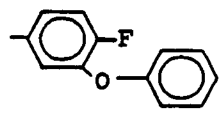 | |
| 91 | C-O-$CH_2$-O | | CH | N | CH | $CH_2$ | H | 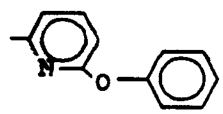 | |
| 92 | C-O-$CH_2$-O | | CH | N | CH | $CH_2$ | H | 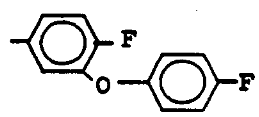 | |
| 93 | C-O-$CH_2$-O | | CH | N | CH | $CH_2$ | CN | 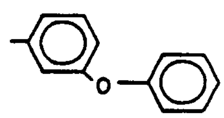 | |
| 94 | C-O-$CH_2$-O | | CH | N | CH | $CH_2$ | CN | 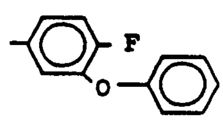 | |
| 95 | C-O-$CH_2$-O | | CH | N | CH | O | H | 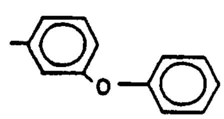 | |

Fortsetzung Tabelle 1

| Verbdg. Nr. | A | $R^1$ | B | C' | D | X | $R^4$ | $R^5$ | physik. Daten |
|---|---|---|---|---|---|---|---|---|---|
| 96 | C-O-$CH_2$-O | | CH | N | CH | O | H | 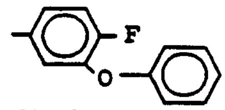 | |
| 97 | C-O-$CH_2$-O | | CH | N | CH | O | H | 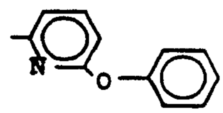 | |
| 98 | C-O-$CH_2$-O | | CH | N | CH | O | CN | 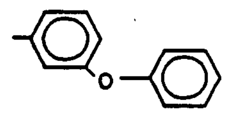 | |
| 99 | C-O-$CH_2$-O | | CH | N | CH | O | $CH_3$ | 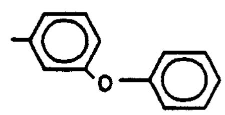 | |
| 100 | CH | EtO | N | CH | CH | $CH_2$ | H | 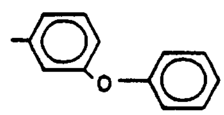 | $Kp_{0.04}$ = 230-245°C |
| 101 | CH | EtO | N | CH | CH | $CH_2$ | H | 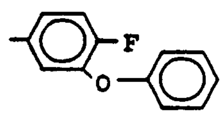 | |
| 102 | CH | EtO | N | CH | CH | $CH_2$ | H | 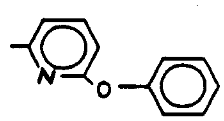 | |
| 103 | CH | EtO | N | CH | CH | $CH_2$ | H | 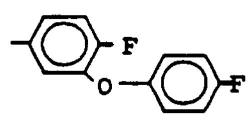 | |
| 104 | CH | EtO | N | CH | CH | $CH_2$ | CN | 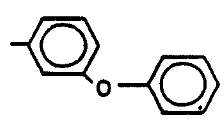 | |
| 105 | CH | EtO | N | CH | CH | $CH_2$ | CN | 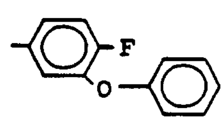 | |

Fortsetzung Tabelle 1

| Verbdg. Nr. | A | $R^1$ | B | C' | D | X | $R^4$ | $R^5$ | physik. Daten |
|---|---|---|---|---|---|---|---|---|---|
| 106 | CH | EtO | N | CH | CH | O | H | 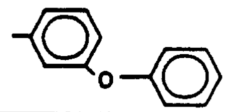 | |
| 107 | CH | EtO | N | CH | CH | O | H | 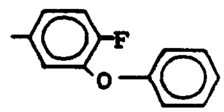 | |
| 108 | CH | EtO | N | CH | CH | O | H | 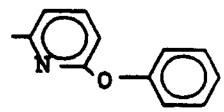 | |
| 109 | CH | EtO | N | CH | CH | O | CN | 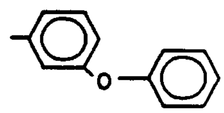 | |
| 110 | CH | EtO | N | CH | CH | O | $CH_3$ | 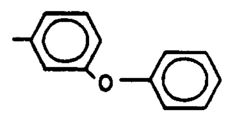 | |
| 111 | CH | Cl | N | CH | CH | $CH_2$ | H | 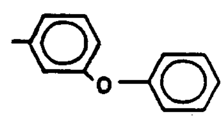 | |
| 112 | CH | Cl | N | CH | CH | $CH_2$ | H | 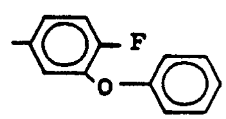 | |
| 113 | CH | Cl | N | CH | CH | $CH_2$ | H | 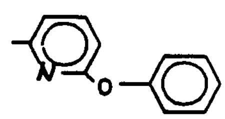 | |
| 114 | CH | Cl | N | CH | CH | $CH_2$ | H | 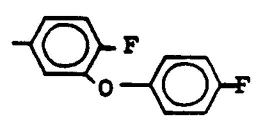 | |
| 115 | CH | Cl | N | CH | CH | $CH_2$ | CN | 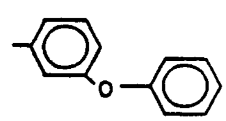 | |

Fortsetzung Tabelle 1

| Verbdg. Nr. | A | $R^1$ | B | C' | D | X | $R^4$ | $R^5$ | physik. Daten |
|---|---|---|---|---|---|---|---|---|---|
| 116 | CH | Cl | N | CH | CH | $CH_2$ | CN | 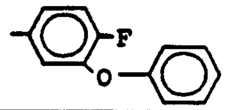 | |
| 117 | CH | Cl | N | CH | CH | O | H | 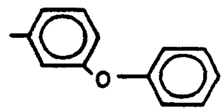 | |
| 118 | CH | Cl | N | CH | CH | O | H | 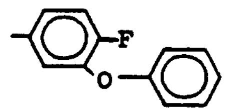 | |
| 119 | CH | Cl | N | CH | CH | O | H | 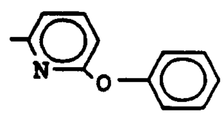 | |
| 120 | CH | Cl | N | CH | CH | O | CN | 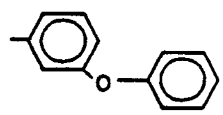 | |
| 121 | CH | Cl | N | CH | CH | O | $CH_3$ | 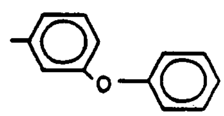 | |
| 122 | CH | $H_3CO$ | N | CH | CH | $CH_2$ | H | 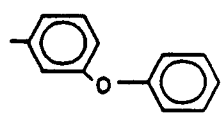 | |
| 123 | CH | $H_3CO$ | N | CH | CH | CH2 | H | 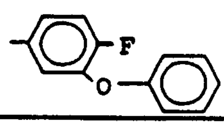 | |
| 124 | CH | $H_3CO$ | N | CH | CH | $CH_2$ | H | 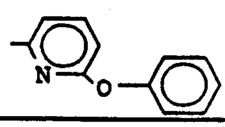 | |
| 125 | CH | $H_3CO$ | N | CH | CH | $CH_2$ | H | 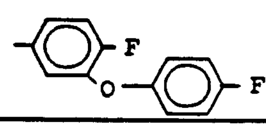 | |

Fortsetzung Tabelle 1

| Verbdg. Nr. | A | $R^1$ | B | C' | D | X | $R^4$ | $R^5$ | physik. Daten |
|---|---|---|---|---|---|---|---|---|---|
| 126 | CH | $H_3CO$ | N | CH | CH | $CH_2$ | CN | 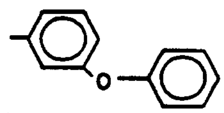 | |
| 127 | CH | $H_3CO$ | N | CH | CH | $CH_2$ | CN | 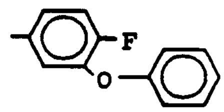 | |
| 128 | CH | $H_3CO$ | N | CH | CH | O | H | 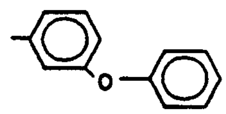 | |
| 129 | CH | $H_3CO$ | N | CH | CH | O | H | 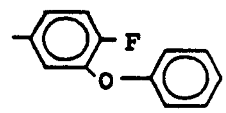 | |
| 130 | CH | $H_3CO$ | N | CH | CH | O | H | 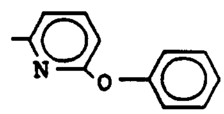 | |
| 131 | CH | $H_3CO$ | N | CH | CH | O | CN | 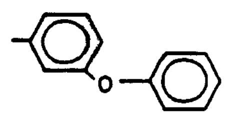 | |
| 132 | CH | $H_3CO$ | N | CH | CH | O | $CH_3$ | 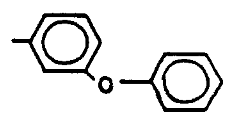 | |
| 133 | CH | EtS | N | CH | CH | $CH_2$ | H | 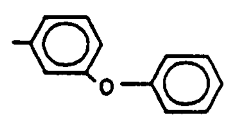 | |
| 134 | CH | EtS | N | CH | CH | $CH_2$ | H | 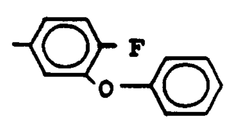 | |
| 135 | CH | EtS | N | CH | CH | $CH_2$ | H | 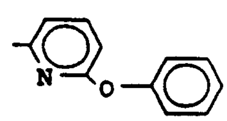 | |

Fortsetzung Tabelle 1

| Verbdg. Nr. | A | $R^1$ | B | C' | D | X | $R^4$ | $R^5$ | physik. Daten |
|---|---|---|---|---|---|---|---|---|---|
| 136 | CH | EtS | N | CH | CH | $CH_2$ | H | 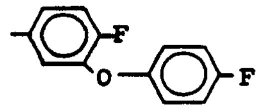 | |
| 137 | CH | EtS | N | CH | CH | $CH_2$ | CN | 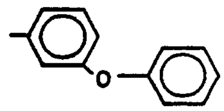 | |
| 138 | CH | EtS | N | CH | CH | $CH_2$ | CN | 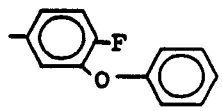 | |
| 139 | CH | EtS | N | CH | CH | O | H | 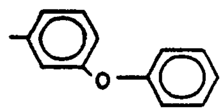 | |
| 140 | CH | EtS | N | CH | CH | O | H | 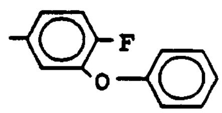 | |
| 141 | CH | EtS | N | CH | CH | O | H | 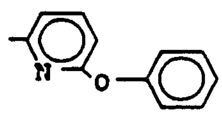 | |
| 142 | CH | EtS | N | CH | CH | O | CN | 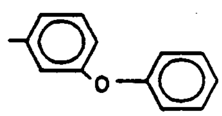 | |
| 143 | CH | EtS | N | CH | CH | O | $CH_3$ | 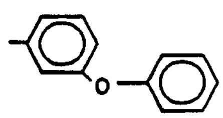 | |
| 144 | CH | $F_2CHO$ | N | CH | CH | $CH_2$ | H | 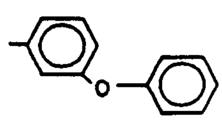 | |
| 145 | CH | $F_2CHO$ | N | CH | CH | $CH_2$ | H | 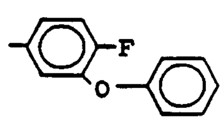 | |

Fortsetzung Tabelle 1

| Verbdg. Nr. | A | $R^1$ | B | C' | D | X | $R^4$ | $R^5$ | physik. Daten |
|---|---|---|---|---|---|---|---|---|---|
| 146 | CH | $F_2CHO$ | N | CH | CH | $CH_2$ | H | 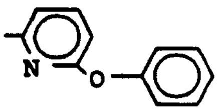 | |
| 147 | CH | $F_2CHO$ | N | CH | CH | $CH_2$ | H | 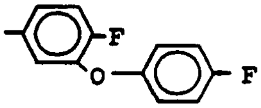 | |
| 148 | CH | $F_2CHO$ | N | CH | CH | $CH_2$ | CN | 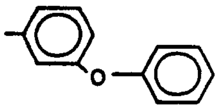 | |
| 149 | CH | $F_2CHO$ | N | CH | CH | $CH_2$ | CN | 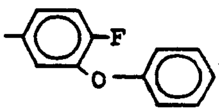 | |
| 150 | CH | $F_2CHO$ | N | CH | CH | O | H | 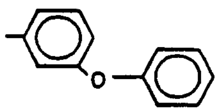 | |
| 151 | CH | $F_2CHO$ | N | CH | CH | O | H | 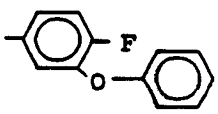 | |
| 152 | CH | $F_2CHO$ | N | CH | CH | O | H | 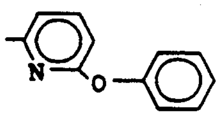 | |
| 153 | CH | $F_2CHO$ | N | CH | CH | O | CN | 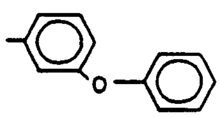 | |
| 154 | CH | $F_2CHO$ | N | CH | CH | O | $CH_3$ | 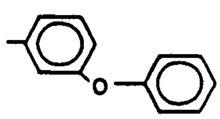 | |
| 155 | CCl | CL | N | CH | CH | $CH_2$ | H | 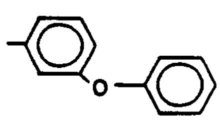 | |

**Fortsetzung Tabelle 1**

| Verbdg. Nr. | A | $R^1$ | B | C' | D | X | $R^4$ | $R^5$ | physik. Daten |
|---|---|---|---|---|---|---|---|---|---|
| 156 | CCl | Cl | N | CH | CH | $CH_2$ | H | 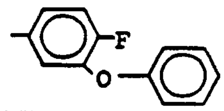 | |
| 157 | CCl | Cl | N | CH | CH | $CH_2$ | H | 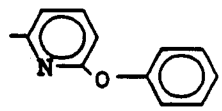 | |
| 158 | CCl | Cl | N | CH | CH | $CH_2$ | H | 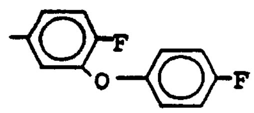 | |
| 159 | CCl | Cl | N | CH | CH | $CH_2$ | CN | 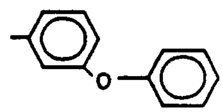 | |
| 160 | CCl | Cl | N | CH | CH | $CH_2$ | CN | 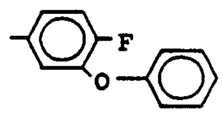 | |
| 161 | CCl | Cl | N | CH | CH | O | H | 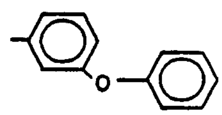 | |
| 162 | CCl | Cl | N | CH | CH | O | H | 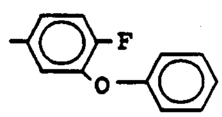 | |
| 163 | CCl | Cl | N | CH | CH | O | H | 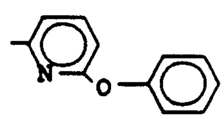 | |
| 164 | CCl | Cl | N | CH | CH | O | CN | 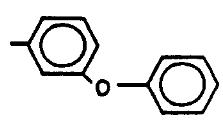 | |
| 165 | CCl | Cl | N | CH | CH | O | $CH_3$ | 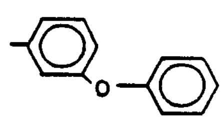 | |

Fortsetzung Tabelle 1

| Verbdg. Nr. | A | $R^1$ | B | C' | D | X | $R^4$ | $R^5$ | physik. Daten |
|---|---|---|---|---|---|---|---|---|---|
| 166 | CCl | EtO | N | CH | CH | $CH_2$ | H | 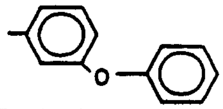 | |
| 167 | CCl | EtO | N | CH | CH | $CH_2$ | H | 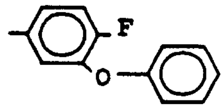 | |
| 168 | CCl | EtO | N | CH | CH | $CH_2$ | H | 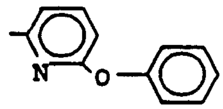 | |
| 169 | CCl | EtO | N | CH | CH | $CH_2$ | H | 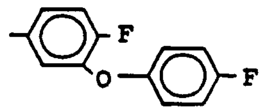 | |
| 170 | CCl | EtO | N | CH | CH | $CH_2$ | CN | 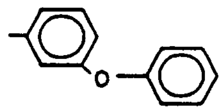 | |
| 171 | CCl | EtO | N | CH | CH | $CH_2$ | CN | 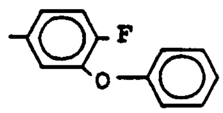 | |
| 172 | CCl | EtO | N | CH | CH | O | H | 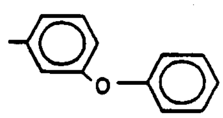 | |
| 173 | CCl | EtO | N | CH | CH | O | H | 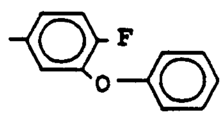 | |
| 174 | CCl | EtO | N | CH | CH | O | H | 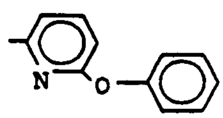 | |
| 175 | CCl | EtO | N | CH | CH | O | CN | 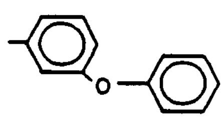 | |

Fortsetzung Tabelle 1

| Verbdg. Nr. | A | $R^1$ | B | C' | D | X | $R^4$ | $R^5$ | physik. Daten |
|---|---|---|---|---|---|---|---|---|---|
| 176 | CCl | EtO | N | CH | CH | O | $CH_3$ | 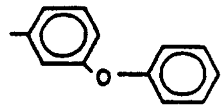 | |
| 177 | C-O-$CH_2$-O | | N | CH | CH | $CH_2$ | H | 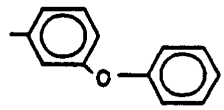 | |
| 178 | C-O-$CH_2$-O | | N | CH | CH | $CH_2$ | H | 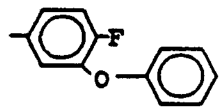 | |
| 179 | C-O-$CH_2$-O | | N | CH | CH | $CH_2$ | H | 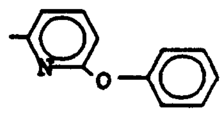 | |
| 180 | C-O-$CH_2$-O | | N | CH | CH | $CH_2$ | H | 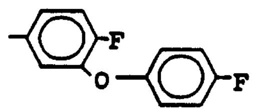 | |
| 181 | C-O-$CH_2$-O | | N | CH | CH | $CH_2$ | CN | 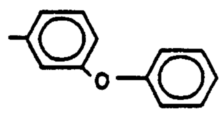 | |
| 182 | C-O-$CH_2$-O | | N | CH | CH | $CH_2$ | CN | 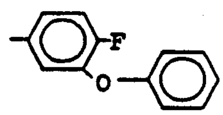 | |
| 183 | C-O-$CH_2$-O | | N | CH | CH | O | H | 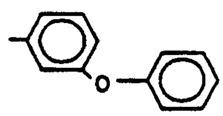 | |
| 184 | C-O-$CH_2$-O | | N | CH | CH | O | H | 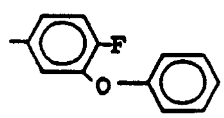 | |
| 185 | C-O-$CH_2$-O | | N | CH | CH | O | H | 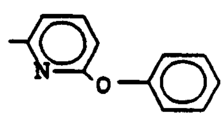 | |

**Fortsetzung Tabelle 1**

| Verbdg. Nr. | A | $R^1$ | B | C' | D | X | $R^4$ | $R^5$ | physik. Daten |
|---|---|---|---|---|---|---|---|---|---|
| 186 | C-O-$CH_2$-O | | N | CH | CH | O | CN | 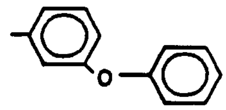 | |
| 187 | C-O-$CH_2$-O | | N | CH | CH | O | $CH_3$ | 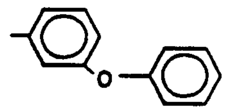 | |
| 188 | C-O-$CH_2$-O | | CH | CH | N | $CH_2$ | H | 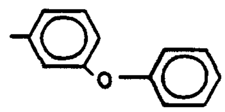 | |
| 189 | C-O-$CH_2$-O | | CH | CH | N | $CH_2$ | H | 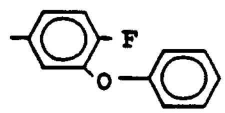 | |
| 190 | C-O-$CH_2$-O | | CH | CH | N | $CH_2$ | H | 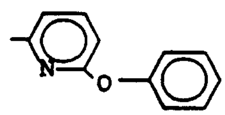 | |
| 191 | C-O-$CH_2$-O | | CH | CH | N | $CH_2$ | H | 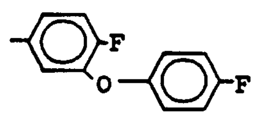 | |
| 192 | C-O-$CH_2$-O | | CH | CH | N | $CH_2$ | CN | 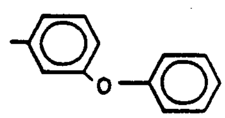 | |
| 193 | C-O-$CH_2$-O | | CH | CH | N | $CH_2$ | CN | 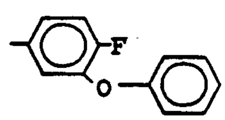 | |
| 194 | C-O-$CH_2$-O | | CH | CH | N | O | H | 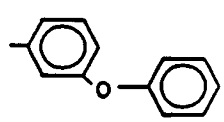 | |
| 195 | C-O-$CH_2$-O | | CH | CH | N | O | H | 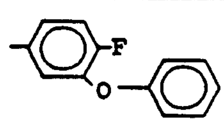 | |

Fortsetzung Tabelle 1

| Verbdg. Nr. | A | $R^1$ | B | C' | D | X | $R^4$ | $R^5$ | physik. Daten |
|---|---|---|---|---|---|---|---|---|---|
| 196 | C-O-$CH_2$-O | | CH | CH | N | O | H | 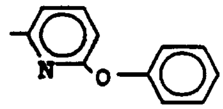 | |
| 197 | C-O-$CH_2$-O | | CH | CH | N | O | CN | 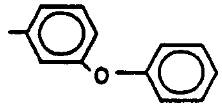 | |
| 198 | C-O-$CH_2$-O | | CH | CH | N | O | $CH_3$ | 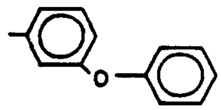 | |
| 199 | N | EtO | CH | N | CH | $CH_2$ | H | 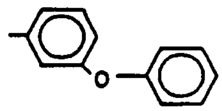 | $Kp_{0.005}$ = 230–245°C |
| 200 | N | EtO | CH | N | CH | $CH_2$ | H | 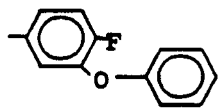 | $Kp_{0.01}$ = 230–240°C |
| 201 | N | EtO | CH | N | CH | $CH_2$ | H | 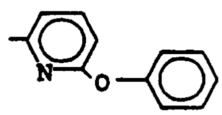 | |
| 202 | N | EtO | CH | N | CH | $CH_2$ | H | 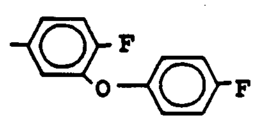 | |
| 203 | N | EtC | CH | N | CH | $CH_2$ | CN | 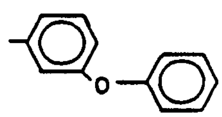 | |
| 204 | N | EtO | CH | N | CH | $CH_2$ | CN | 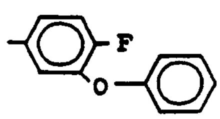 | |
| 205 | N | EtO | CH | N | CH | O | H | 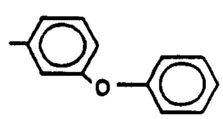 | $Kp_{0.05}$ = 230–240°C |

**Fortsetzung Tabelle 1**

| Verbdg. Nr. | A | $R^1$ | B | C' | D | X | $R^4$ | $R^5$ | physik. Daten |
|---|---|---|---|---|---|---|---|---|---|
| 206 | N | EtO | CH | N | CH | O | H | 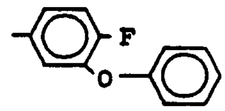 | $Kp_{0.05}$ = 230-235°C |
| 207 | N | EtO | CH | N | CH | O | H | 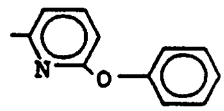 | |
| 208 | N | EtO | CH | N | CH | O | CN | 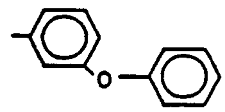 | |
| 209 | N | EtO | CH | N | CH | O | $CH_3$ | 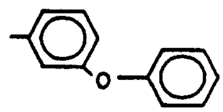 | |
| 210 | N | Cl | CH | N | CH | $CH_2$ | H | 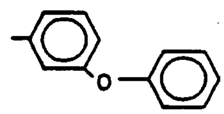 | |
| 211 | N | Cl | CH | N | CH | $CH_2$ | H | 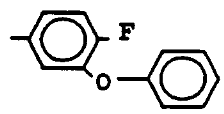 | |
| 212 | N | Cl | CH | N | CH | $CH_2$ | H | 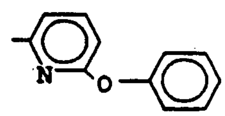 | |
| 213 | N | Cl | CH | N | CH | $CH_2$ | H | 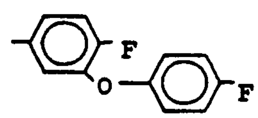 | |
| 214 | N | Cl | CH | N | CH | $CH_2$ | CN | 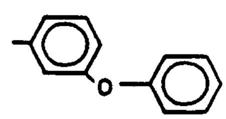 | |
| 215 | N | Cl | CH | N | CH | $CH_2$ | CN | 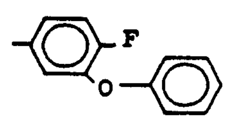 | |

Fortsetzung Tabelle 1

| Verbdg. Nr. | A | $R^1$ | B | C' | D | X | $R^4$ | $R^5$ | physik. Daten |
|---|---|---|---|---|---|---|---|---|---|
| 216 | N | Cl | CH | N | CH | O | H | 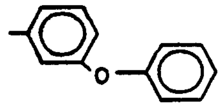 | |
| 217 | N | Cl | CH | N | CH | O | H | 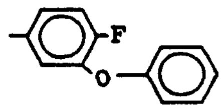 | |
| 218 | N | Cl | CH | N | CH | O | H | 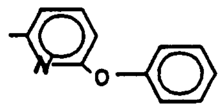 | |
| 219 | N | Cl | CH | N | CH | O | CN | 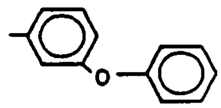 | |
| 220 | N | Cl | CH | N | CH | O | $CH_3$ | 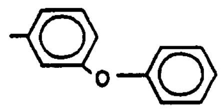 | |
| 221 | N | $H_3CO$ | CH | N | CH | $CH_2$ | H | 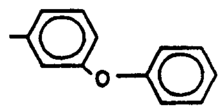 | |
| 222 | N | $H_3CO$ | CH | N | CH | $CH_3$ | H | 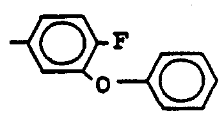 | |
| 223 | N | $H_3CO$ | CH | N | CH | $CH_2$ | H | 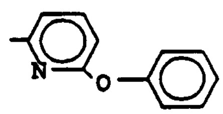 | |
| 224 | N | $H_3CO$ | CH | N | CH | $CH_2$ | H | 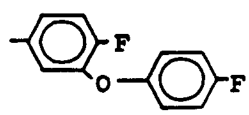 | |
| 225 | N | $H_3CO$ | CH | N | CH | $CH_2$ | CN | 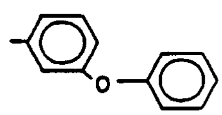 | |

Fortsetzung Tabelle 1

| Verbdg. Nr. | A | $R^1$ | B | C' | D | X | $R^4$ | $R^5$ | physik. Daten |
|---|---|---|---|---|---|---|---|---|---|
| 226 | N | $H_3CO$ | CH | N | CH | $CH_2$ | CN | 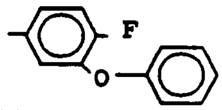 | |
| 227 | N | $H_3CO$ | CH | N | CH | O | H | 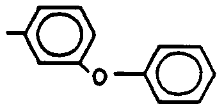 | |
| 228 | N | $H_3CO$ | CH | N | CH | O | H | 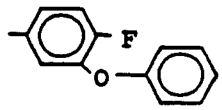 | |
| 229 | N | $H_3CO$ | CH | N | CH | O | H | 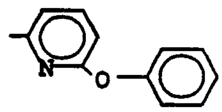 | |
| 230 | N | $H_3CO$ | CH | N | CH | O | CN | 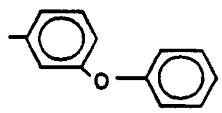 | |
| 231 | N | $H_3CO$ | CH | N | CH | O | $CH_3$ | 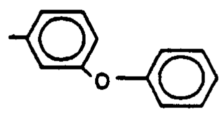 | |
| 232 | N | EtS | CH | N | CH | $CH_2$ | H | 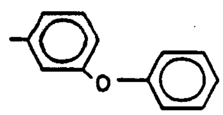 | blaß-gelbes Öl |
| 233 | N | EtS | CH | N | CH | $CH_2$ | H | 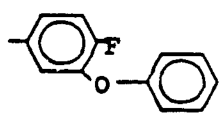 | blaß-gelbes Öl |
| 234 | N | EtS | CH | N | CH | $CH_2$ | H | 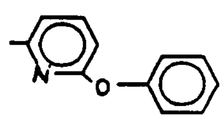 | |
| 235 | N | EtS | CH | N | CH | $CH_2$ | H | 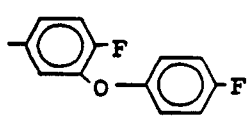 | |

Fortsetzung Tabelle 1

| Verbdg. Nr. | A | $R^1$ | B | C' | D | X | $R^4$ | $R^5$ | physik. Daten |
|---|---|---|---|---|---|---|---|---|---|
| 236 | N | EtS | CH | N | CH | $CH_2$ | CN | 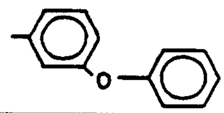 | |
| 237 | N | EtS | CH | N | CH | $CH_2$ | CN | 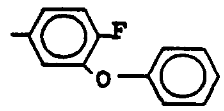 | |
| 238 | N | EtS | CH | N | CH | O | H | 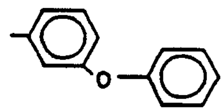 | blaß-gelbes Öl |
| 239 | N | EtS | CH | N | CH | O | H | 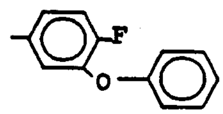 | blaß-gelbes Öl |
| 240 | N | EtS | CH | N | CH | O | H | 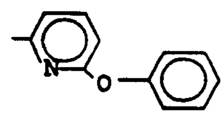 | |
| 241 | N | EtS | CH | N | CH | O | CN | 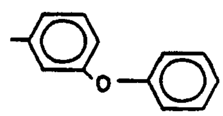 | |
| 242 | N | EtS | CH | N | CH | O | $CH_3$ | 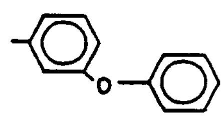 | |
| 243 | N | n-$C_3H_7$ | CH | N | CH | $CH_2$ | H | 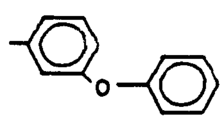 | |
| 244 | N | n-$C_3H_7$ | CH | N | CH | $CH_2$ | H | 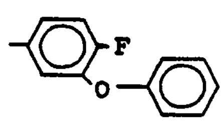 | |
| 245 | N | n-$C_3H_7$ | CH | N | CH | $CH_2$ | H | 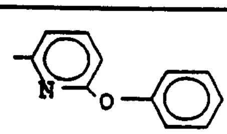 | |

Fortsetzung Tabelle 1

| Verbdg. Nr. | A | $R^1$ | B | C' | D | X | $R^4$ | $R^5$ | physik. Daten |
|---|---|---|---|---|---|---|---|---|---|
| 247 | N | n-$C_3H_7$ | CH | N | CH | $CH_2$ | CN | 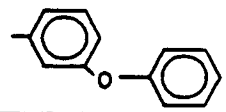 | |
| 248 | N | n-$C_3H_7$ | CH | N | CH | $CH_2$ | CN | 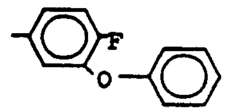 | |
| 249 | N | n-$C_3H_7$ | CH | N | CH | O | H | 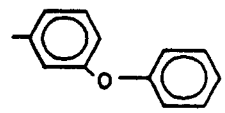 | |
| 250 | N | n-$C_3H_7$ | CH | N | CH | O | H | 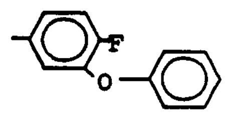 | |
| 251 | N | n-$C_3$H7 | CH | N | CH | O | H | 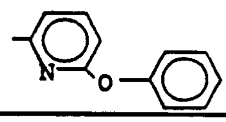 | |
| 252 | N | n-$C_3H_7$ | CH | N | CH | O | CN | 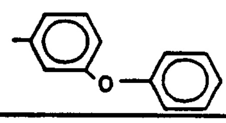 | |
| 253 | N | n-$C_3H_7$ | CH | N | CH | O | $CH_3$ | 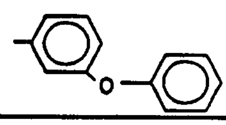 | |
| 254 | CH | EtO | N | CH | N | $CH_2$ | H | 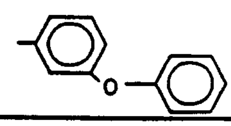 | $Kp_{0.05}$ = 225-245°C |
| 255 | CH | EtO | N | CH | N | $CH_2$ | H | 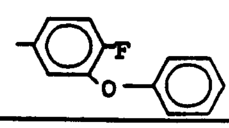 | |
| 256 | CH | EtO | N | CH | N | $CH_2$ | H | 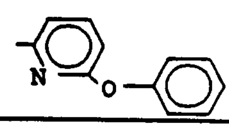 | |

Fortsetzung Tabelle 1

| Verbdg. Nr. | A | $R^1$ | B | C' | D | X | $R^4$ | $R^5$ | physik. Daten |
|---|---|---|---|---|---|---|---|---|---|
| 257 | CH | EtO | N | CH | N | $CH_2$ | H | 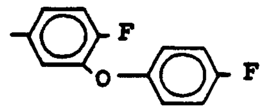 | |
| 258 | CH | EtO | N | CH | N | $CH_2$ | CN | 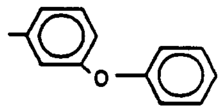 | |
| 259 | CH | EtO | N | CH | N | $CH_2$ | CN | 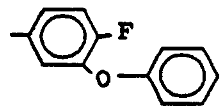 | |
| 260 | CH | EtO | N | CH | N | O | H | 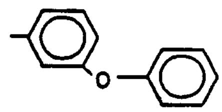 | $Kp_{0.03}$ = 230°C |
| 261 | CH | EtO | N | CH | N | O | H | 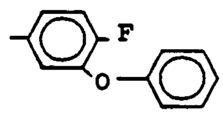 | |
| 262 | CH | EtO | N | CH | N | O | H | 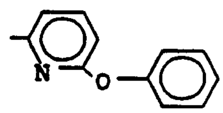 | |
| 263 | CH | EtO | N | CH | N | O | CN | 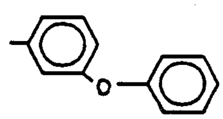 | |
| 264 | CH | EtO | N | CH | N | O | $CH_3$ | 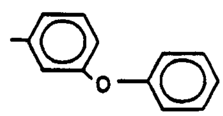 | |
| 265 | CH | Cl | N | CH | N | $CH_2$ | H | 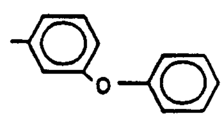 | |
| 266 | CH | Cl | N | CH | N | $CH_2$ | H | 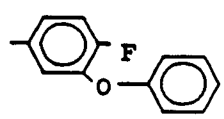 | |

**Fortsetzung Tabelle 1**

| Verbdg. Nr. | A | $R^1$ | B | C' | D | X | $R^4$ | $R^5$ | physik. Daten |
|---|---|---|---|---|---|---|---|---|---|
| 267 | CH | Cl | N | CH | N | $CH_2$ | H | 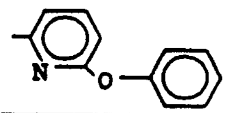 | |
| 268 | CH | Cl | N | CH | N | $CH_2$ | H | 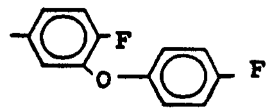 | |
| 269 | CH | Cl | N | CH | N | $CH_2$ | CN | 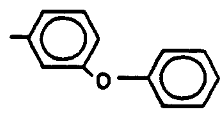 | |
| 270 | CH | Cl | N | CH | N | $CH_2$ | CN | 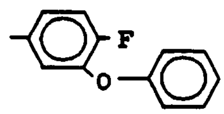 | |
| 271 | CH | Cl | N | CH | N | O | H | 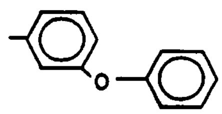 | |
| 272 | CH | Cl | N | CH | N | O | H | 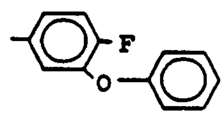 | |
| 273 | CH | Cl | N | CH | N | O | H | 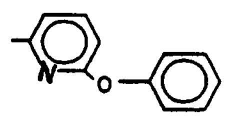 | |
| 274 | CH | Cl | N | CH | N | O | CN | 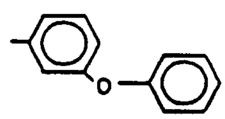 | |
| 275 | CH | Cl | N | CH | N | O | $CH_3$ | 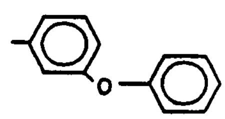 | |
| 276 | CH | $H_3CO$ | N | CH | N | $CH_2$ | H | 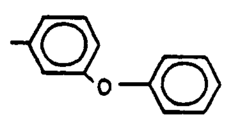 | |

**Fortsetzung Tabelle 1**

| Verbdg. Nr. | A | $R^1$ | B | C' | D | X | $R^4$ | $R^5$ | physik. Daten |
|---|---|---|---|---|---|---|---|---|---|
| 277 | CH | $H_3CO$ | N | CH | N | $CH_2$ | H | 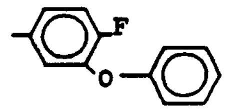 | |
| 278 | CH | $H_3CO$ | N | CH | N | $CH_2$ | H | 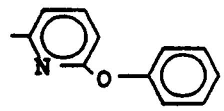 | |
| 279 | CH | $H_3CO$ | N | CH | N | $CH_2$ | H | 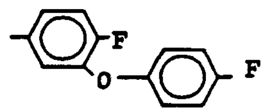 | |
| 280 | CH | $H_3CO$ | N | CH | N | $CH_2$ | CN | 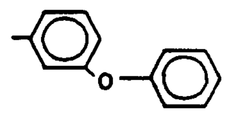 | |
| 281 | CH | $H_3CO$ | N | CH | N | $CH_2$ | CN | 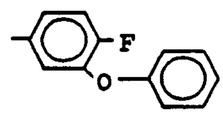 | |
| 282 | CH | $H_3CO$ | N | CH | N | O | H | 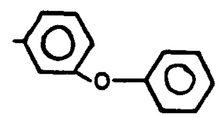 | |
| 283 | CH | $H_3CO$ | N | CH | N | O | H | 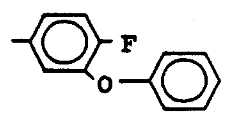 | |
| 284 | CH | $H_3CO$ | N | CH | N | O | H | 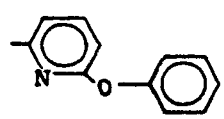 | |
| 285 | CH | $H_3CO$ | N | CH | N | O | CN | 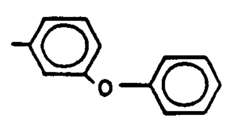 | |
| 286 | CH | $H_3CO$ | N | CH | N | O | $CH_3$ | 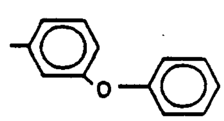 | |

Fortsetzung Tabelle 1

| Verbdg. Nr. | A | $R^1$ | B | C' | D | X | $R^4$ | $R^5$ | physik. Daten |
|---|---|---|---|---|---|---|---|---|---|
| 287 | CH | $F_2CHO$ | N | CH | N | $CH_2$ | H | 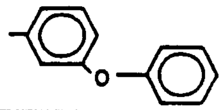 | |
| 288 | CH | $F_2CHO$ | N | CH | N | $CH_2$ | H | 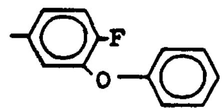 | |
| 289 | CH | $F_2CHO$ | N | CH | N | $CH_2$ | H | 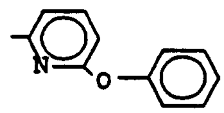 | |
| 290 | CH | $F_2CHO$ | N | CH | N | $CH_2$ | H | 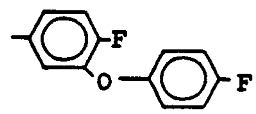 | |
| 291 | CH | $F_2CHO$ | N | CH | N | $CH_2$ | CN | 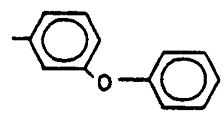 | |
| 292 | CH | $F_2CHO$ | N | CH | N | $CH_2$ | CN | 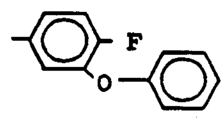 | |
| 293 | CH | $F_2CHO$ | N | CH | N | O | H | 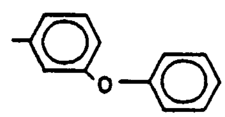 | |
| 294 | CH | $F_2CHO$ | N | CH | N | O | H | 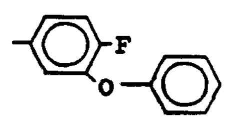 | |
| 295 | CH | $F_2CHO$ | N | CH | N | O | H | 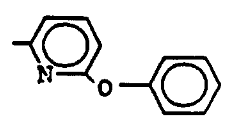 | |
| 296 | CH | $F_2CHO$ | N | CH | N | O | CN | 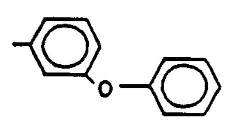 | |

Fortsetzung Tabelle 1

| Verbdg. Nr. | A | $R^1$ | B | C' | D | X | $R^4$ | $R^5$ | physik. Daten |
|---|---|---|---|---|---|---|---|---|---|
| 297 | CH | $F_2CHO$ | N | CH | N | O | $CH_3$ | 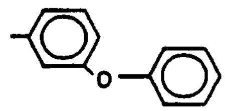 | |
| 298 | CH | EtS | N | CH | N | $CH_2$ | H | 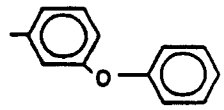 | |
| 299 | CH | EtS | N | CH | N | $CH_2$ | H | 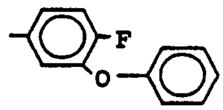 | |
| 300 | CH | EtS | N | CH | N | $CH_2$ | H | 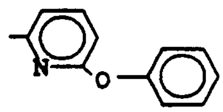 | |
| 301 | CH | EtS | N | CH | N | $CH_2$ | H | 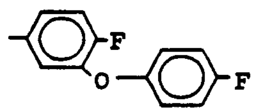 | |
| 302 | CH | EtS | N | CH | N | $CH_2$ | CN | 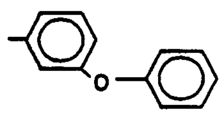 | |
| 303 | CH | EtS | N | CH | N | $CH_2$ | CN | 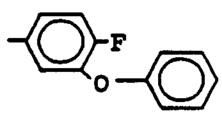 | |
| 304 | CH | EtS | N | CH | N | O | H | 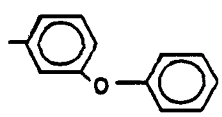 | |
| 305 | CH | EtS | N | CH | N | O | H | 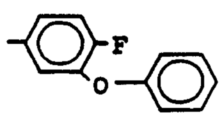 | |
| 306 | CH | EtS | N | CH | N | O | H | 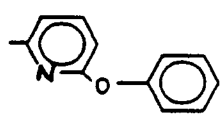 | |

**Fortsetzung Tabelle 1**

| Verbdg. Nr. | A | $R^1$ | B | C' | D | X | $R^4$ | $R^5$ | physik. Daten |
|---|---|---|---|---|---|---|---|---|---|
| 307 | CH | EtS | N | CH | N | O | CN | 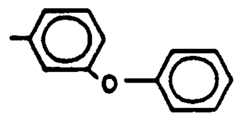 | |
| 308 | CH | EtS | N | CH | N | O | $CH_3$ | 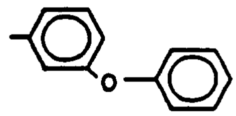 | |
| 309 | N | EtO | CH | CH | N | $CH_2$ | H | 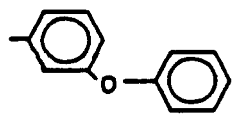 | |
| 310 | N | EtO | CH | CH | N | $CH_2$ | H | 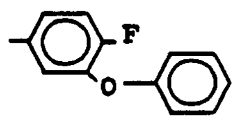 | |
| 311 | N | EtO | CH | CH | N | $CH_2$ | H | 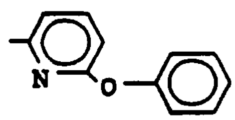 | |
| 312 | N | EtO | CH | CH | N | O | H | 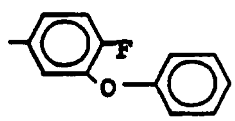 | blaß-gelbes Öl |
| 313 | N | EtO | CH | CH | N | O | H | 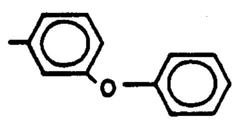 | blaß-gelbes Öl |
| 314 | N | Cl | CH | CH | N | $CH_2$ | H | 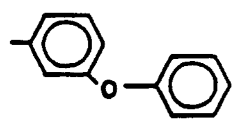 | |
| 315 | N | Cl | CH | CH | N | $CH_2$ | H | 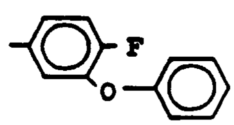 | |

**Fortsetzung Tabelle 1**

| Verbdg. Nr. | A | $R^1$ | B | C' | D | X | $R^4$ | $R^5$ | physik. Daten |
|---|---|---|---|---|---|---|---|---|---|
| 316 | N | Cl | CH | CH | N | $CH_2$ | H | 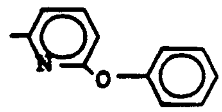 | |
| 317 | N | Cl | CH | CH | N | O | H | 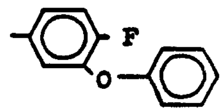 | |
| 318 | N | Cl | CH | CH | N | O | H | 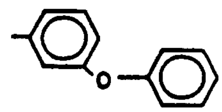 | |
| 319 | N | $CH_3O$ | CH | CH | N | $CH_2$ | H | 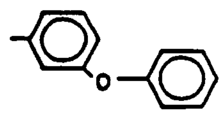 | |
| 320 | N | $CH_3O$ | CH | CH | N | $CH_2$ | H | 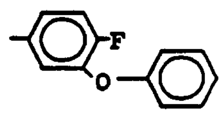 | |
| 321 | N | $CH_3O$ | CH | CH | N | $CH_2$ | H | 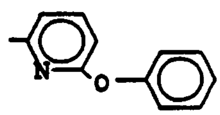 | |
| 322 | N | $CH_3O$ | CH | CH | N | O | H | 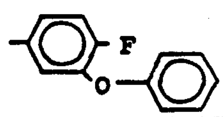 | |
| 323 | N | $CH_3O$ | CH | CH | N | O | H | 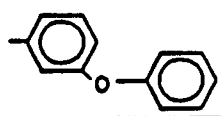 | |
| 324 | N | EtS | CH | CH | N | $CH_2$ | H | 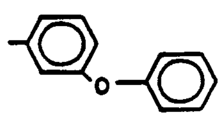 | |
| 325 | N | EtS | CH | CH | N | $CH_2$ | H | 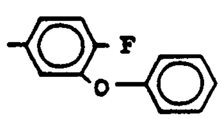 | |

**Fortsetzung Tabelle 1**

| Verbdg. Nr. | A | $R^1$ | B | C' | D | X | $R^4$ | $R^5$ | physik. Daten |
|---|---|---|---|---|---|---|---|---|---|
| 326 | N | EtS | CH | CH | N | $CH_2$ | H | 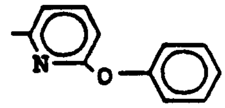 | |
| 327 | N | EtS | CH | CH | N | O | H | 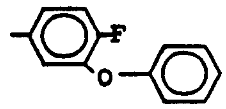 | |
| 328 | N | EtS | CH | CH | N | O | H | 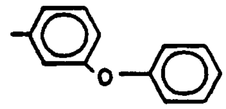 | |
| 329 | CCl | EtO | N | N | CH | $CH_2$ | H | 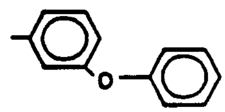 | |
| 330 | CCl | EtO | N | N | CH | $CH_2$ | H | 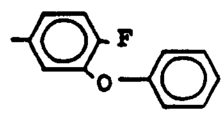 | |
| 331 | CCl | EtO | N | N | CH | $CH_2$ | H | 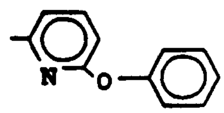 | |
| 332 | CCl | EtO | N | N | CH | O | H | 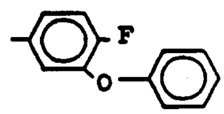 | |
| 333 | CCl | EtO | N | N | CH | O | H | 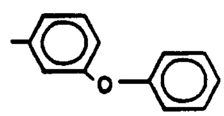 | |
| 334 | CCl | Cl | N | N | CH | $CH_2$ | H | 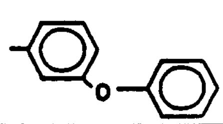 | |
| 335 | CCl | Cl | N | N | CH | $CH_2$ | H | 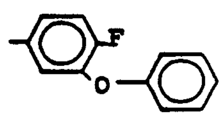 | |

**Fortsetzung Tabelle 1**

| Verbdg. Nr. | A | $R^1$ | B | C' | D | X | $R^4$ | $R^5$ | physik. Daten |
|---|---|---|---|---|---|---|---|---|---|
| 336 | CCl | Cl | N | N | CH | $CH_2$ | H | 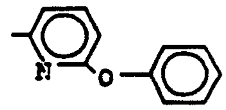 | |
| 337 | CCl | Cl | N | N | CH | O | H | 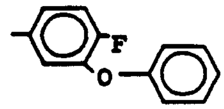 | |
| 338 | CCl | Cl | N | N | CH | O | H | 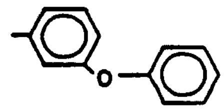 | |
| 339 | CH | EtO | CH | N | N | $CH_2$ | H | 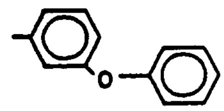 | |
| 340 | CH | EtO | CH | N | N | $CH_2$ | H | 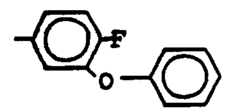 | |
| 341 | CH | EtO | CH | N | N | $CH_2$ | H | 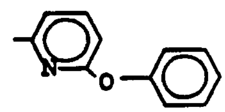 | |
| 342 | CH | EtO | CH | N | N | O | H | 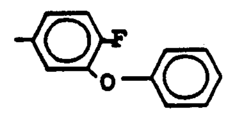 | blaßgelbes Öl |
| 343 | CH | EtO | CH | N | N | O | H | 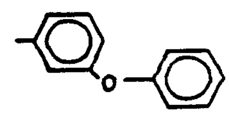 | blaßgelbes Öl |
| 344 | CH | $CH_3O$ | CH | N | N | $CH_2$ | H | 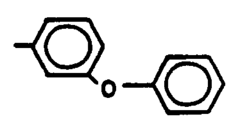 | |
| 345 | CH | $CH_3O$ | CH | N | N | $CH_2$ | H | 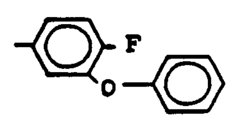 | |

**Fortsetzung Tabelle 1**

| Verbdg. Nr. | A | $R^1$ | B | C' | D | X | $R^4$ | $R^5$ | physik. Daten |
|---|---|---|---|---|---|---|---|---|---|
| 346 | CH | $CH_3O$ | CH | N | N | $CH_2$ | H | 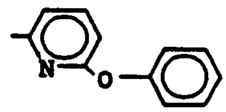 | |
| 347 | CH | $CH_3O$ | CH | N | N | O | H | 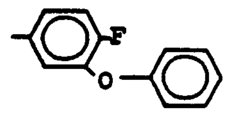 | |
| 348 | CH | $CH_3O$ | CH | N | N | O | H | 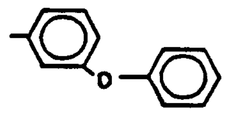 | |
| 349 | CH | Cl | CH | N | N | $CH_2$ | H | 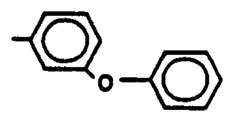 | |
| 350 | CH | Cl | CH | N | N | $CH_2$ | H | 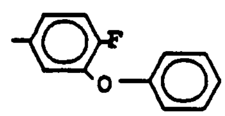 | |
| 351 | CH | Cl | CH | N | N | $CH_2$ | H | 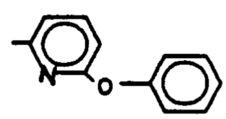 | |
| 352 | CH | Cl | CH | N | N | O | H | 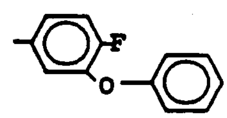 | |
| 353 | CH | Cl | CH | N | N | O | H | 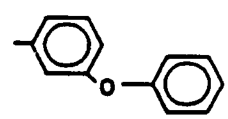 | |
| 354 | CH | EtS | CH | N | N | $CH_2$ | H | 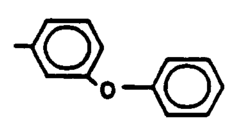 | |
| 355 | CH | EtS | CH | N | N | $CH_2$ | H | 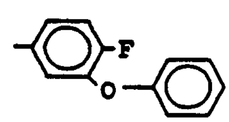 | |

**Fortsetzung Tabelle 1**

| Verbdg. Nr. | A | $R^1$ | B | C | D | X | $R^4$ | $R^5$ | physik. Daten |
|---|---|---|---|---|---|---|---|---|---|
| 356 | CH | EtS | CH | N | N | $CH_2$ | H | 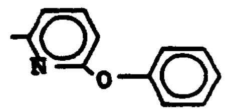 | |
| 357 | CH | EtS | CH | N | N | O | H | 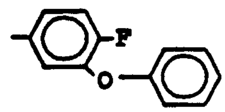 | blaß-gelbes Öl |
| 358 | CH | EtS | CH | N | N | O | H | 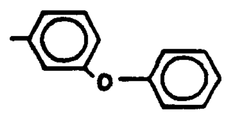 | blaß-gelbes Öl |
| 359 | CCl | Cl | CH | N | N | $CH_2$ | H | 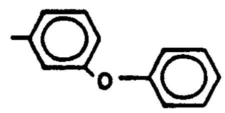 | |
| 360 | CCl | Cl | CH | N | N | $CH_2$ | H | 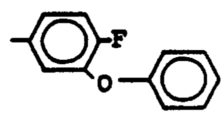 | |
| 361 | CCl | Cl | CH | N | N | $CH_2$ | H | 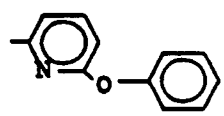 | |
| 362 | CCl | Cl | CH | N | N | O | H | 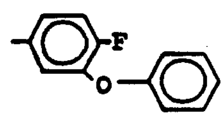 | |
| 363 | CCl | Cl | CH | N | N | O | H | 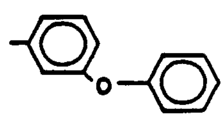 | |
| 364 | CH | EtO | N | N | N | $CH_2$ | H | 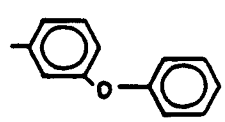 | |
| 365 | CH | EtO | N | N | N | $CH_2$ | H | 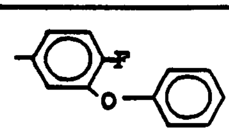 | |

**Fortsetzung Tabelle 1**

| Verbdg. Nr. | A | $R^1$ | B | C' | D | X | $R^4$ | $R^5$ | physik. Daten |
|---|---|---|---|---|---|---|---|---|---|
| 366 | CH | EtO | N | N | N | $CH_2$ | H | 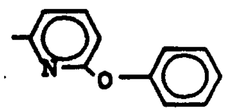 | |
| 367 | CH | EtO | N | N | N | O | H | 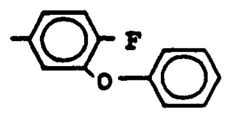 | |
| 368 | CH | EtO | N | N | N | O | H | 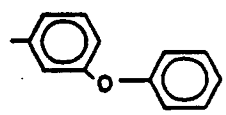 | |
| 369 | CH | $CH_3O$ | N | N | N | $CH_2$ | H | 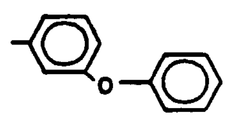 | |
| 370 | CH | $CH_3O$ | N | N | N | $CH_2$ | H | 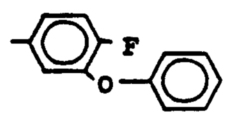 | |
| 371 | CH | $CH_3O$ | N | N | N | $CH_2$ | H | 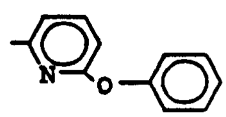 | |
| 372 | CH | $CH_3O$ | N | N | N | O | H | 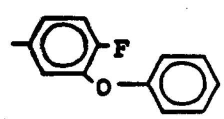 | |
| 373 | CH | $CH_3O$ | N | N | N | O | H | 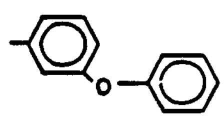 | |
| 374 | CH | Cl | N | N | N | $CH_2$ | H | 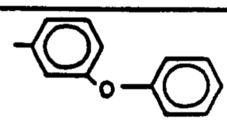 | |
| 375 | CH | Cl | N | N | N | $CH_2$ | H | 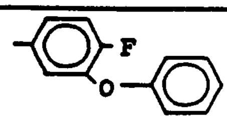 | |

**Fortsetzung Tabelle 1**

| Verbdg. Nr. | A | $R^1$ | B | C' | D | X | $R^4$ | $R^5$ | physik. Daten |
|---|---|---|---|---|---|---|---|---|---|
| 376 | CH | Cl | N | N | N | $CH_2$ | H | 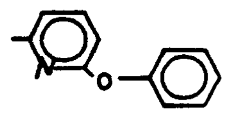 | |
| 377 | CH | Cl | N | N | N | O | H | 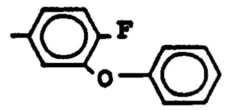 | |
| 378 | CH | Cl | N | N | N | O | H | 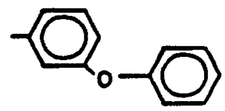 | |
| 379 | CH | EtS | N | N | N | $CH_2$ | H | 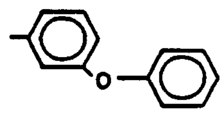 | |
| 380 | CH | EtS | N | N | N | $CH_2$ | H | 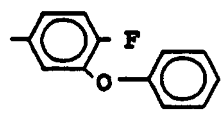 | |
| 381 | CH | EtS | N | N | N | $CH_2$ | H | 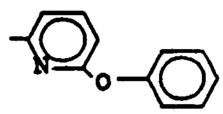 | |
| 382 | CH | EtS | N | N | N | O | H | 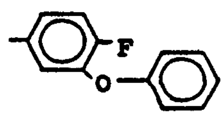 | |
| 383 | CH | EtS | N | N | N | O | H | 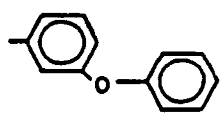 | |
| 384 | N | EtS | N | N | N | $CH_2$ | H | 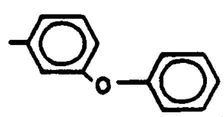 | |
| 385 | N | EtO | N | N | CH | $CH_2$ | H | 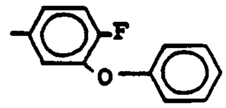 | |

**Fortsetzung Tabelle 1**

| Verbdg. Nr. | A | $R^1$ | B | C' | D | X | $R^4$ | $R^5$ | physik. Daten |
|---|---|---|---|---|---|---|---|---|---|
| 386 | N | EtO | N | N | CH | $CH_2$ | H | 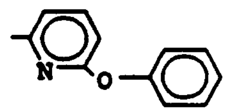 | |
| 387 | N | EtO | N | N | N | O | H | 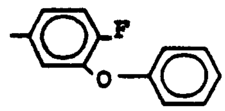 | |
| 388 | N | EtO | N | N | CH | O | H | 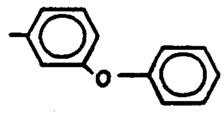 | |
| 389 | N | $CH_3O$ | N | N | CH | $CH_2$ | H | 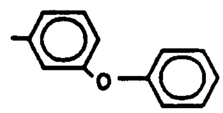 | |
| 390 | N | $CH_3O$ | N | N | CH | $CH_2$ | H | 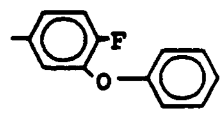 | |
| 391 | N | $CH_3O$ | N | N | CH | $CH_2$ | H | 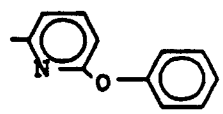 | |
| 392 | N | $CH_3O$ | N | N | CH | O | H | 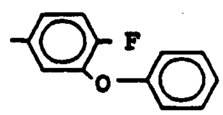 | |
| 393 | N | $CH_3O$ | N | N | CH | O | H | 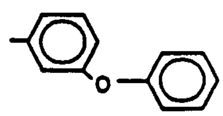 | |
| 394 | N | Cl | N | N | CH | $CH_2$ | H | 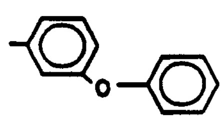 | |
| 395 | N | Cl | N | N | CH | $CH_2$ | H | 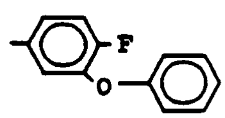 | |

**Fortsetzung Tabelle 1**

| Verbdg. Nr. | A | $R^1$ | B | C' | D | X | $R^4$ | $R^5$ | physik. Daten |
|---|---|---|---|---|---|---|---|---|---|
| 396 | N | Cl | N | N | CH | $CH_2$ | H | 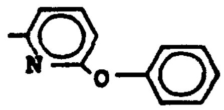 | |
| 397 | N | Cl | N | N | CH | O | H | 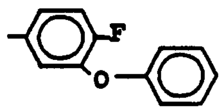 | |
| 398 | N | Cl | N | N | CH | O | H | 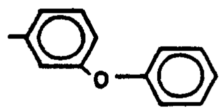 | |
| 399 | N | EtS | N | N | CH | $CH_2$ | H | 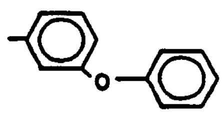 | |
| 400 | N | EtS | N | N | CH | $CH_2$ | H | 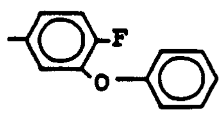 | |
| 401 | N | EtS | N | N | CH | $CH_2$ | H | 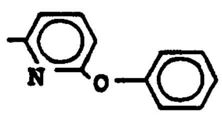 | |
| 402 | N | EtS | N | N | CH | O | H | 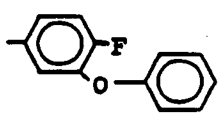 | |
| 403 | N | EtS | N | N | CH | O | H | 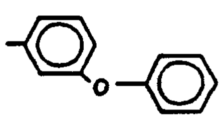 | |
| 404 | N | EtO | N | N | N | $CH_2$ | H | 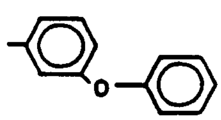 | |
| 405 | N | EtO | N | N | N | $CH_2$ | H | 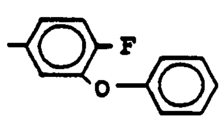 | |

**Fortsetzung Tabelle 1**

| Verbdg. Nr. | A | $R^1$ | B | C' | D | X | $R^4$ | $R^5$ | physik. Daten |
|---|---|---|---|---|---|---|---|---|---|
| 406 | N | EtO | N | N | N | $CH_2$ | H | 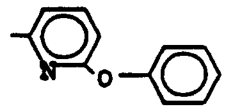 | |
| 407 | N | EtO | N | N | N | O | H | 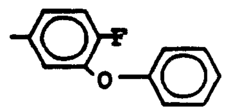 | |
| 408 | N | EtO | N | N | N | O | H | 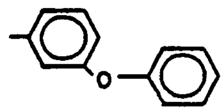 | |
| 409 | N | $CH_3O$ | N | N | N | $CH_2$ | H | 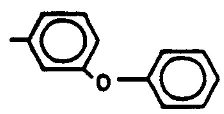 | |
| 410 | N | $CH_3O$ | N | N | N | $CH_2$ | H | 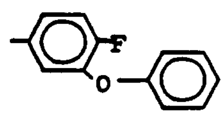 | |
| 411 | N | $CH_3O$ | N | N | N | $CH_2$ | H | 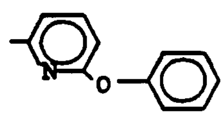 | |
| 412 | N | $CH_3O$ | N | N | N | O | H | 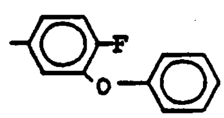 | |
| 413 | N | $CH_3O$ | N | N | N | O | H | 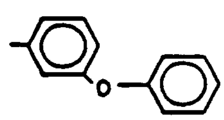 | |
| 414 | N | Cl | N | N | N | $CH_2$ | H | 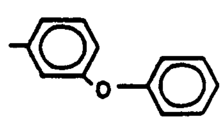 | |
| 415 | N | Cl | N | N | N | $CH_2$ | H | 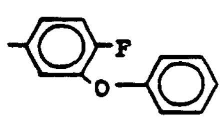 | |

**Fortsetzung Tabelle 1**

| Verbdg. Nr. | A | $R^1$ | B | C' | D | X | $R^4$ | $R^5$ | physik. Daten |
|---|---|---|---|---|---|---|---|---|---|
| 416 | N | Cl | N | N | N | $CH_2$ | H | 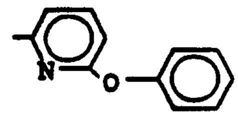 | |
| 417 | N | Cl | N | N | N | O | H | 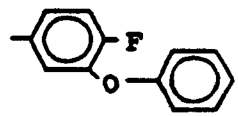 | |
| 418 | N | Cl | N | N | N | O | H | 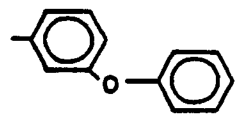 | |
| 419 | N | EtS | N | N | N | $CH_2$ | H | 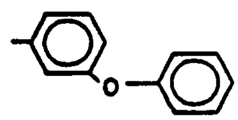 | |
| 420 | N | EtS | N | N | N | $CH_2$ | H | 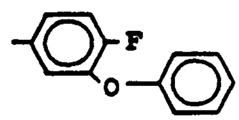 | |
| 421 | N | EtS | N | N | N | $CH_2$ | H | 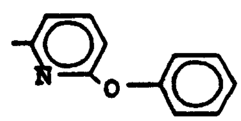 | |
| 422 | N | EtS | N | N | N | O | H | 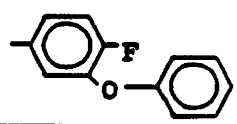 | |
| 423 | N | EtS | N | N | N | O | H | 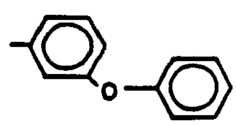 | |

Fortsetzung Tabelle 1

| Verbdg. Nr. | A | $R^1$ | B | C' | D | X | $R^4$ | $R^5$ | physik. Daten |
|---|---|---|---|---|---|---|---|---|---|
| 424 | N | $F_3C-CH_2-O-$ | CH | CH | CH | $CH_2$ | H | 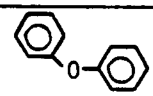 | Kp 0.01 =225-235°C |
| 425 | N | $F_3C-CH_2-O-$ | CH | CH | CH | $CH_2$ | H | 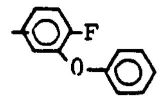 | Kp 0.01 = 230-235°C |
| 426 | N | $F_3C-CH_2-0-$ | CH | CH | CH | $CH_2$ | H | 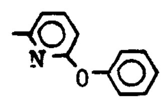 | |
| 427 | N | $F_3C-CH_2-0-$ | CH | CH | CH | $CH_2$ | H | 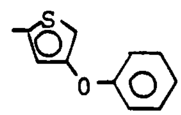 | |
| 428 | N | $F_3C-CH_2-0-$ | CH | CH | CH | $CH_2$ | H | 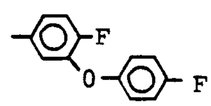 | |
| 429 | N | $F_3C-CH_2-0-$ | CH | CH | CH | 0 | H | 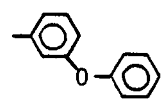 | Kp 0.02 =230-240°C |
| 430 | N | $F_3C-CH_2-0-$ | CH | CH | CH | 0 | H | 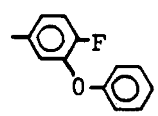 | Kp 0.04 =230-240°C |
| 431 | N | $F_3C-CH_2-0-$ | CH | CH | CH | 0 | H | 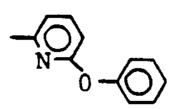 | |

Fortsetzung Tabelle 1

| Verbdg. Nr. | A | $R^1$ | B | C' | D | X | $R^4$ | $R^5$ | physik. Daten |
|---|---|---|---|---|---|---|---|---|---|
| 432 | CH | $F_3C-CH_2-O-$ | N | CH | CH | $CH_2$ | H | 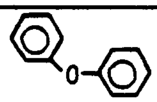 | |
| 433 | CH | $F_3C-CH_2-O-$ | N | CH | CH | $CH_2$ | H | 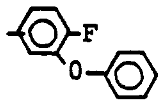 | |
| 434 | CH | $F_3C-CH_2-O-$ | N | CH | CH | $CH_2$ | H | 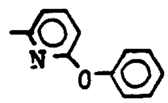 | |
| 435 | CH | $F_3C-CH_2-O-$ | N | CH | CH | $CH_2$ | H | 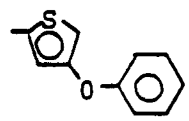 | |
| 436 | CH | $F_3C-CH_2-O-$ | N | CH | CH | $CH_2$ | H | 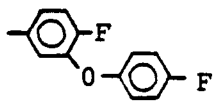 | |
| 437 | CH | $F_3C-CH_2-O-$ | N | CH | CH | O | H | 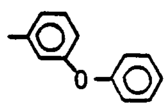 | |
| 438 | CH | $F_3C-CH_2-O-$ | N | CH | CH | O | H | 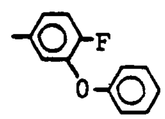 | |
| 439 | CH | $F_3C-CH_2-O-$ | N | CH | CH | O | H | 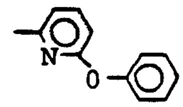 | |

Fortsetzung Tabelle 1

| Verbdg. Nr. | A | $R^1$ | B | C' | D | X | $R^4$ | $R^5$ | physik. Daten |
|---|---|---|---|---|---|---|---|---|---|
| 440 | N | $F_3C-CH_2-O-$ | CH | N | CH | $CH_2$ | H | 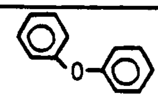 | Kp 0.01 =240-245°C |
| 441 | N | $F_3C-CH_2-O-$ | CH | N | CH | $CH_2$ | H | 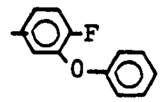 | Kp 0.01 =240-245°C |
| 442 | N | $F_3C-CH_2-O-$ | CH | N | CH | $CH_2$ | H | 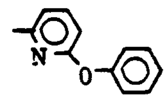 | |
| 443 | N | $F_3C-CH_2-O-$ | CH | N | CH | $CH_2$ | H | 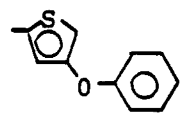 | |
| 444 | N | $F_3C-CH_2-O-$ | CH | N | CH | $CH_2$ | H | 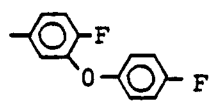 | |
| 445 | N | $F_3C-CH_2-O-$ | CH | N | CH | 0 | H | 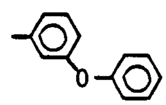 | blaßgelbes Öl |
| 446 | N | $F_3C-CH_2-O-$ | CH | N | CH | 0 | H | 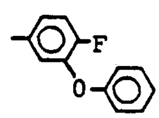 | blaßgelbes Öl |
| 447 | N | $F_3C-CH_2-O-$ | CH | N | CH | 0 | H | 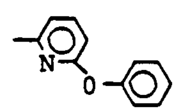 | |

Fortsetzung Tabelle 1

| Verbdg. Nr. | A | $R^1$ | B | C' | D | X | $R^4$ | $R^5$ | physik. Daten |
|---|---|---|---|---|---|---|---|---|---|
| 448 | CH | $F_3C-CH_2-O-$ | N | CH | N | $CH_2$ | H | 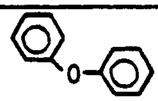 | |
| 449 | CH | $F_3C-CH_2-O-$ | N | CH | N | $CH_2$ | H | 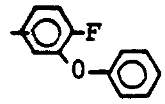 | |
| 450 | CH | $F_3C-CH_2-O-$ | N | CH | N | $CH_2$ | H | 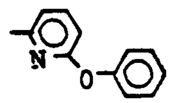 | |
| 451 | CH | $F_3C-CH_2-O-$ | N | CH | N | $CH_2$ | H | 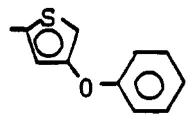 | |
| 452 | CH | $F_3C-CH_2-O-$ | N | CH | N | $CH_2$ | H | 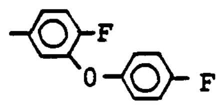 | |
| 453 | CH | $F_3C-CH_2-O-$ | N | CH | N | 0 | H | 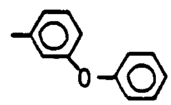 | |
| 454 | CH | $F_3C-CH_2-O-$ | N | CH | N | 0 | H | 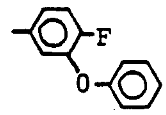 | |
| 455 | CH | $F_3C-CH_2-O-$ | N | CH | N | 0 | H | 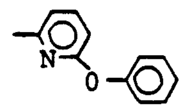 | |

Fortsetzung Tabelle 1

| Verbdg. Nr. | A | $R^1$ | B | C' | D | X | $R^4$ | $R^5$ | physik. Daten |
|---|---|---|---|---|---|---|---|---|---|
| 456 | N | $F_3C-CH_2-O-$ | N | CH | CH | $CH_2$ | H | 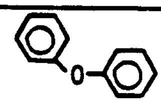 | blaßgelbes Öl |
| 457 | N | $F_3C-CH_2-O-$ | N | CH | CH | $CH_2$ | H | 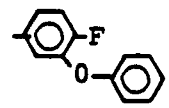 | blaßgelbes Öl |
| 458 | N | $F_3C-CH_2-O-$ | N | CH | CH | $CH_2$ | H | 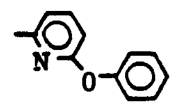 | |
| 459 | N | $F_3C-CH_2-O-$ | N | CH | CH | $CH_2$ | H | 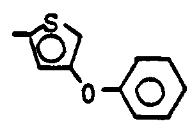 | |
| 460 | N | $F_3C-CH_2-O-$ | N | CH | CH | $CH_2$ | H | 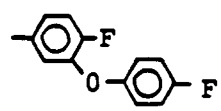 | |
| 461 | N | $F_3C-CH_2-O-$ | N | CH | CH | O | H | 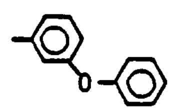 | blaßgelbes Öl |
| 462 | N | $F_3C-CH_2-O-$ | N | CH | CH | O | H | 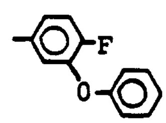 | blaßgelbes Öl |
| 463 | N | $F_3C-CH_2-O-$ | N | CH | CH | O | H | 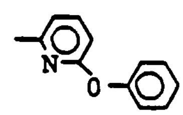 | |

Fortsetzung Tabelle 1

| Verbdg. Nr. | A | $R^1$ | B | C' | D | X | $R^4$ | $R^5$ | physik. Daten |
|---|---|---|---|---|---|---|---|---|---|
| 464 | N | $F_3C-CH_2-O-$ | CH | CH | N | $CH_2$ | H | 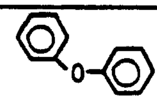 | blaßgelbes Öl |
| 465 | N | $F_3C-CH_2-O-$ | CH | CH | N | $CH_2$ | H | 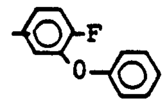 | blaßgelbes Öl |
| 466 | N | $F_3C-CH_2-O-$ | CH | CH | N | $CH_2$ | H | 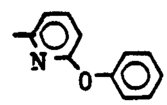 | |
| 467 | N | $F_3C-CH_2-O-$ | CH | CH | N | $CH_2$ | H | 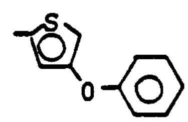 | |
| 468 | N | $F_3C-CH_2-O-$ | CH | CH | N | $CH_2$ | H | 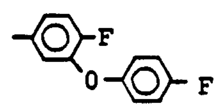 | |
| 469 | N | $F_3C-CH_2-O-$ | CH | CH | N | 0 | H | 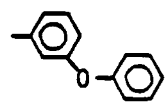 | blaßgelbes Öl |
| 470 | N | $F_3C-CH_2-O-$ | CH | CH | N | 0 | H | 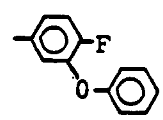 | blaßgelbes Öl |
| 471 | N | $F_3C-CH_2-O-$ | CH | CH | N | 0 | H | 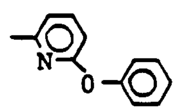 | |

Fortsetzung Tabelle 1

| Verbdg. Nr. | A | $R^1$ | B | C' | D | X | $R^4$ | $R^5$ | physik. Daten |
|---|---|---|---|---|---|---|---|---|---|
| 472 | N | $F_3C-CH_2-O-$ | N | N | CH | $CH_2$ | H | 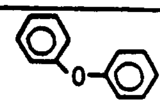 | |
| 473 | N | $F_3C-CH_2-O-$ | N | N | CH | $CH_2$ | H | 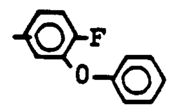 | |
| 474 | N | $F_3C-CH_2-O-$ | N | N | CH | $CH_2$ | H | 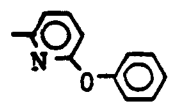 | |
| 475 | N | $F_3C-CH_2-O-$ | N | N | CH | $CH_2$ | H | 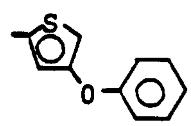 | |
| 476 | N | $F_3C-CH_2-O-$ | N | N | CH | $CH_2$ | H | 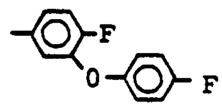 | |
| 477 | N | $F_3C-CH_2-O-$ | N | N | CH | O | H | 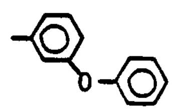 | |
| 478 | N | $F_3C-CH_2-O-$ | N | N | CH | O | H | 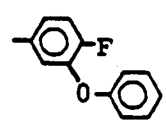 | |
| 479 | N | $F_3C-CH_2-O-$ | N | N | CH | O | H | 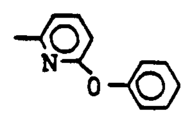 | |

Fortsetzung Tabelle 1

| Verbdg. Nr. | A | $R^1$ | B | C' | D | X | $R^4$ | $R^5$ | physik. Daten |
|---|---|---|---|---|---|---|---|---|---|
| 480 | N | $F_3C-CH_2-O-$ | N | CH | N | $CH_2$ | H | 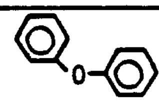 | |
| 481 | N | $F_3C-CH_2-O-$ | N | CH | N | $CH_2$ | H | 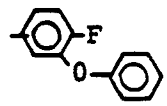 | |
| 482 | N | $F_3C-CH_2-O-$ | N | CH | N | $CH_2$ | H | 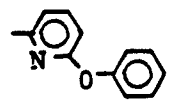 | |
| 483 | N | $F_3C-CH_2-O-$ | N | CH | N | $CH_2$ | H | 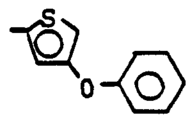 | |
| 484 | N | $F_3C-CH_2-O-$ | N | CH | N | $CH_2$ | H | 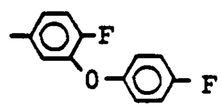 | |
| 485 | N | $F_3C-CH_2-O-$ | N | CH | N | O | H | 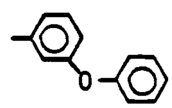 | |
| 486 | N | $F_3C-CH_2-O-$ | N | CH | N | O | H | 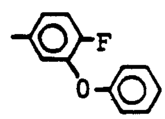 | |
| 487 | N | $F_3C-CH_2-O-$ | N | CH | N | O | H | 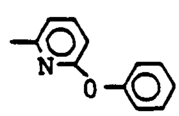 | |

Fortsetzung Tabelle 1

| Verbdg. Nr. | A | $R^1$ | B | C' | D | X | $R^4$ | $R^5$ | physik. Daten |
|---|---|---|---|---|---|---|---|---|---|
| 488 | N | $F_3C-CH_2-O-$ | N | N | N | $CH_2$ | H | 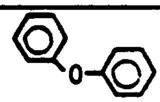 | |
| 489 | N | $F_3C-CH_2-O-$ | N | N | N | $CH_2$ | H | 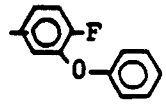 | |
| 490 | N | $F_3C-CH_2-O-$ | N | N | N | $CH_2$ | H | 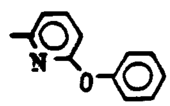 | |
| 491 | N | $F_3C-CH_2-O-$ | N | N | N | $CH_2$ | H | 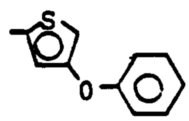 | |
| 492 | N | $F_3C-CH_2-O-$ | N | N | N | $CH_2$ | H | 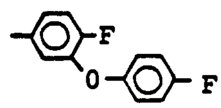 | |
| 493 | N | $F_3C-CH_2-O-$ | N | N | N | O | H | 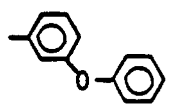 | |
| 494 | N | $F_3C-CH_2-O-$ | N | N | N | O | H | 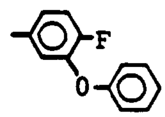 | |
| 495 | N | $F_3C-CH_2-O-$ | N | N | N | O | H | 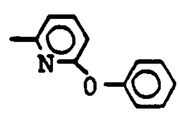 | |

Tabelle 2

Verbindungen der Formel I mit $R^2$,$R^3$ = $CH_2$-$CH_2$; A= N, CH oder $CR^1$

| Verbdg. Nr. | A | $R^1$ | B | C' | D | X | $R^4$ | $R^5$ | physik. Daten |
|---|---|---|---|---|---|---|---|---|---|
| 496 | N | EtO | CH | CH | CH | $CH_2$ | H | 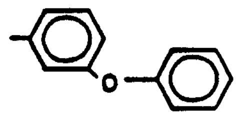 | |
| 497 | N | EtO | CH | CH | CH | $CH_2$ | H | 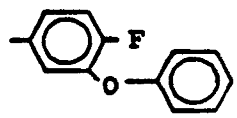 | |
| 498 | N | EtO | CH | CH | CH | $CH_2$ | H | 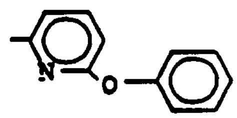 | |
| 4°9 | N | EtO | CH | CH | CH | $CH_2$ | H | 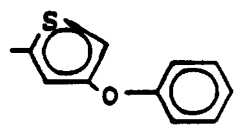 | |
| 500 | N | EtO | CH | CH | CH | $CH_2$ | H | 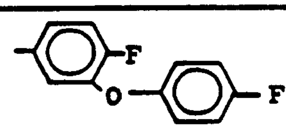 | |
| 501 | N | EtO | CH | CH | CH | $CH_2$ | CN | 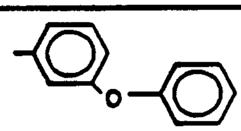 | |

**Fortsetzung Tabelle 2**

| Verbdg. Nr. | A | $R^1$ | B | C' | D | X | $R^4$ | $R^5$ | physik. Daten |
|---|---|---|---|---|---|---|---|---|---|
| 502 | N | EtO | CH | CH | CH | $CH_2$ | CN | 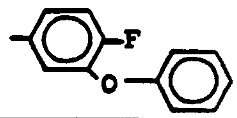 | |
| 503 | N | EtO | CH | CH | CH | O | H | 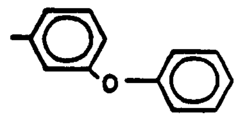 | blaß-gelbes Öl |
| 504 | N | EtO | CH | CH | CH | O | H | 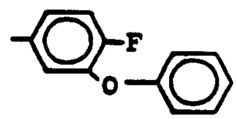 | |
| 505 | N | EtO | CH | CH | CH | O | H | 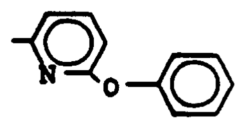 | |
| 506 | N | EtO | CH | CH | CH | O | CN | 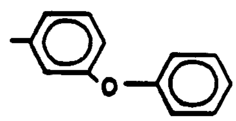 | |
| 507 | N | Cl | CH | CH | CH | $CH_2$ | H | 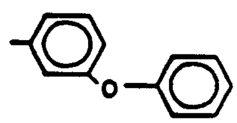 | |
| 508 | N | Cl | CH | CH | CH | $CH_2$ | H | 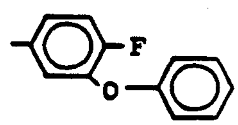 | |
| 509 | N | Cl | CH | CH | CH | $CH_2$ | H | 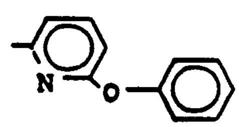 | |
| 510 | N | Cl | CH | CH | CH | $CH_2$ | H | 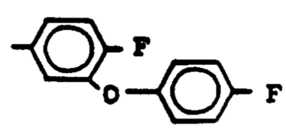 | |

**Fortsetzung Tabelle 2**

| Verbdg. Nr. | A | $R^1$ | B | C' | D | X | $R^4$ | $R^5$ | physik. Daten |
|---|---|---|---|---|---|---|---|---|---|
| 511 | N | Cl | CH | CH | CH | $CH_2$ | CN | 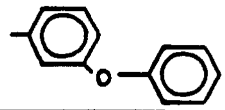 | |
| 512 | N | Cl | CH | CH | CH | $CH_2$ | CN | 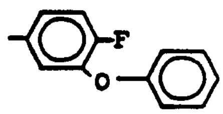 | |
| 513 | N | Cl | CH | CH | CH | O | H | 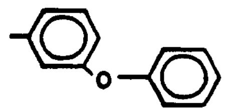 | blaß-glbes Öl |
| 514 | N | Cl | CH | CH | CH | O | H | 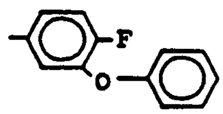 | |
| 515 | N | Cl | CH | CH | CH | O | H | 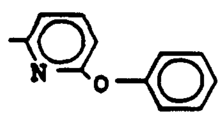 | |
| 516 | N | Cl | CH | CH | CH | O | CN | 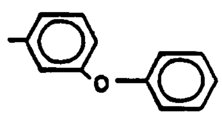 | |
| 517 | N | Cl | CH | CH | CH | O | $CH_3$ | 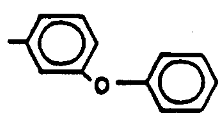 | |
| 518 | N | $H_3CO$ | CH | CH | CH | $CH_2$ | H | 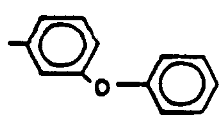 | |
| 519 | N | $H_3CO$ | CH | CH | CH | $CH_2$ | H | 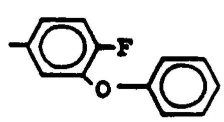 | |
| 520 | N | $H_3CO$ | CH | CH | CH | $CH_2$ | H | 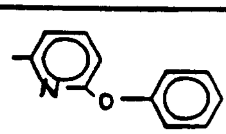 | |

**Fortsetzung Tabelle 2**

| Verbdg. Nr. | A | $R^1$ | B | C' | D | X | $R^4$ | $R^5$ | physik. Daten |
|---|---|---|---|---|---|---|---|---|---|
| 521 | N | $H_3CO$ | CH | CH | CH | $CH_2$ | H | 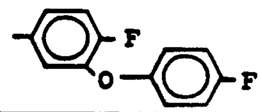 | |
| 522 | N | $H_3CO$ | CH | CH | CH | $CH_2$ | CN | 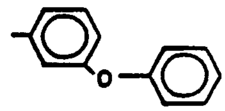 | |
| 523 | N | $H_3CO$ | CH | CH | CH | $CH_2$ | CN | 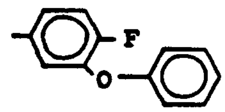 | |
| 524 | N | $H_3CO$ | CH | CH | CH | O | H | 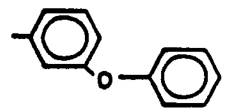 | |
| 525 | N | $H_3CO$ | CH | CH | CH | O | H | 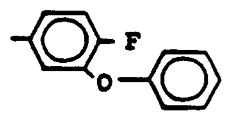 | |
| 526 | N | $H_3CO$ | CH | CH | CH | O | H | 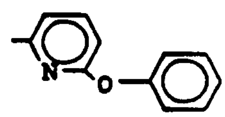 | |
| 527 | N | $H_3CO$ | CH | CH | CH | O | CN | 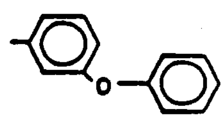 | |
| 528 | N | $H_3CO$ | CH | CH | CH | O | $CH_3$ | 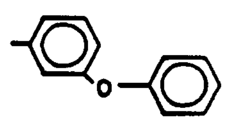 | |
| 529 | N | EtS | CH | CH | CH | $CH_2$ | H | 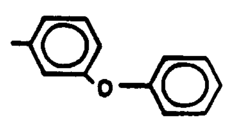 | |
| 530 | N | EtS | CH | CH | CH | $CH_2$ | H | 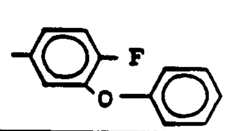 | |

**Fortsetzung Tabelle 1**

| Verbdg. Nr. | A | $R^1$ | B | C' | D | X | $R^4$ | $R^5$ | physik. Daten |
|---|---|---|---|---|---|---|---|---|---|
| 531 | N | EtS | CH | CH | CH | $CH_2$ | H | 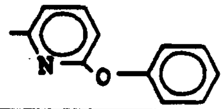 | |
| 532 | N | EtS | CH | CH | CH | $CH_2$ | H | 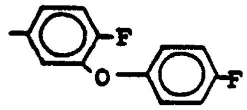 | |
| 533 | N | EtS | CH | CH | CH | $CH_2$ | CN | 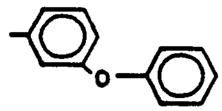 | |
| 534 | N | EtS | CH | CH | CH | $CH_2$ | CN | 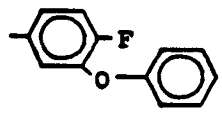 | |
| 535 | N | EtS | CH | CH | CH | O | H | 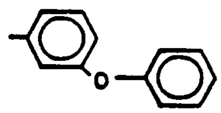 | |
| 536 | N | EtS | CH | CH | CH | O | H | 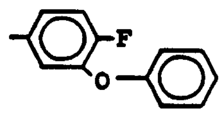 | |
| 537 | N | EtS | CH | CH | CH | O | H | 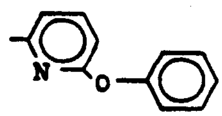 | |
| 538 | N | EtS | CH | CH | CH | O | CN | 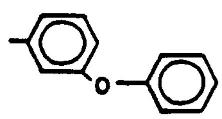 | |
| 539 | N | EtS | CH | CH | CH | O | $CH_3$ | 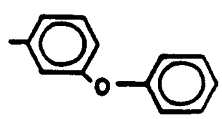 | |
| 540 | N | $H_3CS$ | CH | CH | CH | $CH_2$ | H | 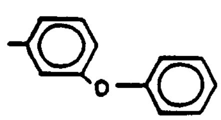 | |

Fortsetzung Tabelle 2

| Verbdg. Nr. | A | $R^1$ | B | C' | D | X | $R^4$ | $R^5$ | physik. Daten |
|---|---|---|---|---|---|---|---|---|---|
| 541 | N | $H_3CS$ | CH | CH | CH | $CH_2$ | H | 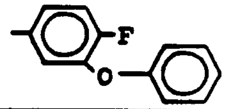 | |
| 542 | N | $H_3CS$ | CH | CH | CH | $CH_2$ | H | 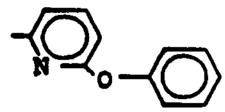 | |
| 543 | N | $H_3CS$ | CH | CH | CH | $CH_2$ | H | 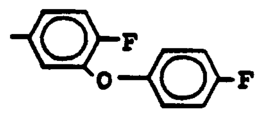 | |
| 544 | N | $H_3CS$ | CH | CH | CH | $CH_2$ | CN | 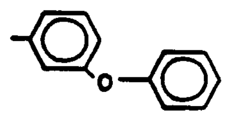 | |
| 545 | N | $H_3CS$ | CH | CH | CH | $CH_2$ | CN | 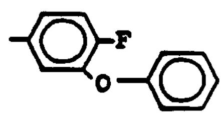 | |
| 546 | N | $H_3CS$ | CH | CH | CH | O | H | 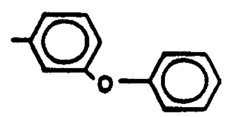 | |
| 547 | N | $H_3CS$ | CH | CH | CH | O | H | 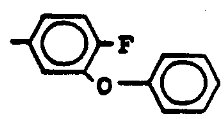 | |
| 548 | N | $H_3CS$ | CH | CH | CH | O | H | 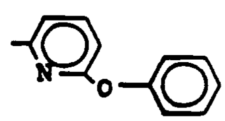 | |
| 549 | N | $H_3CS$ | CH | CH | CH | O | CN | 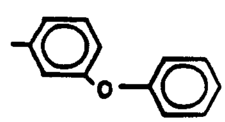 | |
| 550 | N | $H_3CS$ | CH | CH | CH | O | $CH_3$ | 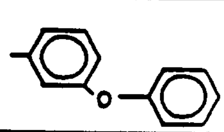 | |

Fortsetzung Tabelle 2

| Verbdg. Nr. | A | $R^1$ | B | C' | D | X | $R^4$ | $R^5$ | physik. Daten |
|---|---|---|---|---|---|---|---|---|---|
| 551 | CCl | Cl | CH | N | CH | $CH_2$ | H | 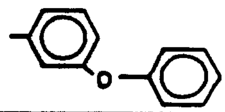 | |
| 552 | CCl | Cl | CH | N | CH | $CH_2$ | H | 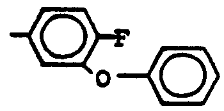 | |
| 553 | CCl | Cl | CH | N | CH | $CH_2$ | H | 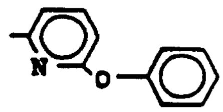 | |
| 554 | CCl | Cl | CH | N | CH | $CH_2$ | H | 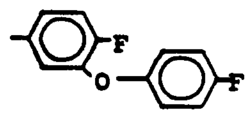 | |
| 555 | CCl | Cl | CH | N | CH | $CH_2$ | CN | 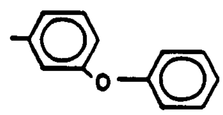 | |
| 556 | CCl | Cl | CH | N | CH | $CH_2$ | CN | 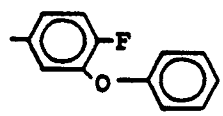 | |
| 557 | CCl | Cl | CH | N | CH | O | H | 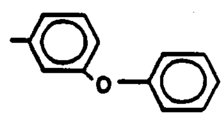 | |
| 558 | CCl | Cl | CH | N | CH | O | H | 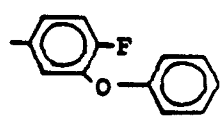 | |
| 559 | CCl | Cl | CH | N | CH | O | H | 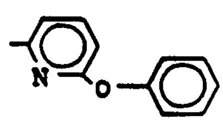 | |
| 560 | CCl | Cl | CH | N | CH | O | CN | 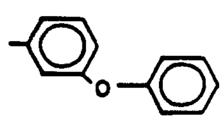 | |

**Fortsetzung Tabelle 2**

| Verbdg. Nr. | A | $R^1$ | B | C' | D | X | $R^4$ | $R^5$ | physik. Daten |
|---|---|---|---|---|---|---|---|---|---|
| 561 | CCl | Cl | CH | N | CH | O | $CH_3$ | 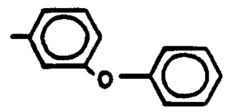 | |
| 562 | CCl | EtO | CH | N | CH | $CH_2$ | H | 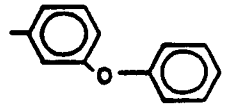 | |
| 563 | CCl | EtO | CH | N | CH | $CH_2$ | H | 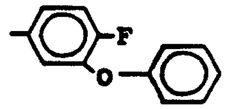 | |
| 564 | CCl | EtO | CH | N | CH | $CH_2$ | H | 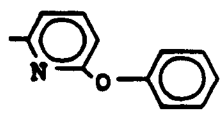 | |
| 565 | CCl | EtO | CH | N | CH | $CH_2$ | H | 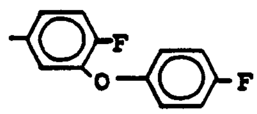 | |
| 566 | CCl | EtO | CH | N | CH | $CH_2$ | CN | 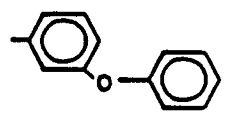 | |
| 567 | CCl | EtO | CH | N | CH | $CH_2$ | CN | 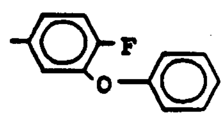 | |
| 568 | CCl | EtO | CH | N | CH | O | H | 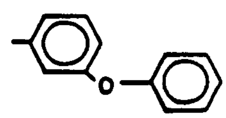 | |
| 569 | CCl | EtO | CH | N | CH | O | H | 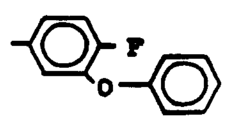 | |
| 570 | CCl | EtO | CH | N | CH | O | H | 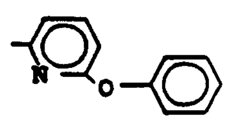 | |

**Fortsetzung Tabelle 2**

| Verbdg. Nr. | A | $R^1$ | B | C' | D | X | $R^4$ | $R^5$ | physik. Daten |
|---|---|---|---|---|---|---|---|---|---|
| 571 | CCl | EtO | CH | N | CH | O | CN | 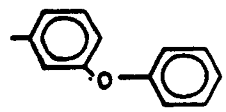 | |
| 572 | CCl | EtO | CH | N | CH | O | $CH_3$ | 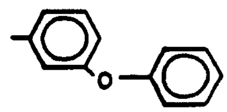 | |
| 573 | CF | EtO | CH | N | CH | $CH_2$ | H | 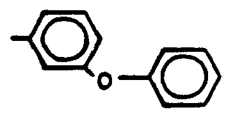 | |
| 574 | CF | EtO | CH | N | CH | $CH_2$ | H | 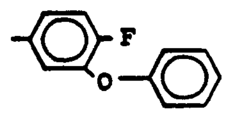 | |
| 575 | CF | EtO | CH | N | CH | $CH_2$ | H | 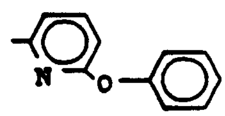 | |
| 576 | CF | EtO | CH | N | CH | $CH_2$ | H | 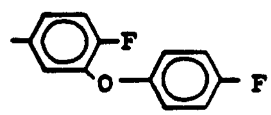 | |
| 577 | CF | EtO | CH | N | CH | $CH_2$ | CN | 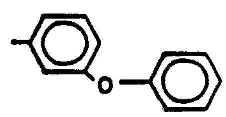 | |
| 578 | CF | EtO | CH | N | CH | $CH_2$ | CN | 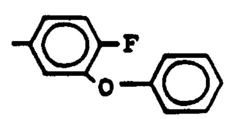 | |
| 579 | CF | EtO | CH | N | CH | O | H | 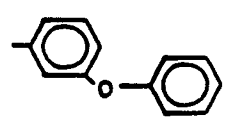 | |
| 580 | CF | EtO | CH | N | CH | O | H | 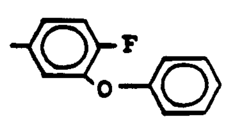 | |

Fortsetzung Tabelle 2

| Verbdg. Nr. | A | $R^1$ | B | C' | D | X | $R^4$ | $R^5$ | physik. Daten |
|---|---|---|---|---|---|---|---|---|---|
| 581 | CF | EtO | CH | N | CH | O | H | 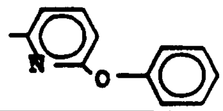 | |
| 582 | CF | EtO | CH | N | CH | O | CN | 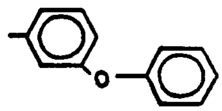 | |
| 583 | CF | EtO | CH | N | CH | O | $CH_3$ | 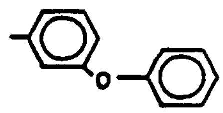 | |
| 584 | $C-O-CH_2-O$ | | CH | N | CH | $CH_2$ | H | 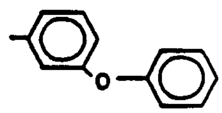 | |
| 585 | $C-O-CH_2-O$ | | CH | N | CH | $CH_2$ | H | 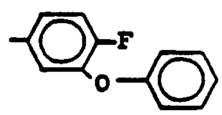 | |
| 586 | $C-O-CH_2-O$ | | CH | N | CH | $CH_2$ | H | 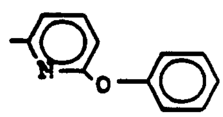 | |
| 587 | $C-O-CH_2-O$ | | CH | N | CH | $CH_2$ | H | 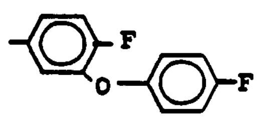 | |
| 588 | $C-O-CH_2-O$ | | CH | N | CH | $CH_2$ | CN | 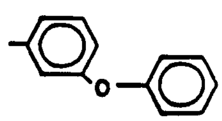 | |
| 589 | $C-O-CH_2-O$ | | CH | N | CH | $CH_2$ | CN | 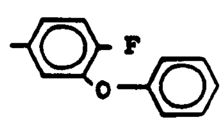 | |
| 590 | $C-O-CH_2-O$ | | CH | N | CH | O | H | 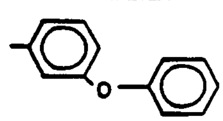 | |

**Fortsetzung Tabelle 2**

| Verbdg. Nr. | A | $R^1$ | B | C' | D | X | $R^4$ | $R^5$ | physik. Daten |
|---|---|---|---|---|---|---|---|---|---|
| 591 | C-O-$CH_2$-O | | CH | N | CH | O | H | 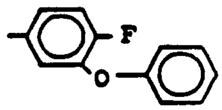 | |
| 592 | C-O-$CH_2$-O | | CH | N | CH | O | H | 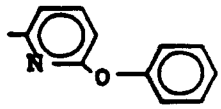 | |
| 593 | C-O-$CH_2$-O | | CH | N | CH | O | CN | 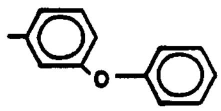 | |
| 594 | C-O-$CH_2$-O | | CH | N | CH | O | $CH_3$ | 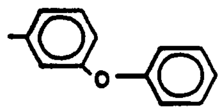 | |
| 595 | CH | EtO | N | CH | CH | $CH_2$ | H | 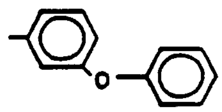 | |
| 596 | CH | EtO | N | CH | CH | $CH_2$ | H | 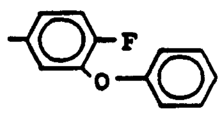 | |
| 597 | CH | EtO | N | CH | CH | $CH_2$ | H | 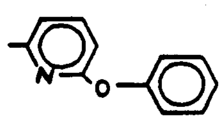 | |
| 598 | CH | EtO | N | CH | CH | $CH_2$ | H | 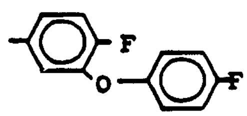 | |
| 599 | CH | EtO | N | CH | CH | $CH_2$ | CN | 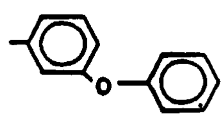 | |
| 600 | CH | EtO | N | CH | CH | $CH_2$ | CN | 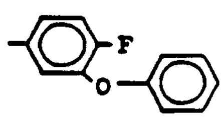 | |

Fortsetzung Tabelle 2

| Verbdg. Nr. | A | $R^1$ | B | C' | D | X | $R^4$ | $R^5$ | physik. Daten |
|---|---|---|---|---|---|---|---|---|---|
| 601 | CH | EtO | N | CH | CH | O | H | 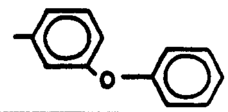 | |
| 602 | CH | EtO | N | CH | CH | O | H | 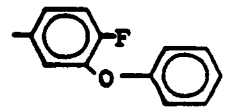 | |
| 603 | CH | EtO | N | CH | CH | O | H | 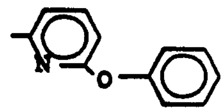 | |
| 604 | CH | EtO | N | CH | CH | O | CN | 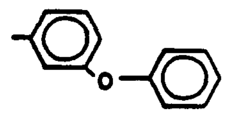 | |
| 605 | CH | EtO | N | CH | CH | O | $CH_3$ | 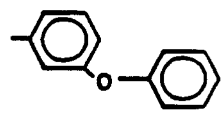 | |
| 606 | CH | Cl | N | CH | CH | $CH_2$ | H | 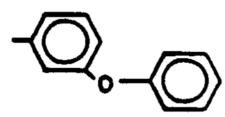 | |
| 607 | CH | Cl | N | CH | CH | $CH_2$ | H | 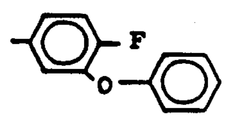 | |
| 608 | CH | Cl | N | CH | CH | $CH_2$ | H | 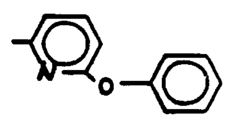 | |
| 609 | CH | Cl | N | CH | CH | $CH_2$ | H | 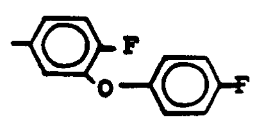 | |
| 610 | CH | Cl | N | CH | CH | $CH_2$ | CN | 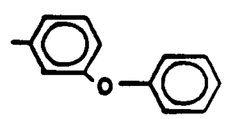 | |

**Fortsetzung Tabelle 2**

| Verbdg. Nr. | A | $R^1$ | B | C' | D | X | $R^4$ | $R^5$ | physik. Daten |
|---|---|---|---|---|---|---|---|---|---|
| 611 | CH | Cl | N | CH | CH | $CH_2$ | CN | 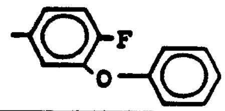 | |
| 612 | CH | Cl | N | CH | CH | O | H | 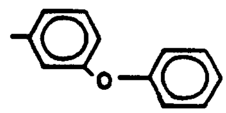 | |
| 613 | CH | Cl | N | CH | CH | O | H | 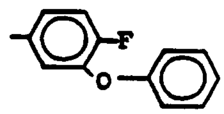 | |
| 614 | CH | Cl | N | CH | CH | O | H | 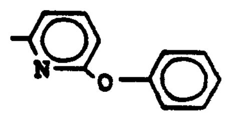 | |
| 615 | CH | Cl | N | CH | CH | O | CN | 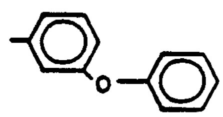 | |
| 616 | CH | Cl | N | CH | CH | O | $CH_3$ | 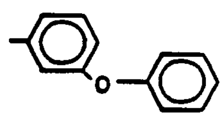 | |
| 617 | CH | $H_3CO$ | N | CH | CH | $CH_2$ | H | 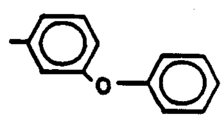 | |
| 618 | CH | $H_3CO$ | N | CH | CH | CH2 | H | 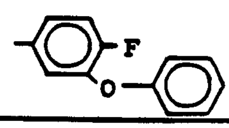 | |
| 619 | CH | $H_3CO$ | N | CH | CH | $CH_2$ | H | 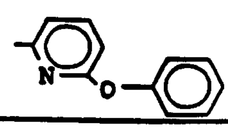 | |
| 620 | CH | $H_3CO$ | N | CH | CH | $CH_2$ | H | 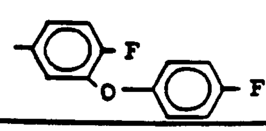 | |

**Fortsetzung Tabelle 2**

| Verbdg. Nr. | A | $R^1$ | B | C' | D | X | $R^4$ | $R^5$ | physik. Daten |
|---|---|---|---|---|---|---|---|---|---|
| 621 | CH | $H_3CO$ | N | CH | CH | $CH_2$ | CN | 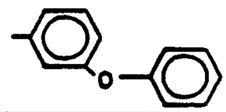 | |
| 622 | CH | $H_3CO$ | N | CH | CH | $CH_2$ | CN | 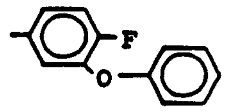 | |
| 623 | CH | $H_3CO$ | N | CH | CH | O | H | 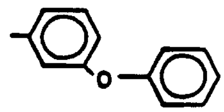 | |
| 624 | CH | $H_3CO$ | N | CH | CH | O | H | 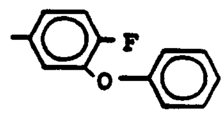 | |
| 625 | CH | $H_3CO$ | N | CH | CH | O | H | 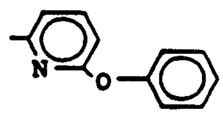 | |
| 626 | CH | $H_3CO$ | N | CH | CH | O | CN | 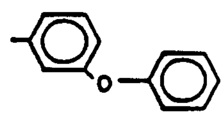 | |
| 627 | CH | $H_3CO$ | N | CH | CH | O | $CH_3$ | 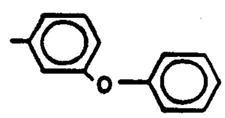 | |
| 628 | CH | EtS | N | CH | CH | $CH_2$ | H | 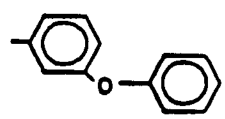 | |
| 629 | CH | EtS | N | CH | CH | $CH_2$ | H | 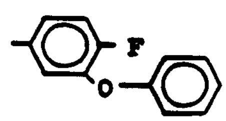 | |
| 630 | CH | EtS | N | CH | CH | $CH_2$ | H | 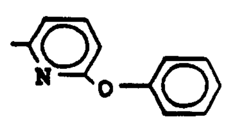 | |

Fortsetzung Tabelle 2

| Verbdg. Nr. | A | $R^1$ | B | C' | D | X | $R^4$ | $R^5$ | physik. Daten |
|---|---|---|---|---|---|---|---|---|---|
| 631 | CH | EtS | N | CH | CH | $CH_2$ | H | 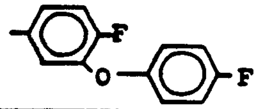 | |
| 632 | CH | EtS | N | CH | CH | $CH_2$ | CN | 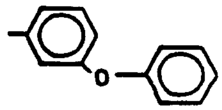 | |
| 633 | CH | EtS | N | CH | CH | $CH_2$ | CN | 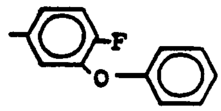 | |
| 634 | CH | EtS | N | CH | CH | O | H | 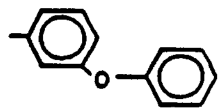 | |
| 635 | CH | EtS | N | CH | CH | O | H | 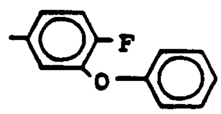 | |
| 636 | CH | EtS | N | CH | CH | O | H | 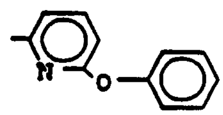 | |
| 637 | CH | EtS | N | CH | CH | O | CN | 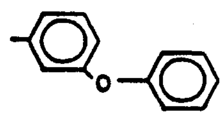 | |
| 638 | CH | EtS | N | CH | CH | O | $CH_3$ | 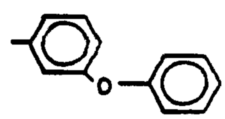 | |
| 639 | CH | $F_2CHO$ | N | CH | CH | $CH_2$ | H | 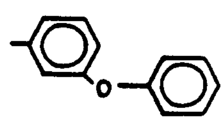 | |
| 640 | CH | $F_2CHO$ | N | CH | CH | $CH_2$ | H | 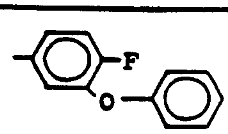 | |

**Fortsetzung Tabelle 2**

| Verbdg. Nr. | A | $R^1$ | B | C' | D | X | $R^4$ | $R^5$ | physik. Daten |
|---|---|---|---|---|---|---|---|---|---|
| 641 | CH | $F_2CHO$ | N | CH | CH | $CH_2$ | H | 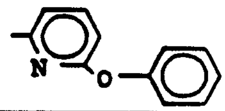 | |
| 642 | CH | $F_2CHO$ | N | CH | CH | $CH_2$ | H | 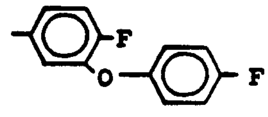 | |
| 643 | CH | $F_2CHO$ | N | CH | CH | $CH_2$ | CN | 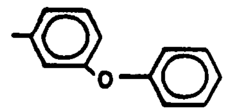 | |
| 644 | CH | $F_2CHO$ | N | CH | CH | $CH_2$ | CN | 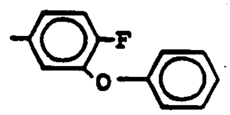 | |
| 645 | CH | $F_2CHO$ | N | CH | CH | O | H | 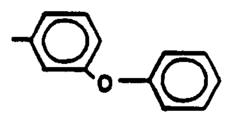 | |
| 646 | CH | $F_2CHO$ | N | CH | CH | O | H | 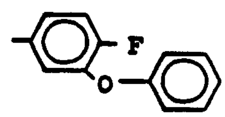 | |
| 647 | CH | $F_2CHO$ | N | CH | CH | O | H | 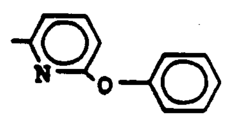 | |
| 648 | CH | $F_2CHO$ | N | CH | CH | O | CN | 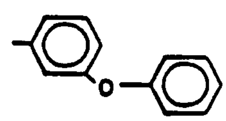 | |
| 649 | CH | $F_2CHO$ | N | CH | CH | O | $CH_3$ | 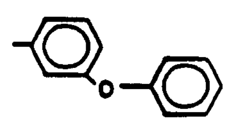 | |
| 650 | CCl | CL | N | CH | CH | $CH_2$ | H | 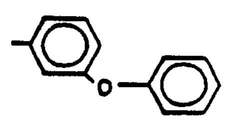 | |

**Fortsetzung Tabelle 2**

| Verbdg. Nr. | A | $R^1$ | B | C' | D | X | $R^4$ | $R^5$ | physik. Daten |
|---|---|---|---|---|---|---|---|---|---|
| 651 | CCl | Cl | N | CH | CH | $CH_2$ | H | 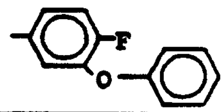 | |
| 652 | CCl | Cl | N | CH | CH | $CH_2$ | H | 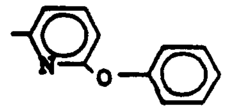 | |
| 653 | CCl | Cl | N | CH | CH | $CH_2$ | H | 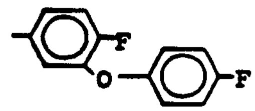 | |
| 654 | CCl | Cl | N | CH | CH | $CH_2$ | CN | 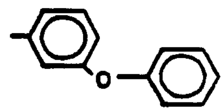 | |
| 655 | CCl | Cl | N | CH | CH | $CH_2$ | CN | 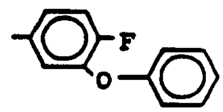 | |
| 656 | CCl | Cl | N | CH | CH | O | H | 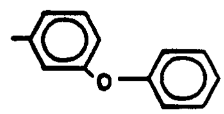 | |
| 657 | CCl | Cl | N | CH | CH | O | H | 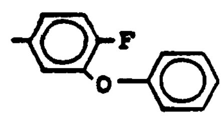 | |
| 658 | CCl | Cl | N | CH | CH | O | H | 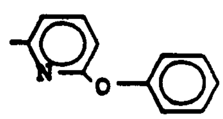 | |
| 659 | CCl | Cl | N | CH | CH | O | CN | 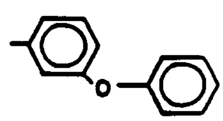 | |
| 660 | CCl | Cl | N | CH | CH | O | $CH_3$ | 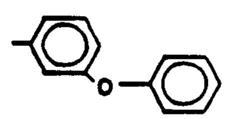 | |

**Fortsetzung Tabelle 2**

| Verbdg. Nr. | A | $R^1$ | B | C' | D | X | $R^4$ | $R^5$ | physik. Daten |
|---|---|---|---|---|---|---|---|---|---|
| 661 | CCl | EtO | N | CH | CH | $CH_2$ | H | 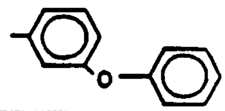 | |
| 662 | CCl | EtO | N | CH | CH | $CH_2$ | H | 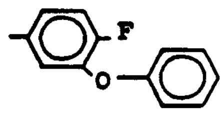 | |
| 663 | CCl | EtO | N | CH | CH | $CH_2$ | H | 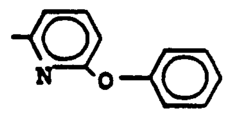 | |
| 664 | CCl | EtO | N | CH | CH | $CH_2$ | H | 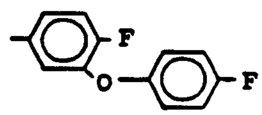 | |
| 665 | CCl | EtO | N | CH | CH | $CH_2$ | CN | 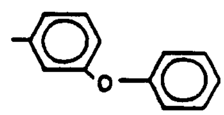 | |
| 666 | CCl | EtO | N | CH | CH | $CH_2$ | CN | 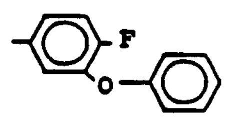 | |
| 667 | CCl | EtO | N | CH | CH | O | H | 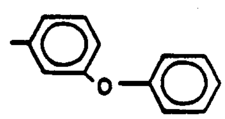 | |
| 668 | CCl | EtO | N | CH | CH | O | H | 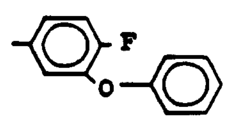 | |
| 669 | CCl | EtO | N | CH | CH | O | H | 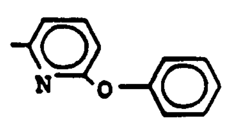 | |
| 670 | CCl | EtO | N | CH | CH | O | CN | 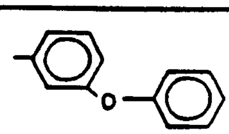 | |

Fortsetzung Tabelle 2

| Verbdg. Nr. | A | $R^1$ | B | C' | D | X | $R^4$ | $R^5$ | physik. Daten |
|---|---|---|---|---|---|---|---|---|---|
| 671 | CCl | EtO | N | CH | CH | O | $CH_3$ | 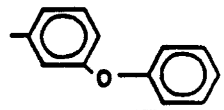 | |
| 672 | C-O-$CH_2$-O | | N | CH | CH | $CH_2$ | H | 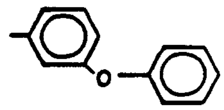 | |
| 673 | C-O-$CH_2$-O | | N | CH | CH | $CH_2$ | H | 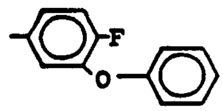 | |
| 674 | C-O-$CH_2$-O | | N | CH | CH | $CH_2$ | H | 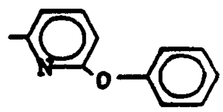 | |
| 675 | C-O-$CH_2$-O | | N | CH | CH | $CH_2$ | H | 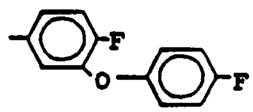 | |
| 676 | C-O-$CH_2$-O | | N | CH | CH | $CH_2$ | CN | 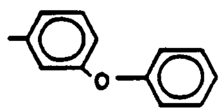 | |
| 677 | C-O-$CH_2$-O | | N | CH | CH | $CH_2$ | CN | 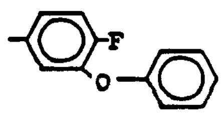 | |
| 678 | C-O-$CH_2$-O | | N | CH | CH | O | H | 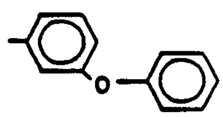 | |
| 679 | C-O-$CH_2$-O | | N | CH | CH | O | H | 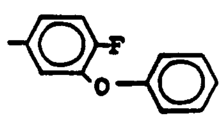 | |
| 680 | C-O-$CH_2$-O | | N | CH | CH | O | H | 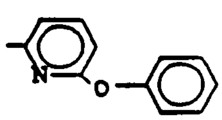 | |

**Fortsetzung Tabelle 2**

| Verbdg. Nr. | A | $R^1$ | B | C' | D | X | $R^4$ | $R^5$ | physik. Daten |
|---|---|---|---|---|---|---|---|---|---|
| 681 | $C-O-CH_2-O$ | N | CH | CH | O | CN | 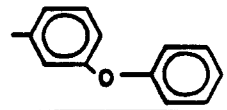 | |
| 682 | $C-O-CH_2-O$ | N | CH | CH | O | $CH_3$ | 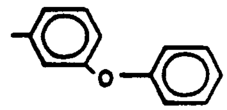 | |
| 683 | $C-O-CH_2-O$ | CH | CH | N | $CH_2$ | H | 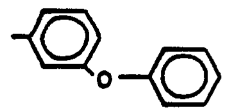 | |
| 634 | $C-O-CH_2-O$ | CH | CH | N | $CH_2$ | H | 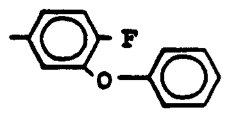 | |
| 685 | $C-O-CH_2-O$ | CH | CH | N | $CH_2$ | H | 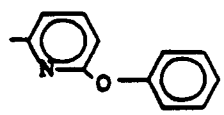 | |
| 686 | $C-O-CH_2-O$ | CH | CH | N | $CH_2$ | H | 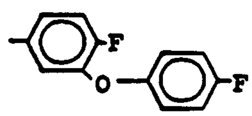 | |
| 687 | $C-O-CH_2-O$ | CH | CH | N | $CH_2$ | CN | 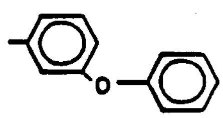 | |
| 688 | $C-O-CH_2-O$ | CH | CH | N | $CH_2$ | CN | 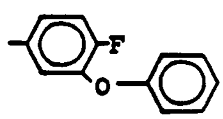 | |
| 689 | $C-O-CH_2-O$ | CH | CH | N | O | H | 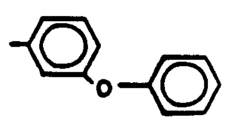 | |
| 690 | $C-O-CH_2-O$ | CH | CH | N | O | H | 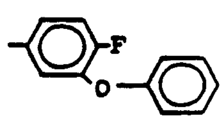 | |

**Fortsetzung Tabelle 2**

| Verbdg. Nr. | A | $R^1$ | B | C' | D | X | $R^4$ | $R^5$ | physik. Daten |
|---|---|---|---|---|---|---|---|---|---|
| 691 | C-O-$CH_2$-O | | CH | CH | N | O | H | 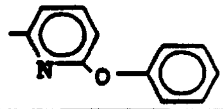 | |
| 692 | C-O-$CH_2$-O | | CH | CH | N | O | CN | 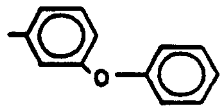 | |
| 693 | C-O-$CH_2$-O | | CH | CH | N | O | $CH_3$ | 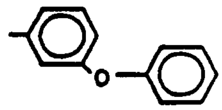 | |
| 694 | N | EtO | CH | N | CH | $CH_2$ | H | 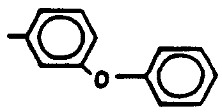 | |
| 695 | N | EtO | CH | N | CH | $CH_2$ | H | 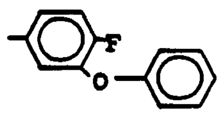 | |
| 696 | N | EtO | CH | N | CH | $CH_2$ | H | 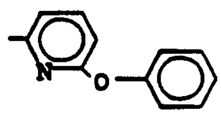 | |
| 697 | N | EtO | CH | N | CH | $CH_2$ | H | 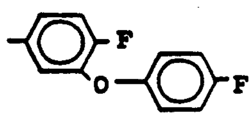 | |
| 698 | N | EtO | CH | N | CH | $CH_2$ | CN | 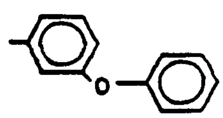 | |
| 699 | N | EtO | CH | N | CH | $CH_2$ | CN | 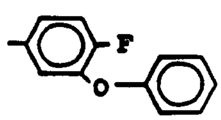 | |
| 700 | N | EtO | CH | N | CH | O | H | 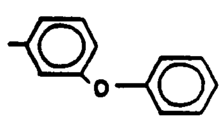 | |

**Fortsetzung Tabelle** 2

| Verbdg. Nr. | A | $R^1$ | B | C' | D | X | $R^4$ | $R^5$ | physik. Daten |
|---|---|---|---|---|---|---|---|---|---|
| 701 | N | EtO | CH | N | CH | O | H | 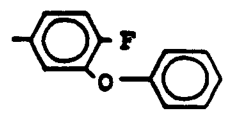 | |
| 702 | N | EtO | CH | N | CH | O | H | 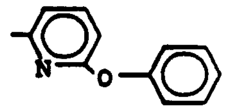 | |
| 703 | N | EtO | CH | N | CH | O | CN | 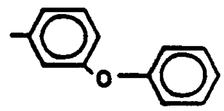 | |
| 704 | N | EtO | CH | N | CH | O | $CH_3$ | 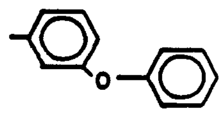 | |
| 705 | N | Cl | CH | N | CH | $CH_2$ | H | 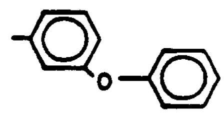 | |
| 706 | N | Cl | CH | N | CH | $CH_2$ | H | 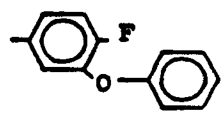 | |
| 707 | N | Cl | CH | N | CH | $CH_2$ | H | 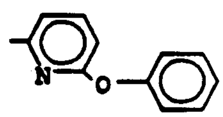 | |
| 708 | N | Cl | CH | N | CH | $CH_2$ | H | 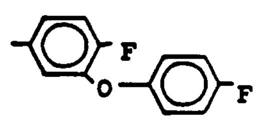 | |
| 709 | N | Cl | CH | N | CH | $CH_2$ | CN | 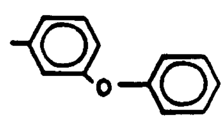 | |
| 710 | N | Cl | CH | N | CH | $CH_2$ | CN | 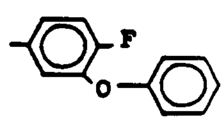 | |

Fortsetzung Tabelle 2

| Verbdg. Nr. | A | $R^1$ | B | C' | D | X | $R^4$ | $R^5$ | physik. Daten |
|---|---|---|---|---|---|---|---|---|---|
| 711 | N | Cl | CH | N | CH | O | H | 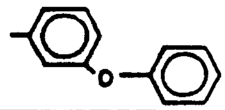 | |
| 712 | N | Cl | CH | N | CH | O | H | 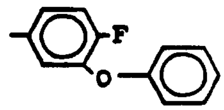 | |
| 713 | N | Cl | CH | N | CH | O | H | 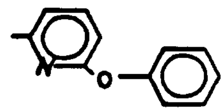 | |
| 714 | N | Cl | CH | N | CH | O | CN | 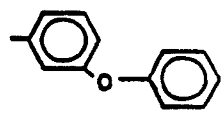 | |
| 715 | N | Cl | CH | N | CH | O | $CH_3$ | 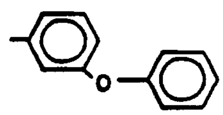 | |
| 716 | N | $H_3CO$ | CH | N | CH | $CH_2$ | H | 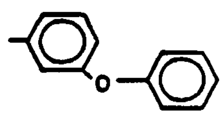 | |
| 717 | N | $H_3CO$ | CH | N | CH | $CH_3$ | H | 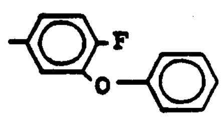 | |
| 718 | N | $H_3CO$ | CH | N | CH | $CH_2$ | H | 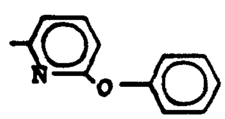 | |
| 719 | N | $H_3CO$ | CH | N | CH | $CH_2$ | H | 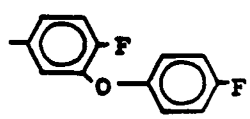 | |
| 720 | N | $H_3CO$ | CH | N | CH | $CH_2$ | CN | 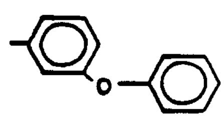 | |

**Fortsetzung Tabelle 2**

| Verbdg. Nr. | A | $R^1$ | B | C' | D | X | $R^4$ | $R^5$ | physik. Daten |
|---|---|---|---|---|---|---|---|---|---|
| 721 | N | $H_3CO$ | CH | N | CH | $CH_2$ | CN | 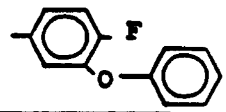 | |
| 722 | N | $H_3CO$ | CH | N | CH | O | H | 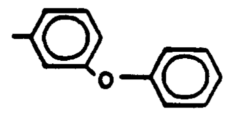 | |
| 723 | N | $H_3CO$ | CH | N | CH | O | H | 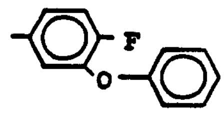 | |
| 724 | N | $H_3CO$ | CH | N | CH | O | H | 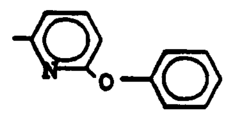 | |
| 725 | N | $H_3CO$ | CH | N | CH | O | CN | 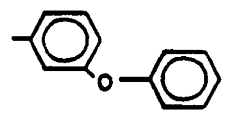 | |
| 726 | N | $H_3CO$ | CH | N | CH | O | $CH_3$ | 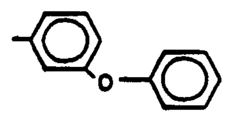 | |
| 727 | N | EtS | CH | N | CH | $CH_2$ | H | 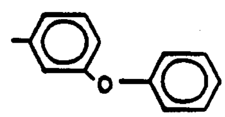 | |
| 728 | N | EtS | CH | N | CH | $CH_2$ | H | 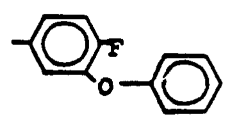 | |
| 729 | N | EtS | CH | N | CH | $CH_2$ | H | 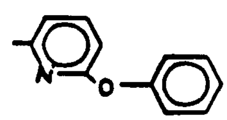 | |
| 730 | N | EtS | CH | N | CH | $CH_2$ | H | 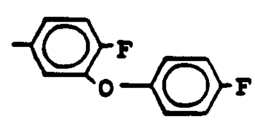 | |

Fortsetzung Tabelle 2

| Verbdg. Nr. | A | $R^1$ | B | C' | D | X | $R^4$ | $R^5$ | physik. Daten |
|---|---|---|---|---|---|---|---|---|---|
| 731 | N | EtS | CH | N | CH | $CH_2$ | CN | 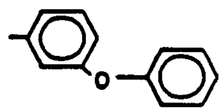 | |
| 732 | N | EtS | CH | N | CH | $CH_2$ | CN | 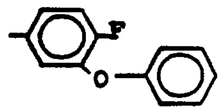 | |
| 733 | N | EtS | CH | N | CH | O | H | 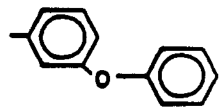 | |
| 734 | N | EtS | CH | N | CH | O | H | 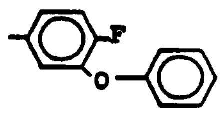 | |
| 735 | N | EtS | CH | N | CH | O | H | 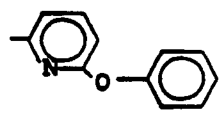 | |
| 736 | N | EtS | CH | N | CH | O | CN | 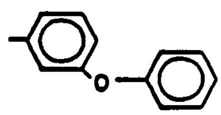 | |
| 737 | N | EtS | CH | N | CH | O | $CH_3$ | 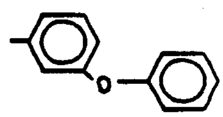 | |
| 738 | N | n-$C_3H_7$ | CH | N | CH | $CH_2$ | H | 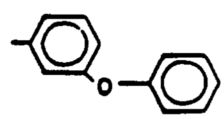 | |
| 739 | N | n-$C_3H_7$ | CH | N | CH | $CH_2$ | H | 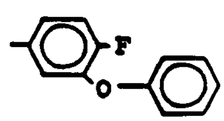 | |
| 740 | N | n-$C_3H_7$ | CH | N | CH | $CH_2$ | H | 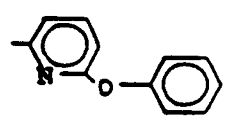 | |

Fortsetzung Tabelle 2

| Verbdg. Nr. | A | $R^1$ | B | C' | D | X | $R^4$ | $R^5$ | physik. Daten |
|---|---|---|---|---|---|---|---|---|---|
| 741 | N | $n-C_3H_7$ | CH | N | CH | $CH_2$ | CN | 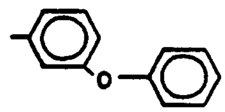 | |
| 742 | N | $n-C_3H_7$ | CH | N | CH | $CH_2$ | CN | 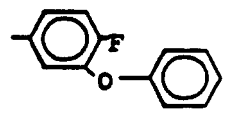 | |
| 743 | N | $n-C_3H_7$ | CH | N | CH | O | H | 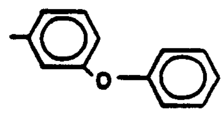 | |
| 744 | N | $n-C_3H_7$ | CH | N | CH | O | H | 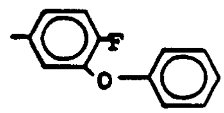 | |
| 745 | N | $n-C_3H7$ | CH | N | CH | O | H | 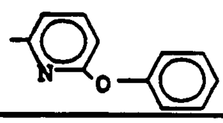 | |
| 746 | N | $n-C_3H_7$ | CH | N | CH | O | CN | 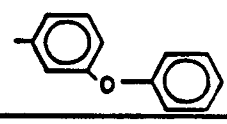 | |
| 747 | N | $n-C_3H_7$ | CH | N | CH | O | $CH_3$ | 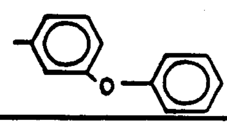 | |
| 748 | CH | EtO | N | CH | N | $CH_2$ | H | 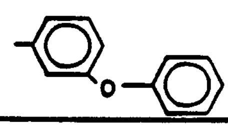 | |
| 749 | CH | EtO | N | CH | N | $CH_2$ | H | 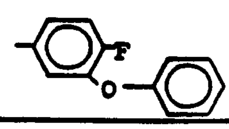 | |
| 750 | CH | EtO | N | CH | N | $CH_2$ | H | 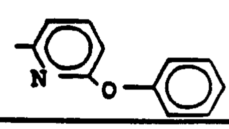 | |

**Fortsetzung Tabelle 2**

| Verbdg. Nr. | A | $R^1$ | B | C' | D | X | $R^4$ | $R^5$ | physik. Daten |
|---|---|---|---|---|---|---|---|---|---|
| 751 | CH | EtO | N | CH | N | $CH_2$ | H | 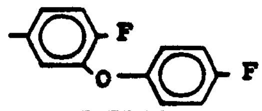 | |
| 752 | CH | EtO | N | CH | N | $CH_2$ | CN | 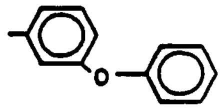 | |
| 753 | CH | EtO | N | CH | N | $CH_2$ | CN | 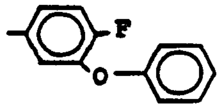 | |
| 754 | CH | EtO | N | CH | N | O | H | 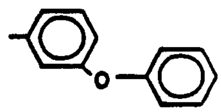 | |
| 755 | CH | EtO | N | CH | N | O | H | 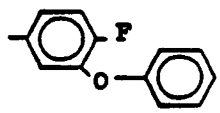 | |
| 756 | CH | EtO | N | CH | N | O | H | 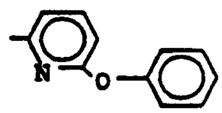 | |
| 757 | CH | EtO | N | CH | N | O | CN | 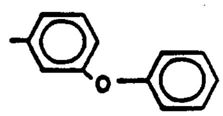 | |
| 758 | CH | EtO | N | CH | N | O | $CH_3$ | 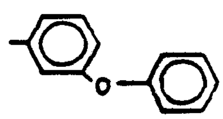 | |
| 759 | CH | Cl | N | CH | N | $CH_2$ | H | 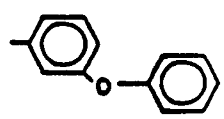 | |
| 760 | CH | Cl | N | CH | N | $CH_2$ | H | 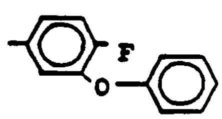 | |

**Fortsetzung Tabelle 2**

| Verbdg. Nr. | A | $R^1$ | B | C' | D | X | $R^4$ | $R^5$ | physik. Daten |
|---|---|---|---|---|---|---|---|---|---|
| 761 | CH | Cl | N | CH | N | $CH_2$ | H | 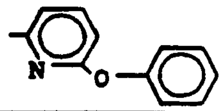 | |
| 762 | CH | Cl | N | CH | N | $CH_2$ | H | 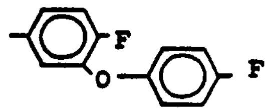 | |
| 763 | CH | Cl | N | CH | N | $CH_2$ | CN | 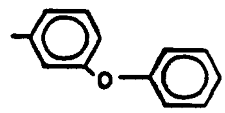 | |
| 764 | CH | Cl | N | CH | N | $CH_2$ | CN | 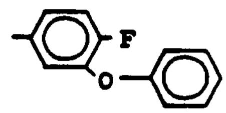 | |
| 765 | CH | Cl | N | CH | N | O | H | 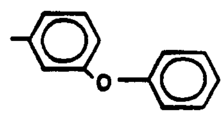 | |
| 776 | CH | Cl | N | CH | N | O | H | 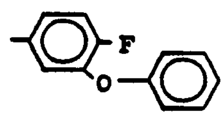 | |
| 777 | CH | Cl | N | CH | N | O | H | 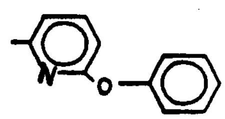 | |
| 778 | CH | Cl | N | CH | N | O | CN | 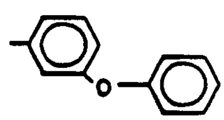 | |
| 779 | CH | Cl | N | CH | N | O | $CH_3$ | 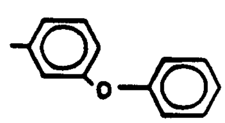 | |
| 780 | CH | $H_3CO$ | N | CH | N | $CH_2$ | H | 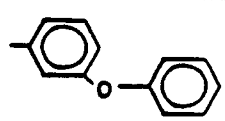 | |

Fortsetzung Tabelle 2

| Verbdg. Nr. | A | $R^1$ | B | C' | D | X | $R^4$ | $R^5$ | physik. Daten |
|---|---|---|---|---|---|---|---|---|---|
| 781 | CH | $H_3CO$ | N | CH | N | $CH_2$ | H | 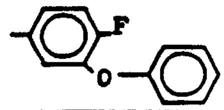 | |
| 782 | CH | $H_3CO$ | N | CH | N | $CH_2$ | H | 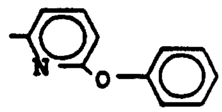 | |
| 783 | CH | $H_3CO$ | N | CH | N | $CH_2$ | H | 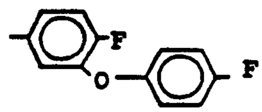 | |
| 784 | CH | $H_3CO$ | N | CH | N | $CH_2$ | CN | 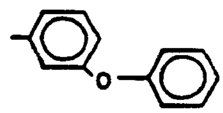 | |
| 785 | CH | $H_3CO$ | N | CH | N | $CH_2$ | CN | 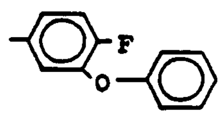 | |
| 786 | CH | $H_3CO$ | N | CH | N | O | H | 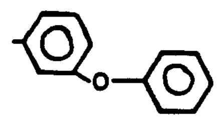 | |
| 787 | CH | $H_3CO$ | N | CH | N | O | H | 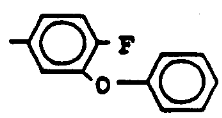 | |
| 788 | CH | $H_3CO$ | N | CH | N | O | H | 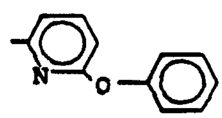 | |
| 789 | CH | $H_3CO$ | N | CH | N | O | CN | 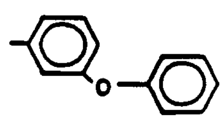 | |
| 790 | CH | $H_3CO$ | N | CH | N | O | $CH_3$ | 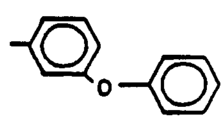 | |

**Fortsetzung Tabelle 2**

| Verbdg. Nr. | A | $R^1$ | B | C' | D | X | $R^4$ | $R^5$ | physik. Daten |
|---|---|---|---|---|---|---|---|---|---|
| 791 | CH | $F_2CHO$ | N | CH | N | $CH_2$ | H | 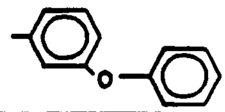 | |
| 792 | CH | $F_2CHO$ | N | CH | N | $CH_2$ | H | 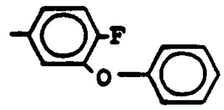 | |
| 793 | CH | $F_2CHO$ | N | CH | N | $CH_2$ | H | 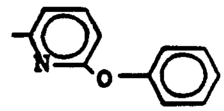 | |
| 794 | CH | $F_2CHO$ | N | CH | N | $CH_2$ | H | 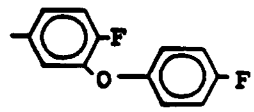 | |
| 795 | CH | $F_2CHO$ | N | CH | N | $CH_2$ | CN | 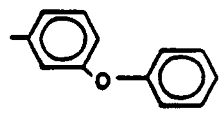 | |
| 796 | CH | $F_2CHO$ | N | CH | N | $CH_2$ | CN | 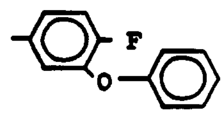 | |
| 797 | CH | $F_2CHO$ | N | CH | N | O | H | 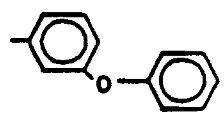 | |
| 798 | CH | $F_2CHO$ | N | CH | N | O | H | 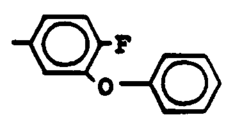 | |
| 799 | CH | $F_2CHO$ | N | CH | N | O | H | 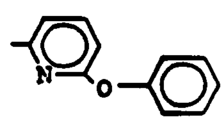 | |
| 800 | CH | $F_2CHO$ | N | CH | N | O | CN | 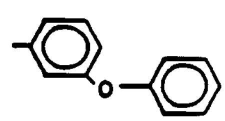 | |

**Fortsetzung Tabelle 2**

| Verbdg. Nr. | A | $R^1$ | B | C' | D | X | $R^4$ | $R^5$ | physik. Daten |
|---|---|---|---|---|---|---|---|---|---|
| 801 | CH | $F_2CHO$ | N | CH | N | O | $CH_3$ | 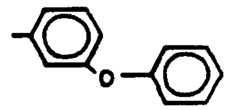 | |
| 802 | CH | EtS | N | CH | N | $CH_2$ | H | 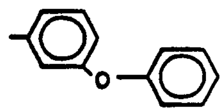 | |
| 803 | CH | EtS | N | CH | N | $CH_2$ | H | 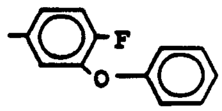 | |
| 804 | CH | EtS | N | CH | N | $CH_2$ | H | 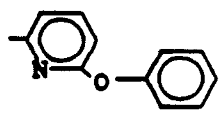 | |
| 805 | CH | EtS | N | CH | N | $CH_2$ | H | 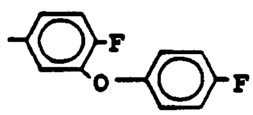 | |
| 806 | CH | EtS | N | CH | N | $CH_2$ | CN | 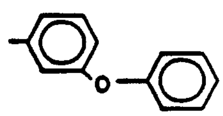 | |
| 807 | CH | EtS | N | CH | N | $CH_2$ | CN | 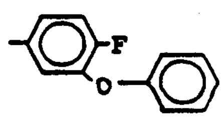 | |
| 808 | CH | EtS | N | CH | N | O | H | 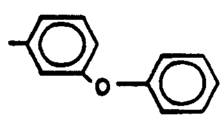 | |
| 809 | CH | EtS | N | CH | N | O | H | 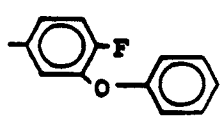 | |
| 810 | CH | EtS | N | CH | N | O | H | 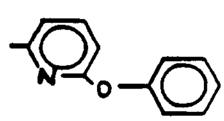 | |

**Fortsetzung Tabelle 2**

| Verbdg. Nr. | A | $R^1$ | B | C' | D | X | $R^4$ | $R^5$ | physik. Daten |
|---|---|---|---|---|---|---|---|---|---|
| 811 | CH | EtS | N | CH | N | O | CN | 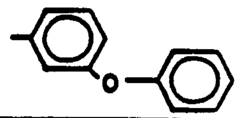 | |
| 812 | CH | EtS | N | CH | N | O | $CH_3$ | 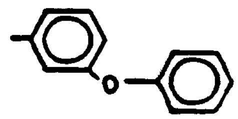 | |
| 813 | N | EtO | CH | CH | N | $CH_2$ | H | 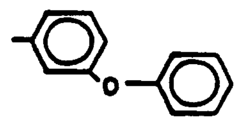 | |
| 814 | N | EtO | CH | CH | N | $CH_2$ | H | 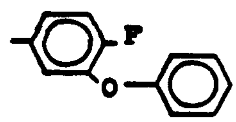 | |
| 815 | N | EtO | CH | CH | N | $CH_2$ | H | 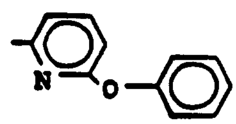 | |
| 816 | N | EtO | CH | CH | N | O | H | 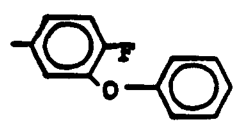 | |
| 817 | N | EtO | CH | CH | N | O | H | 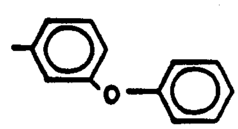 | |
| 818 | N | Cl | CH | CH | N | $CH_2$ | H | 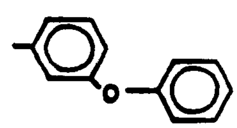 | |
| 819 | N | Cl | CH | CH | N | $CH_2$ | H | 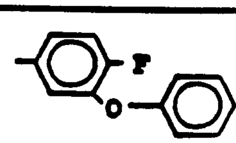 | |

**Fortsetzung Tabelle 2**

| Verbdg. Nr. | A | $R^1$ | B | C' | D | X | $R^4$ | $R^5$ | physik. Daten |
|---|---|---|---|---|---|---|---|---|---|
| 820 | N | Cl | CH | CH | N | $CH_2$ | H | 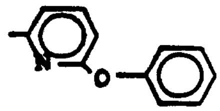 | |
| 821 | N | Cl | CH | CH | N | O | H | 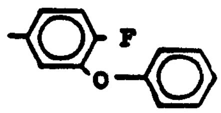 | |
| 822 | N | Cl | CH | CH | N | O | H | 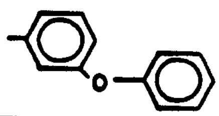 | |
| 823 | N | $CH_3O$ | CH | CH | N | $CH_2$ | H | 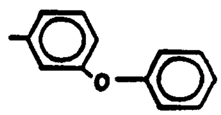 | |
| 824 | N | $CH_3O$ | CH | CH | N | $CH_2$ | H | 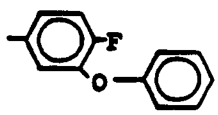 | |
| 825 | N | $CH_3O$ | CH | CH | N | $CH_2$ | H | 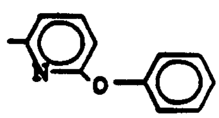 | |
| 826 | N | $CH_3O$ | CH | CH | N | O | H | 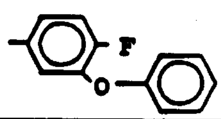 | |
| 827 | N | $CH_3O$ | CH | CH | N | O | H | 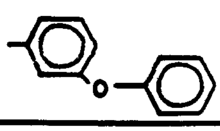 | |
| 828 | N | EtS | CH | CH | N | $CH_2$ | H | 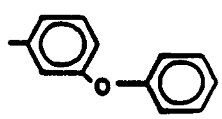 | |
| 829 | N | EtS | CH | CH | N | $CH_2$ | H | 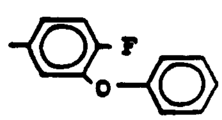 | |

**Fortsetzung Tabelle 2**

| Verbdg. Nr. | A | $R^1$ | B | C' | D | X | $R^4$ | $R^5$ | physik. Daten |
|---|---|---|---|---|---|---|---|---|---|
| 830 | N | EtS | CH | CH | N | $CH_2$ | H | 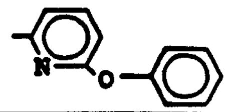 | |
| 831 | N | EtS | CH | CH | N | O | H | 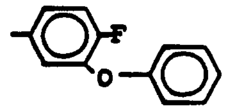 | |
| 832 | N | EtS | CH | CH | N | O | H | 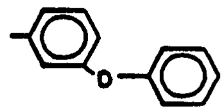 | |
| 833 | CCl | EtO | N | N | CH | $CH_2$ | H | 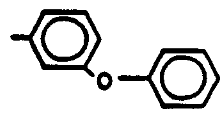 | |
| 834 | CCl | EtO | N | N | CH | $CH_2$ | H | 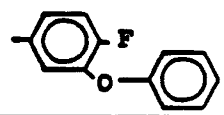 | |
| 835 | CCl | EtO | N | N | CH | $CH_2$ | H | 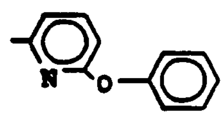 | |
| 836 | CCl | EtO | N | N | CH | O | H | 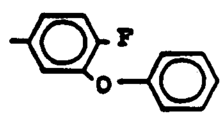 | |
| 837 | CCl | EtO | N | N | CH | O | H | 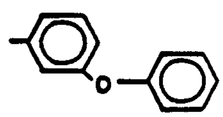 | |
| 838 | CCl | Cl | N | N | CH | $CH_2$ | H | 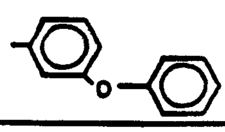 | |
| 839 | CCl | Cl | N | N | CH | $CH_2$ | H | 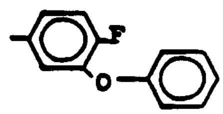 | |

**Fortsetzung Tabelle 2**

| Verbdg. Nr. | A | $R^1$ | B | C' | D | X | $R^4$ | $R^5$ | physik. Daten |
|---|---|---|---|---|---|---|---|---|---|
| 840 | CCl | Cl | N | N | CH | $CH_2$ | H | 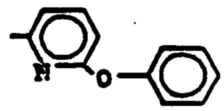 | |
| 841 | CCl | Cl | N | N | CH | O | H | 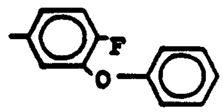 | |
| 842 | CCl | Cl | N | N | CH | O | H | 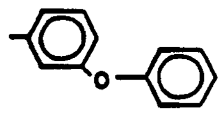 | |
| 843 | CH | EtO | CH | N | N | $CH_2$ | H | 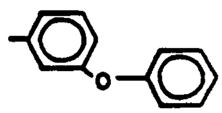 | |
| 844 | CH | EtO | CH | N | N | $CH_2$ | H | 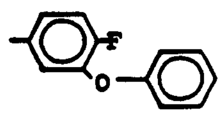 | |
| 845 | CH | EtO | CH | N | N | $CH_2$ | H | 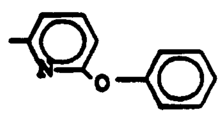 | |
| 846 | CH | EtO | CH | N | N | O | H | 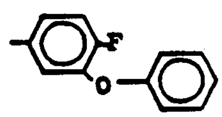 | |
| 847 | CH | EtO | CH | N | N | O | H | 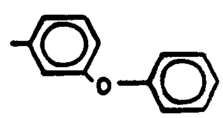 | |
| 848 | CH | $CH_3O$ | CH | N | N | $CH_2$ | H | 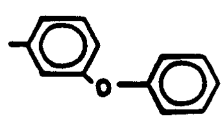 | |
| 849 | CH | $CH_3O$ | CH | N | N | $CH_2$ | H | 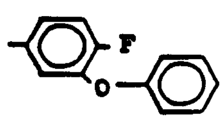 | |

**Fortsetzung Tabelle 2**

| Verbdg. Nr. | A | $R^1$ | B | C' | D | X | $R^4$ | $R^5$ | physik. Daten |
|---|---|---|---|---|---|---|---|---|---|
| 850 | CH | $CH_3O$ | CH | N | N | $CH_2$ | H | 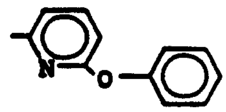 | |
| 851 | CH | $CH_3O$ | CH | N | N | O | H | 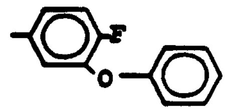 | |
| 852 | CH | $CH_3O$ | CH | N | N | O | H | 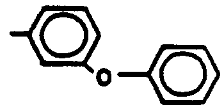 | |
| 853 | CH | Cl | CH | N | N | $CH_2$ | H | 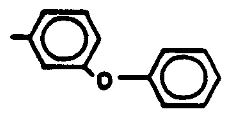 | |
| 854 | CH | Cl | CH | N | N | $CH_2$ | H | 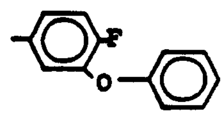 | |
| 855 | CH | Cl | CH | N | N | $CH_2$ | H | 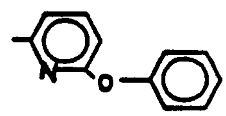 | |
| 856 | CH | Cl | CH | N | N | O | H | 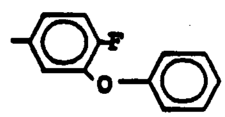 | |
| 857 | CH | Cl | CH | N | N | O | H | 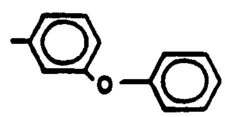 | |
| 858 | CH | EtS | CH | N | N | $CH_2$ | H | 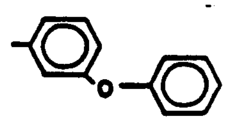 | |
| 859 | CH | EtS | CH | N | N | $CH_2$ | H | 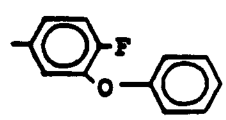 | |

**Fortsetzung Tabelle 2**

| Verbdg. Nr. | A | $R^1$ | B | C | D | X | $R^4$ | $R^5$ | physik. Daten |
|---|---|---|---|---|---|---|---|---|---|
| 860 | CH | EtS | CH | N | N | $CH_2$ | H | 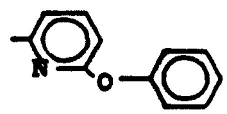 | |
| 861 | CH | EtS | CH | N | N | O | H | 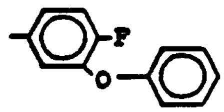 | |
| 862 | CH | EtS | CH | N | N | O | H | 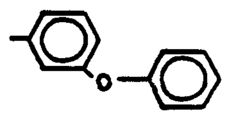 | |
| 863 | CCl | Cl | CH | N | N | $CH_2$ | H | 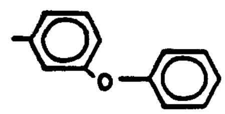 | |
| 864 | CCl | Cl | CH | N | N | $CH_2$ | H | 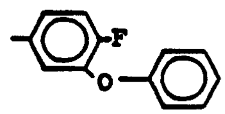 | |
| 865 | CCl | Cl | CH | N | N | $CH_2$ | H | 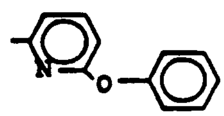 | |
| 866 | CCl | Cl | CH | N | N | O | H | 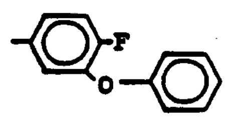 | |
| 857 | CCl | Cl | CH | N | N | O | H | 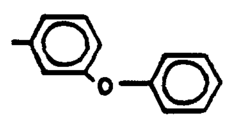 | |
| 868 | CH | EtO | N | N | N | $CH_2$ | H | 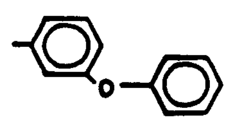 | |
| 869 | CH | EtO | N | N | N | $CH_2$ | H | 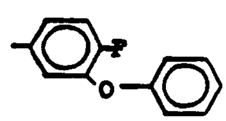 | |

**Fortsetzung Tabelle** 2

| Verbdg. Nr. | A | $R^1$ | B | C' | D | X | $R^4$ | $R^5$ | physik. Daten |
|---|---|---|---|---|---|---|---|---|---|
| 870 | CH | EtO | N | N | N | $CH_2$ | H | 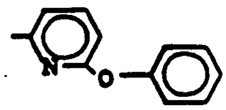 | |
| 871 | CH | EtO | N | N | N | O | H | 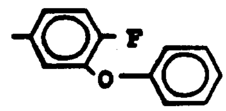 | |
| 872 | CH | EtO | N | N | N | O | H | 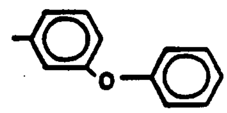 | |
| 873 | CH | $CH_3O$ | N | N | N | $CH_2$ | H | 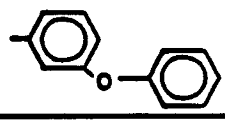 | |
| 874 | CH | $CH_3O$ | N | N | N | $CH_2$ | H | 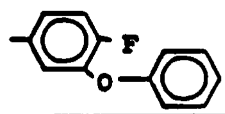 | |
| 875 | CH | $CH_3O$ | N | N | N | $CH_2$ | H | 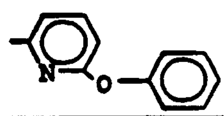 | |
| 876 | CH | $CH_3O$ | N | N | N | O | H | 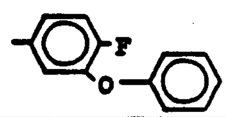 | |
| 877 | CH | $CH_3O$ | N | N | N | O | H | 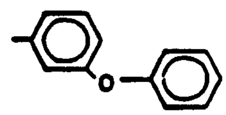 | |
| 878 | CH | Cl | N | N | N | $CH_2$ | H | 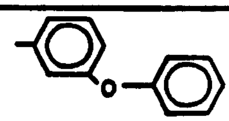 | |
| 879 | CH | Cl | N | N | N | $CH_2$ | H | 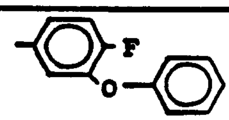 | |

**Fortsetzung Tabelle 2**

| Verbdg. Nr. | A | $R^1$ | B | C' | D | X | $R^4$ | $R^5$ | physik. Daten |
|---|---|---|---|---|---|---|---|---|---|
| 880 | CH | Cl | N | N | N | $CH_2$ | H | 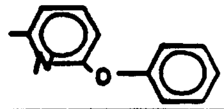 | |
| 881 | CH | Cl | N | N | N | O | H | 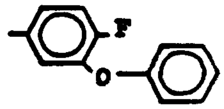 | |
| 882 | CH | Cl | N | N | N | O | H | 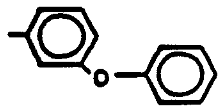 | |
| 883 | CH | EtS | N | N | N | $CH_2$ | H | 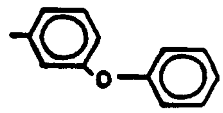 | |
| 884 | CH | EtS | N | N | N | $CH_2$ | H | 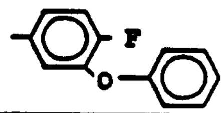 | |
| 885 | CH | EtS | N | N | N | $CH_2$ | H | 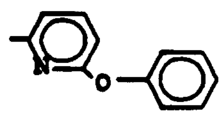 | |
| 886 | CH | EtS | N | N | N | O | H | 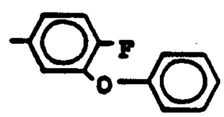 | |
| 887 | CH | EtS | N | N | N | O | H | 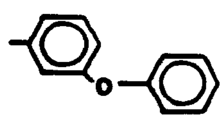 | |
| 888 | N | EtS | N | N | N | $CH_2$ | H | 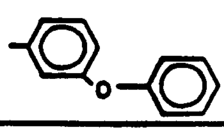 | |
| 889 | N | EtO | N | N | CH | $CH_2$ | H | 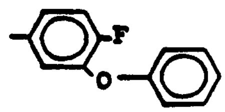 | |

**Fortsetzung Tabelle 2**

| Verbdg. Nr. | A | $R^1$ | B | C' | D | X | $R^4$ | $R^5$ | physik. Daten |
|---|---|---|---|---|---|---|---|---|---|
| 890 | N | EtO | N | N | CH | $CH_2$ | H | 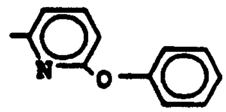 | |
| 891 | N | EtO | N | N | N | O | H | 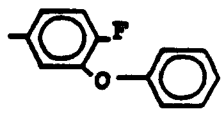 | |
| 892 | N | EtO | N | N | CH | O | H | 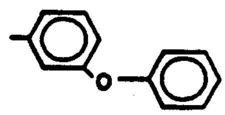 | |
| 893 | N | $CH_3O$ | N | N | CH | $CH_2$ | H | 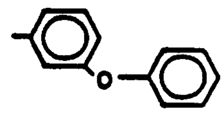 | |
| 894 | N | $CH_3O$ | N | N | CH | $CH_2$ | H | 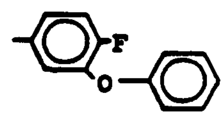 | |
| 895 | N | $CH_3O$ | N | N | CH | $CH_2$ | H | 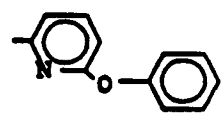 | |
| 896 | N | $CH_3O$ | N | N | CH | O | H | 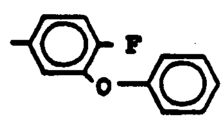 | |
| 897 | N | $CH_3O$ | N | N | CH | O | H | 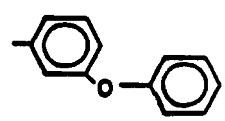 | |
| 898 | N | Cl | N | N | CH | $CH_2$ | H | 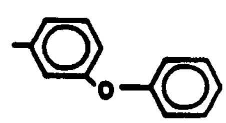 | |
| 899 | N | Cl | N | N | CH | $CH_2$ | H | 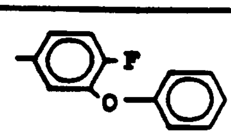 | |

**Fortsetzung Tabelle 2**

| Verbdg. Nr. | A | $R^1$ | B | C' | D | X | $R^4$ | $R^5$ | physik. Daten |
|---|---|---|---|---|---|---|---|---|---|
| 900 | N | Cl | N | N | CH | $CH_2$ | H | 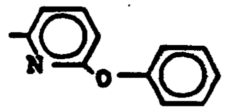 | |
| 901 | N | Cl | N | N | CH | O | H | 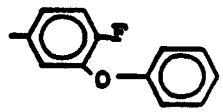 | |
| 902 | N | Cl | N | N | CH | O | H | 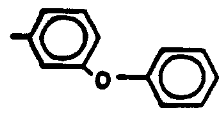 | |
| 903 | N | EtS | N | N | CH | $CH_2$ | H | 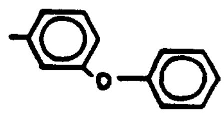 | |
| 904 | N | EtS | N | N | CH | $CH_2$ | H | 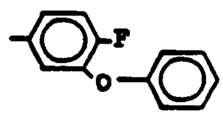 | |
| 905 | N | EtS | N | N | CH | $CH_2$ | H | 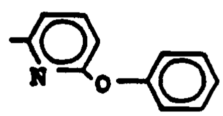 | |
| 906 | N | EtS | N | N | CH | O | H | 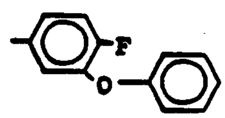 | |
| 907 | N | EtS | N | N | CH | O | H | 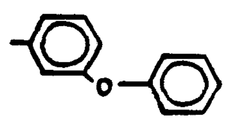 | |
| 908 | N | EtO | N | N | N | $CH_2$ | H | 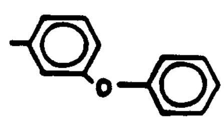 | |
| 909 | N | EtO | N | N | N | $CH_2$ | H | 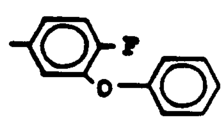 | |

**Fortsetzung Tabelle 2**

| Verbdg. Nr. | A | $R^1$ | B | C' | D | X | $R^4$ | $R^5$ | physik. Daten |
|---|---|---|---|---|---|---|---|---|---|
| 910 | N | EtO | N | N | N | $CH_2$ | H | 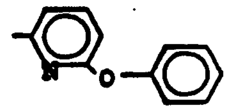 | |
| 911 | N | EtO | N | N | N | O | H | 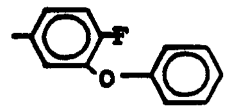 | |
| 912 | N | EtO | N | N | N | O | H | 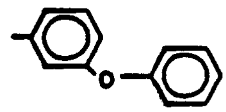 | |
| 913 | N | $CH_3O$ | N | N | N | $CH_2$ | H | 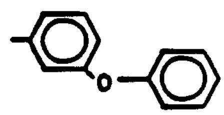 | |
| 914 | N | $CH_3O$ | N | N | N | $CH_2$ | H | 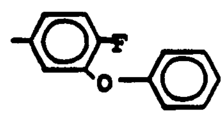 | |
| 915 | N | $CH_3O$ | N | N | N | $CH_2$ | H | 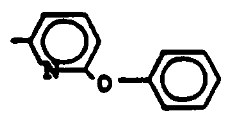 | |
| 916 | N | $CH_3O$ | N | N | N | O | H | 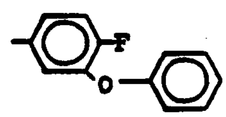 | |
| 917 | N | $CH_3O$ | N | N | N | O | H | 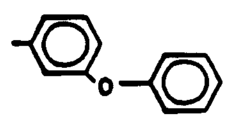 | |
| 918 | N | Cl | N | N | N | $CH_2$ | H | 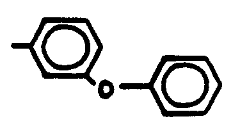 | |
| 919 | N | Cl | N | N | N | $CH_2$ | H | 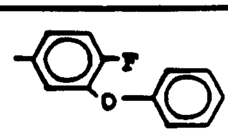 | |

**Fortsetzung Tabelle 2**

| Verbdg. Nr. | A | $R^1$ | B | C' | D | X | $R^4$ | $R^5$ | physik. Daten |
|---|---|---|---|---|---|---|---|---|---|
| 920 | N | Cl | N | N | N | $CH_2$ | H | 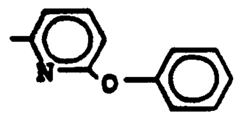 | |
| 921 | N | Cl | N | N | N | O | H | 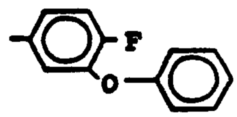 | |
| 923 | N | Cl | N | N | N | O | H | 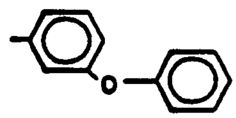 | |
| 924 | N | EtS | N | N | N | $CH_2$ | H | 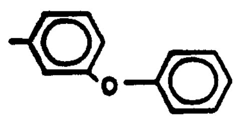 | |
| 925 | N | EtS | N | N | N | $CH_2$ | H | 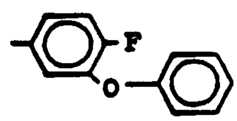 | |
| 926 | N | EtS | N | N | N | $CH_2$ | H | 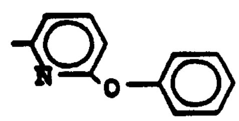 | |
| 927 | N | EtS | N | N | N | O | H | 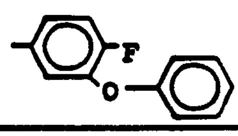 | |
| 928 | N | EtS | N | N | N | O | H | 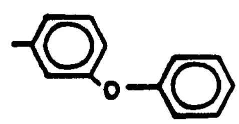 | |

Fortsetzung Tabelle 2

| Verbdg. Nr. | A | $R^1$ | B | C' | D | X | $R^4$ | $R^5$ | physik. Daten |
|---|---|---|---|---|---|---|---|---|---|
| 929 | N | $F_3C-CH_2-O-$ | CH | CH | CH | $CH_2$ | H | 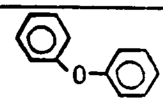 | |
| 930 | N | $F_3C-CH_2-O-$ | CH | CH | CH | $CH_2$ | H | 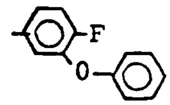 | |
| 931 | N | $F_3C-CH_2-O-$ | CH | CH | CH | $CH_2$ | H | 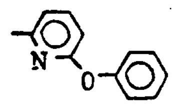 | |
| 932 | N | $F_3C-CH_2-O-$ | CH | CH | CH | $CH_2$ | H | 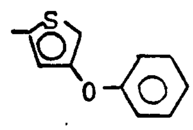 | |
| 933 | N | $F_3C-CH_2-O-$ | CH | CH | CH | $CH_2$ | H | 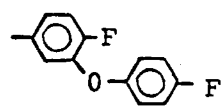 | |
| 934 | N | $F_3C-CH_2-O-$ | CH | CH | CH | O | H | 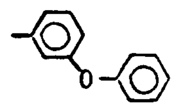 | |
| 935 | N | $F_3C-CH_2-O-$ | CH | CH | CH | O | H | 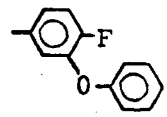 | |
| 936 | N | $F_3C-CH_2-O-$ | CH | CH | CH | O | H | 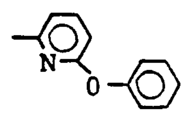 | |

Fortsetzung Tabelle 2

| Verbdg. Nr. | A | $R^1$ | B | C' | D | X | $R^4$ | $R^5$ | physik. Daten |
|---|---|---|---|---|---|---|---|---|---|
| 937 | CH | $F_3C-CH_2-O-$ | N | CH | CH | $CH_2$ | H | 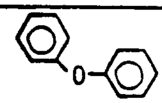 | |
| 938 | CH | $F_3C-CH_2-O-$ | N | CH | CH | $CH_2$ | H | 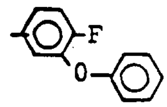 | |
| 939 | CH | $F_3C-CH_2-0-$ | N | CH | CH | $CH_2$ | H | 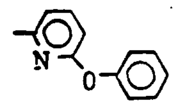 | |
| 940 | CH | $F_3C-CH_2-0-$ | N | CH | CH | $CH_2$ | H | 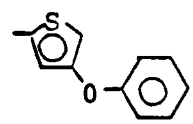 | |
| 941 | CH | $F_3C-CH_2-0-$ | N | CH | CH | $CH_2$ | H | 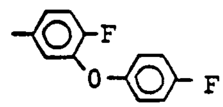 | |
| 942 | CH | $F_3C-CH_2-0-$ | N | CH | CH | 0 | H | 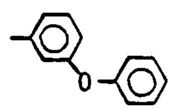 | |
| 943 | CH | $F_3C-CH_2-0-$ | N | CH | CH | 0 | H | 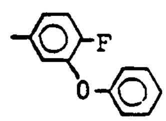 | |
| 944 | CH | $F_3C-CH_2-0-$ | N | CH | CH | 0 | H | 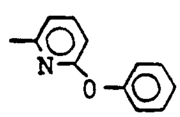 | |

Fortsetzung Tabelle 2

| Verbdg. Nr. | A | $R^1$ | B | C' | D | X | $R^4$ | $R^5$ | physik. Daten |
|---|---|---|---|---|---|---|---|---|---|
| 945 | N | $F_3C-CH_2-O-$ | CH | N | CH | $CH_2$ | H | 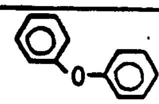 | |
| 946 | N | $F_3C-CH_2-O-$ | CH | N | CH | $CH_2$ | H | 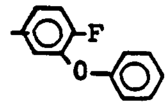 | |
| 947 | N | $F_3C-CH_2-O-$ | CH | N | CH | $CH_2$ | H | 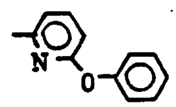 | |
| 948 | N | $F_3C-CH_2-O-$ | CH | N | CH | $CH_2$ | H | 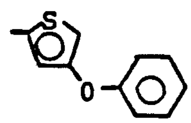 | |
| 949 | N | $F_3C-CH_2-O-$ | CH | N | CH | $CH_2$ | H | 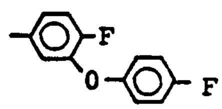 | |
| 950 | N | $F_3C-CH_2-O-$ | CH | N | CH | O | H | 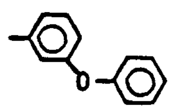 | |
| 951 | N | $F_3C-CH_2-O-$ | CH | N | CH | O | H | 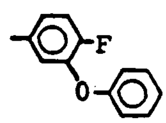 | |
| 952 | N | $F_3C-CH_2-O-$ | CH | N | CH | O | H | 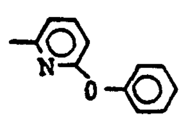 | |

Fortsetzung Tabelle 2

| Verbdg. Nr. | A | $R^1$ | B | C' | D | X | $R^4$ | $R^5$ | physik. Daten |
|---|---|---|---|---|---|---|---|---|---|
| 953 | CH | $F_3C-CH_2-O-$ | N | CH | N | $CH_2$ | H | 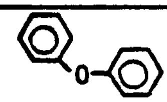 | |
| 954 | CH | $F_3C-CH_2-O-$ | N | CH | N | $CH_2$ | H | 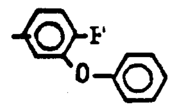 | |
| 955 | CH | $F_3C-CH_2-O-$ | N | CH | N | $CH_2$ | H | 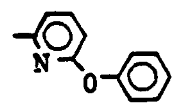 | |
| 956 | CH | $F_3C-CH_2-O-$ | N | CH | N | $CH_2$ | H | 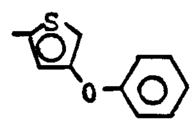 | |
| 957 | CH | $F_3C-CH_2-O-$ | N | CH | N | $CH_2$ | H | 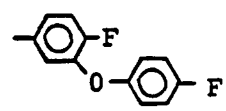 | |
| 958 | CH | $F_3C-CH_2-O-$ | N | CH | N | 0 | H | 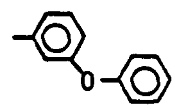 | |
| 959 | CH | $F_3C-CH_2-O-$ | N | CH | N | 0 | H | 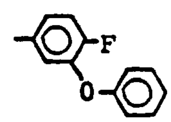 | |
| 960 | CH | $F_3C-CH_2-O-$ | N | CH | N | 0 | H | 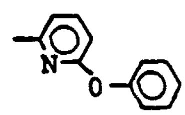 | |

Fortsetzung Tabelle 2

| Verbdg. Nr. | A | $R^1$ | B | C' | D | X | $R^4$ | $R^5$ | physik. Daten |
|---|---|---|---|---|---|---|---|---|---|
| 961 | N | $F_3C-CH_2-O-$ | N | CH | CH | $CH_2$ | H | 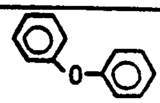 | |
| 962 | N | $F_3C-CH_2-O-$ | N | CH | CH | $CH_2$ | H | 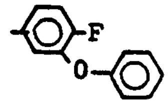 | |
| 963 | N | $F_3C-CH_2-O-$ | N | CH | CH | $CH_2$ | H | 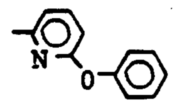 | |
| 964 | N | $F_3C-CH_2-O-$ | N | CH | CH | $CH_2$ | H | 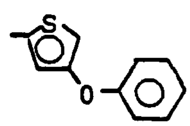 | |
| 965 | N | $F_3C-CH_2-O-$ | N | CH | CH | $CH_2$ | H | 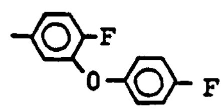 | |
| 966 | N | $F_3C-CH_2-O-$ | N | CH | CH | O | H | 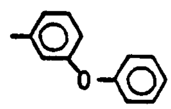 | |
| 967 | N | $F_3C-CH_2-O-$ | N | CH | CH | O | H | 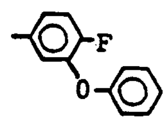 | |
| 968 | N | $F_3C-CH_2-O-$ | N | CH | CH | O | H | 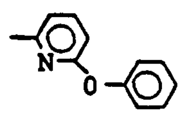 | |

Fortsetzung Tabelle 2

| Verbdg. Nr. | A | $R^1$ | B | C' | D | X | $R^4$ | $R^5$ | physik. Daten |
|---|---|---|---|---|---|---|---|---|---|
| 969 | N | $F_3C-CH_2-O-$ | CH | CH | N | $CH_2$ | H | 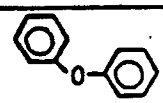 | |
| 970 | N | $F_3C-CH_2-O-$ | CH | CH | N | $CH_2$ | H | 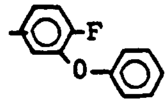 | |
| 971 | N | $F_3C-CH_2-O-$ | CH | CH | N | $CH_2$ | H | 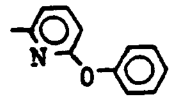 | |
| 972 | N | $F_3C-CH_2-O-$ | CH | CH | N | $CH_2$ | H | 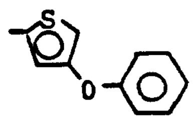 | |
| 973 | N | $F_3C-CH_2-O-$ | CH | CH | N | $CH_2$ | H | 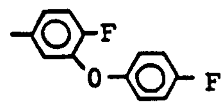 | |
| 974 | N | $F_3C-CH_2-O-$ | CH | CH | N | O | H | 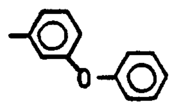 | |
| 975 | N | $F_3C-CH_2-O-$ | CH | CH | N | O | H | 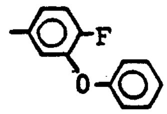 | |
| 976 | N | $F_3C-CH_2-O-$ | CH | CH | N | O | H | 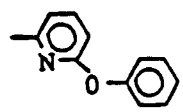 | |

Fortsetzung Tabelle 2

| Verbdg. Nr. | A | $R^1$ | B | C' | D | X | $R^4$ | $R^5$ | physik. Daten |
|---|---|---|---|---|---|---|---|---|---|
| 977 | N | $F_3C-CH_2-O-$ | N | N | CH | $CH_2$ | H | 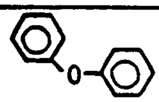 | |
| 978 | N | $F_3C-CH_2-O-$ | N | N | CH | $CH_2$ | H | 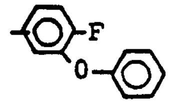 | |
| 979 | N | $F_3C-CH_2-O-$ | N | N | CH | $CH_2$ | H | 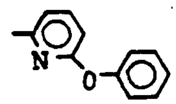 | |
| 980 | N | $F_3C-CH_2-O-$ | N | N | CH | $CH_2$ | H | 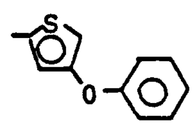 | |
| 981 | N | $F_3C-CH_2-O-$ | N | N | CH | $CH_2$ | H | 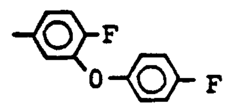 | |
| 982 | N | $F_3C-CH_2-O-$ | N | N | CH | O | H | 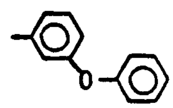 | |
| 983 | N | $F_3C-CH_2-O-$ | N | N | CH | O | H | 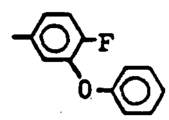 | |
| 984 | N | $F_3C-CH_2-O-$ | N | N | CH | O | H | 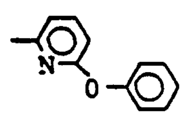 | |

Fortsetzung Tabelle 2

| Verbdg. Nr. | A | $R^1$ | B | C' | D | X | $R^4$ | $R^5$ | physik. Daten |
|---|---|---|---|---|---|---|---|---|---|
| 985 | N | $F_3C-CH_2-O-$ | N | CH | N | $CH_2$ | H | 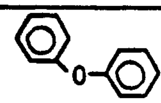 | |
| 986 | N | $F_3C-CH_2-O-$ | N | CH | N | $CH_2$ | H | 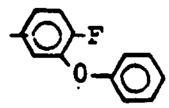 | |
| 987 | N | $F_3C-CH_2-O-$ | N | CH | N | $CH_2$ | H | 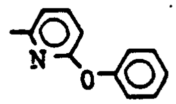 | |
| 988 | N | $F_3C-CH_2-O-$ | N | CH | N | $CH_2$ | H | 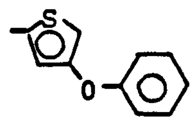 | |
| 989 | N | $F_3C-CH_2-O-$ | N | CH | N | $CH_2$ | H | 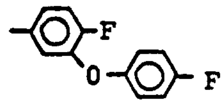 | |
| 990 | N | $F_3C-CH_2-O-$ | N | CH | N | 0 | H | 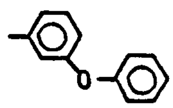 | |
| 991 | N | $F_3C-CH_2-O-$ | N | CH | N | 0 | H | 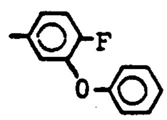 | |
| 992 | N | $F_3C-CH_2-O-$ | N | CH | N | 0 | H | 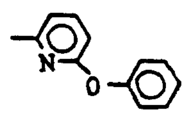 | |

Fortsetzung Tabelle 2

| Verbdg. Nr. | A | $R^1$ | B | C' | D | X | $R^4$ | $R^5$ | physik. Daten |
|---|---|---|---|---|---|---|---|---|---|
| 993 | N | $F_3C-CH_2-O-$ | N | N | N | $CH_2$ | H | 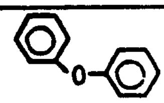 | |
| 994 | N | $F_3C-CH_2-O-$ | N | N | N | $CH_2$ | H | 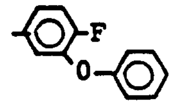 | |
| 995 | N | $F_3C-CH_2-O-$ | N | N | N | $CH_2$ | H | 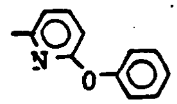 | |
| 996 | N | $F_3C-CH_2-O-$ | N | N | N | $CH_2$ | H | 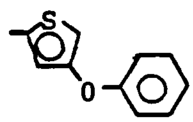 | |
| 997 | N | $F_3C-CH_2-O-$ | N | N | N | $CH_2$ | H | 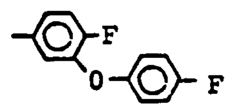 | |
| 998 | N | $F_3C-CH_2-O-$ | N | N | N | O | H | 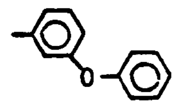 | |
| 999 | N | $F_3C-CH_2-O-$ | N | N | N | O | H | 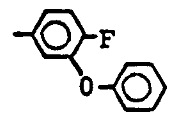 | |
| 1000 | N | $F_3C-CH_2-O-$ | N | N | N | O | H | 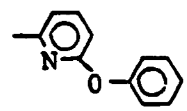 | |

## C. Biologische Beispiele

### Beispiel 1

Mit Kundebohnenblattlaus (Aphis craccivora) stark besetzte Ackerbohnen (Vicia faba) wurden mit wäßrigen Verdünnungen von Emulsionskonzentraten mit 1 000 ppm Wirkstoffgehalt bis zum Stadium des beginnenden Abtropfens besprüht. Nach 3 Tagen betrug die Mortalität jeweils 100 % bei den Präparaten mit den Wirkstoffen der Beispiele 1, 2, 5, 8, 9, 18, 19, 41, 199, 200, 205, 206, 232, 238, 239, 312, 342, 343, 357, 424, 425, 429, 430, 440, 441, 445, 446, 462, 497, 503, 504 und 513.

### Beispiel 2

Mit Weißer Fliege (Trialeurodes Vaporarium) stark besetzte Bohnenpflanzen (Phaseolus vulgaris) wurden mit wäßrigen Verdünnungen von Emulsionskonzentraten (1 000 ppm Wirkstoffgehalt) bis zum beginnenden Abtropfen gespritzt. Nach Aufstellen der Pflanzen im Gewächshaus erfolgt nach 14 Tragen die

mikroskopische Kontrolle, mit dem Ergebnis jeweils 100 %iger Mortalität bei den Präparaten mit den Wirkstoffen der Beispiele 2, 8, 9, 18, 206, 239, 424, 425, 429, 430, 440, 441, 445, 446 und 503.

**Beispiel 3**

| | |
|---|---|
| Versuchsdurchführung: | analog Beispiel 2 |
| Versuchstier: | Tetranychus urticae (Bohnenspinnmilbe) |
| Versuchspflanze: | Phaseolus vulgaris (Buschbohne) |
| Aufwandmenge: | 1 000 ppm Wirkstoff in der Spritzbrühe |

Die nach 8 Tagen ermittelte Wirksamkeit erbrachte für die Verbindungen 425, 429, 430, 445 und 446 100 % Mortalität.

**Beispiel 4**

Mit Citrus-Schmierlaus (Pseudococcus citri) stark befallene Bohnenpflanzen (Phaseodus vulgaris) wurden mit wäßrigen Verdünnungen von Emulsionskonzentraten (jeweils 1 000 ppm Wirkstoff in der Spritzbrühe) bis zum Stadium beginnenden Abtropfens besprüht.

Nach einer Standzeit von 7 Tagen im Gewächshaus bei 20 - 25 °C erfolgte die Kontrolle.

100 % Mortalität wurde für die Verbindungen gemäß Beispiel 2, 5, 8, 9, 18, 19, 41, 200, 205, 206, 238, 342, 424, 429, 430, 446, 503 und 504 festgestellt.

**Beispiel 5**

Baumwollwanzen (Oncopeltus fasciatus) wurden mit wäßbrigen Verdünnungen von Emulsionskonzentraten (jeweils 1 000 ppm Wirkstoff in der Spritzbrühe) der Wirkstoffe aus Beispiel 2, 9, 19, 206, 425, 430, 441, 446 und 504 behandelt. Anschließend wurden die Wanzen in mit luftdurchlässigen Deckeln versehenen Behältern bei Zimmertemperatur aufgestellt.

5 Tage nach der Behandlung wurde die Mortalität festgestellt und betrug in Jedem Einzelfall 100 %.

**Beispiel 6**

Die Bodeninnenseiten von mit künstlichem Nährmedium beschichteten Petrischalen wurden nach dem Erstarren des Futterbreis mit jeweils 3 ml einer 2 000 ppm an Wirkstoff enthaltenden, wäßrigen Emulsion besprüht. Nach Abrocknen des Spritzbelages und Einsetzen von 10 Larven des gemeinen Baumwollwurms (Prodenia litura) wurden die Schalen 7 Tage bei 21 °C aufbewahrt und dann der Wirkungsgrad der jeweiligen Verbindung (ausgedrückt in % Mortalität) bestimmt. Die Verbindungen 1, 2, 5, 8, 9, 18, 19, 41, 200, 205, 206, 239, 424, 425, 430, 445, 446, 503 und 504 ergaben in diesem Test eine Wirksamkeit von jeweils 100 %.

**Beispiel 7**

Blätter der Bohne (Phaseolus vulgaris) wurden mit einer wäßrigen Emulsion der Verbindung aus Beispiel 1 in einer Konzentration von 1 000 ppm (bezogen auf Wirkstoff) behandelt und zu gleich behandelten Larven des Mexikanischen Bohnenkäfers (Epilachna varivestis) in Beobachtungskäfige gestellt. Eine Auswertung nach 48 Stunden ergab eine 100 %ige Abtötung der Versuchstiere. Als gleichermaßen wirksam erwiesen sich die Verbindungen gemäß Beispiel 2, 8, 9, 18, 19, 41, 200, 206, 239, 342, 424, 425, 429, 430, 441, 445, 446, 497, 503 und 504.

**Beispiel 8**

Auf die Innenseite des Deckels und des Bodens einer Petrischale wurden mittels einer Pipette 1 ml aus Beispiel 1 als Wirkstoff in Aceton mit einer Konzentration von 1 000 ppm gleichmäßig aufgetragen und bis zur vollständigen Verdunstung des Lösungsmittels die Schale offen belassen. Danach wurden je 10 Stubenfliegen (Musca domestica) in die Petrischalen gesetzt, die Schalen mit dem Deckel verschlossen und nach 3 Stunden eine 100 %ige Abtötung der Versuchstiere festgestellt. Ebenso wirksam erwiesen sich die Verbindungen gemäß Beispiel 2, 8, 9, 18, 19, 41, 200, 206, 238, 312, 424, 425, 429, 430, 440, 441, 445, 446, 503, 504 und 513.

**Beispiel 9**

Mit Hilfe einer Pipette wurde auf die Innenseite des Deckels und des Bodens einer Petrischale jeweils 1 ml einer Wirkstofflösung in Aceton mit einer Konzentration von 2 000 ppm gleichmäßig aufgetragen. Nach vollständigem Verdunsten des Lösungsmittel wurden je Petrischale 10 Larven (L4) der deutschen Schabe (Blatella germanica) eingesetzt und die Schalen mit Deckeln verschlossen. Nach 72 h wurde die Wirkung (ausgedrückt in % Mortalität) festgestellt. Die Verbindungen 1, 2, 8, 9, 18, 19, 41, 200, 206, 239, 312, 424, 425, 429, 430, 441, 446, 503, 504 und 513 ergaben in diesem Test Wirksamkeit von jeweils 100 %.

**Patentansprüche**
**Patentansprüche für folgende Vertragsstaaten : AT, BE, CH, DE, FR, GB, GR, IT, LI, NL**

**1.** Verbindungen der Formel I und deren Steroisomere

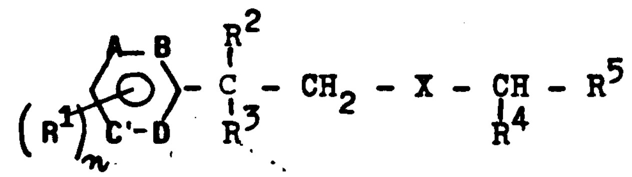

(I),

worin

| | |
|---|---|
| A,B,C',D = | unabhängig voneinander CH oder N, wobei mindestens eines der Symbole A,B,C',D einem Stickstoffatom entsprechen muß, |
| X = | $CH_2$ oder Sauerstoff, |
| $R^1$ = | ein an ein C-Atom gebundener Rest, der H, $(C_1\text{-}C_4)$-Alkyl, Tri$(C_1\text{-}C_4)$alkylsilyl, Halogen, Nitro, Cyano, $(C_2\text{-}C_6)$Alkenyl, $(C_2\text{-}C_6)$-Alkinyl, Amino, $(C_3\text{-}C_7)$Cycloalkyl, Phenyl, Phenoxy, $(C_1\text{-}C_5)$Alkoxy, $(C_2\text{-}C_4)$-Alkenyloxy, $(C_2\text{-}C_4)$Alkinyloxy, Hydroxycarbonyl, $(C_1\text{-}C_4)$-Alkylthio, $(C_3\text{-}C_7)$Cycloalkyloxy, $(C_1\text{-}C_6)$Alkylcarbonyl, $(C_1\text{-}C_4)$Alkoxycarbonyl, $(C_2\text{-}C_4)$Alkenyloxycarbonyl, $(C_3\text{-}C_5)$Alkinyloxycarbonyl, $(C_1\text{-}C_4)$-Halogenalkyl, $(C_1\text{-}C_4)$-Alkoxy$(C_1\text{-}C_4)$alkyl, $(C_1\text{-}C_3)$Halogenalkoxy, $(C_1\text{-}C_3)$Halogenalkylthio, Halogen$(C_1\text{-}C_4)$alkoxy$(C_1\text{-}C_4)$alkyl, $(C_1\text{-}C_4)$-Alkylthio$(C_1\text{-}C_4)$alkyl, $(C_1\text{-}C_4)$Alkoxy$(C_1\text{-}C_4)$alkoxy, Halogen$(C_1\text{-}C_4)$alkoxy$(C_1\text{-}C_4)$alkoxy, $(C_2\text{-}C_4)$Alkenyloxy$(C_1\text{-}C_4)$alkoxy, Halogen$(C_2\text{-}C_4)$alkenyloxy, $(C_1\text{-}C_4)$Alkoxy$(C_1\text{-}C_4)$alkylthio, $(C_1\text{-}C_4)$Alkylthio$(C_1\text{-}C_4)$alkoxy, $(C_1\text{-}C_4)$-Alkylthio$(C_1\text{-}C_4)$alkylthio, Halogen$(C_1\text{-}C_4)$alkoxycarbonyl, Halogen$(C_2\text{-}C_4)$-alkenyloxycarbonyl oder Di$(C_1\text{-}C_6$alkyl)amino oder zwei Reste $R^1$ wenn sie ortho ständig zueinander orientiert sind zusammen einen Methylendioxy-, Ethylendioxy- oder $(C_3\text{-}C_5)$Alkylenrest, |
| $R^2$,$R^3$ = | unabhängig voneinander $(C_1\text{-}C_3)$Alkyl, $(C_2\text{-}C_8)$Alkenyl, Phenyl oder $R^2$ und $R^3$ eine Alkglenkette, die - zusammen mit dem quartären Kohlenstoffatom-einen unsubstituierten oder fluor-substituierten Ring mit drei bis sechs Ringgliedern ergibt, |
| $R^4$ = | -H, F, $-CCl_3$, -C≡CH, $(C_1\text{-}C_4)$Alkyl, |

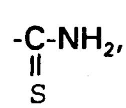

| | |
|---|---|
| $R^5$ = | Phenylrest der Formel (A) |

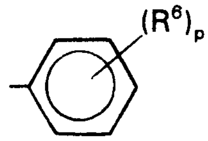

(A),

worin
$R^6$ = unabhängig voneinander H, Halogen, $(C_1-C_4)$Alkyl, $(C_1-C_4)$Alkoxy, $(C_1-C_4)$Halogenalkyl, Phenyl oder N-Pyrrolyl oder einen Rest der Formel (B) bedeutet

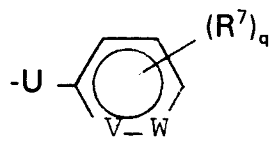

(B),

worin
$R^7$ = unabhängig voneinander H, Halogen, $(C_1-C_4)$Alkyl, $(C_1-C_4)$Alkoxy oder $(C_1-C_4)$Halogenalkyl
U = $-CH_2-$,
$>$C = O, -O- oder -S-,
V, W = CH oder N, wobei für V = N, W = CH bedeuten muß und vice versa,
p = eine ganze Zahl von 1 bis 5 und
q = 1 oder 2 oder
$R^5$ = eine mono- oder disubstituierte Pyridyl-Gruppe der allgemeinen Formel (C),

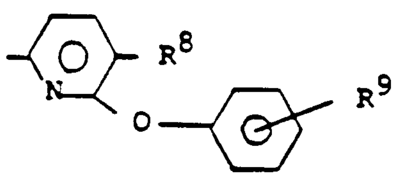

(C),

worin
$R^8$ = Halogen oder H und
$R^9$ = Wasserstoff, halogen außer Jod, $(C_1-C_4)$Alkyl, $(C_1-C_4)$Alkoxy oder $(C_1-C_4)$Halogenalkyl oder
$R^5$ = ein Heterocyclus der allgemeinen Formel (D)

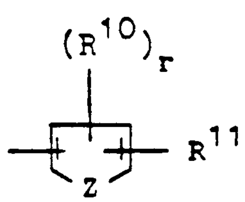

(D)

worin
Z = S,
$R^{10}$ = H, Halogen, $(C_1-C_4)$Alkyl, $(C_1-C_4)$Alkoxy, $(C_1-C_4)$Halogenalkyl, CN oder $NO_2$,
$R^{11}$ = gegebenenfalls substituiertes Benzyl, Propargyl, Allyl oder Phenoxy und
r = 1 oder 2
bedeutet.

**2.** Verfahren zur Herstellung der Verbindungen der Formel I, dadurch gekennzeichnet, daß man
a) für Verbindungen mit X = O eine Verbindung der Formel (II) oder (III)

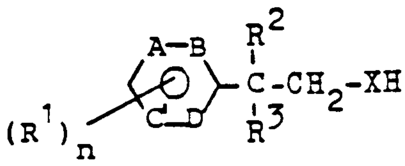 (II) 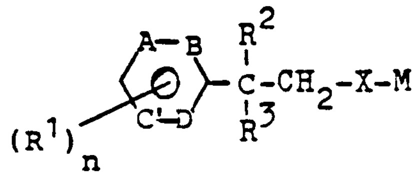 (III)

mit einem Alkylierungsmittel der Formel IV,

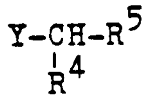 (IV),

worin Y eine nucleofuge Abgangsgruppe wie z. B. Halogen oder Sulfonat bedeutet und M einem Alkalimetall- oder Erdalkalimetall-Äquivalent, insbesondere Li, Na, K, Mg, entspricht,
gegebenenfalls in Gegenwart einer Base,
oder
b) für Verbindungen mit X = O eine Verbindung der Formel (V)

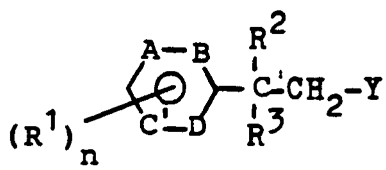 (V)

mit einer XH-aciden Verbindung des Typs (VI)

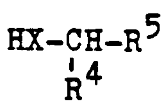 (VI)

in Gegenwart einer Base
oder mit einer metallorganischen Verbindung des Typs VII

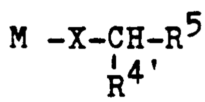 (VII),

mit $R^{4'}$ = H oder $(C_1-C_4)$Alkyl,
oder
c) für Verbindungen mit X = $CH_2$ eine Verbindung der allgemeinen Formel VIII

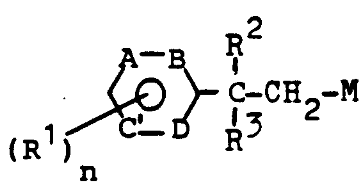 (VIII)

mit einer Verbindung des Typs (IX)

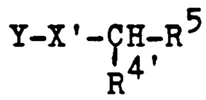 (IX) ,

mit X' $=CH_2$,
oder
d) eine Verbindung der Formel (X)

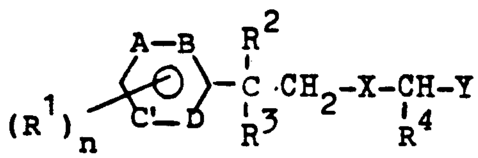 (X)

mit einem metallorganischen Reagenz des Typs (XI)

M - $R^5$ (XI)

oder
e) für Verbindungen mit $R^4 = H$ und $X = CH_2$ einen Aldehyd der Formel (XII)

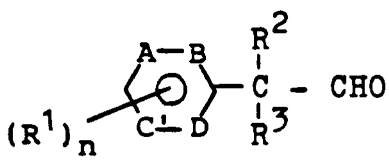 (XII)

mit einem Keton der Formel (XIII)

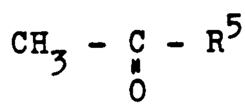 (XIII)

zu einer Zwischenstufe (XIV)

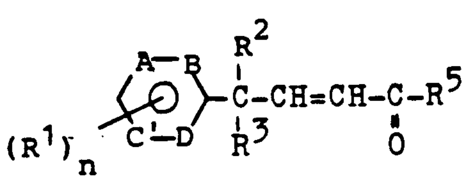 (XIV)

kondensiert und diese anschließend auf übliche Weise reduziert,
oder
f) für Verbindungen mit $X = CH_2$ einen Aldehyd der Formel (XV)

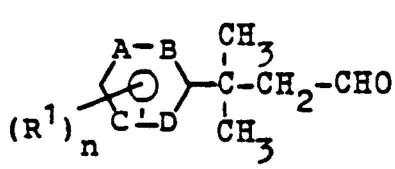 (XV)

mit einem Ylid der Formel (XVI) oder einem Phosphonester der Formel (XVII)

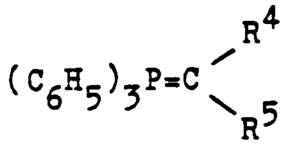 (XVI), 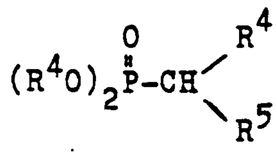 (XVII)

zu einer Zwischenstufe (XVIII)

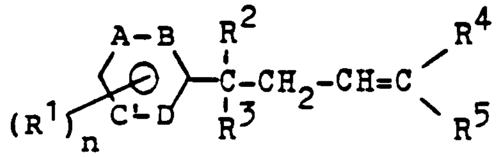 (XVIII)

kondensiert und diese anschließend mit einem Reduktionsmittel zum Endprodukt (I) mit $X = CH_2$ umsetzt.

**3.** Insektizide oder akarizide Mittel, die eine Verbindung der Formel I in einer wirksamen Menge und übliche Formulierungshilfsmittel enthalten.

**4.** Verwendung der Verbindungen von Formel I zur Bekämpfung von Insekten oder Akariden.

**5.** Verfahren zur Bekämpfung von Insekten oder Akariden, dadurch gekennzeichnet, daß man diese oder deren Lebensraum mit einer wirksamen Menge einer Verbindung der Formel I behandelt.

**Patentansprüche für folgenden Vertragsstaat : ES**

**1.** Verfahren zur Herstellung von Verbindungen der Formel I und deren Stereoisomere

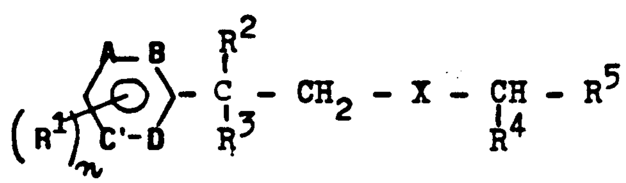 (I),

worin

A,B,C′,D = unabhängig voneinander CH oder N, wobei mindestens eines der Symbole A,B,C′,D einem Stickstoffatom entsprechen muß,

X = $CH_2$ oder Sauerstoff,

$R^1$ = ein an ein C-Atom gebundener Rest, der H, $(C_1-C_4)$-Alkyl, Tri$(C_1-C_4)$alkylsilyl, Halogen, Nitro, Cyano, $(C_2-C_6)$Alkenyl, $(C_2-C_6)$-Alkinyl, Amino, $(C_3-C_7)$Cycloalkyl, Phenyl, Phenoxy, $(C_1-C_5)$Alkoxy, $(C_2-C_4)$-Alkenyloxy, $(C_2-C_4)$Alkinyloxy, Hydroxycarbonyl, $(C_1-C_4)$-Alkylthio, $(C_3-C_7)$Cycloalkyloxy, $(C_1-C_6)$Alkylcarbonyl, $(C_1-C_4)$Alkoxycarbonyl, $(C_2-C_4)$Alkenyloxycarbonyl, $(C_3-C_5)$Alkinyloxycarbonyl, $(C_1-C_4)$-Halogenalkyl, $(C_1-C_4)$-Alkoxy$(C_1-C_4)$alkyl, $(C_1-C_3)$Halogenalkoxy, $(C_1-C_3)$Halogenalkylthio, Halogen$(C_1-C_4)$alkoxy$(C_1-C_4)$alkyl, $(C_1-C_4)$-Alkylthio$(C_1-C_4)$alkyl, $(C_1-C_4)$Alkoxy$(C_1-C_4)$alkoxy, Halogen$(C_1-C_4)$alkoxy$(C_1-C_4)$alkoxy, $(C_2-C_4)$Alkenyloxy$(C_1-C_4)$alkoxy, Halogen$(C_2-C_4)$alkenyloxy, $(C_1-C_4)$Alkoxy$(C_1-C_4)$alkylthio, $(C_1-C_4)$Alkylthio$(C_1-C_4)$alkoxy, $(C_1-C_4)$-Alkylthio$(C_1-C_4)$alkylthio, Halogen$(C_1-C_4)$alkoxycarbonyl, Halogen$(C_2-C_4)$-alkenyloxycarbonyl oder Di$(C_1-C_6$alkyl)amino oder zwei Reste $R^1$ wenn sie ortho ständig zueinander orientiert sind zusammen einen Methylendioxy-, Ethylendioxy-

oder ($C_3$-$C_5$)Alkylenrest,

$R^2$,$R^3$ = unabhängig voneinander ($C_1$-$C_3$)Alkyl, ($C_2$-$C_8$)Alkenyl, Phenyl oder $R^2$ und $R^3$ eine Alkylenkette, die - zusammen mit dem quartären Kohlenstoffatom-einen unsubstituierten oder fluor-substituierten Ring mit drei bin sechs Ringgliedern ergibt,

$R^4$ = -H, F, $-CCl_3$, -C≡CH, ($C_1$-$C_4$)Alkyl,

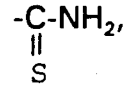

$R^5$ = Phenylrest der Formel (A)

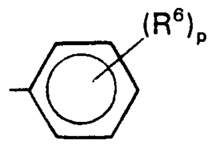

(A),

worin
$R^6$ = unabhängig voneinander H, Halogen, ($C_1$-$C_4$)Alkyl, ($C_1$-$C_4$)Alkoxy, ($C_1$-$C_4$)Halogenalkyl, Phenyl oder N-Pyrrolyl oder einen Rest der Formel (B) bedeutet

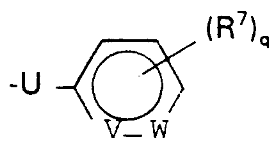

(B),

worin
$R^7$ = unabhängig voneinander H, Halogen, ($C_1$-$C_4$)Alkyl, ($C_1$-$C_4$)Alkoxy oder ($C_1$-$C_4$)Halogenalkyl
U = $-CH_2-$,
$>$C = O, -O- oder -S-,
V, W = CH oder N, wobei für V = N, W = CH bedeuten muß und vice versa,
p = eine ganze Zahl von 1 bis 5 und
q = 1 oder 2 oder
$R^5$ = eine mono- oder disubstituierte Pyridyl-Gruppe der allgemeinen Formel (C),

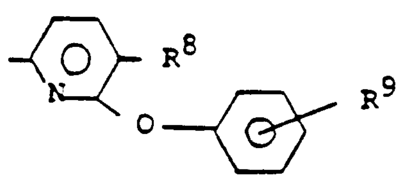

(C) ,

worin
$R^8$ = Halogen oder H und
$R^9$ = Wasserstoff, halogen außer Jod, ($C_1$-$C_4$)Alkyl, ($C_1$-$C_4$)Alkoxy oder ($C_1$-$C_4$)Halogenalkyl oder
$R^5$ = ein Heterocyclus der allgemeinen Formel (D)

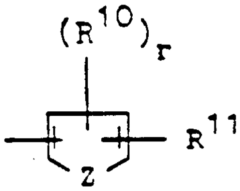 (D)

worin
Z = S,
$R^{10}$ = H, Halogen, $(C_1\text{-}C_4)$Alkyl, $(C_1\text{-}C_4)$Alkoxy, $(C_1\text{-}C_4)$Halogenalkyl, CN oder $NO_2$,
$R^{11}$ = gegebenenfalls substituiertes Benzyl, Propargyl, Allyl oder Phenoxy und
r = 1 oder 2
bedeutet.

a) für Verbindungen mit X = O eine Verbindung der Formel (II) oder (III)

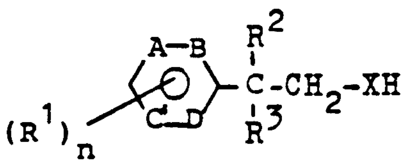 (II) 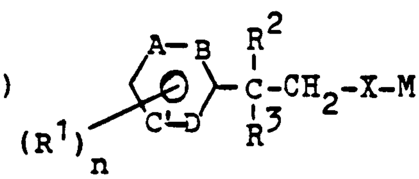 (III)

mit einem Alkylierungsmittel der Formel IV,

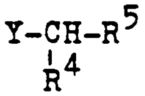 (IV),

worin Y eine nucleofuge Abgangsgruppe wie z. B. Halogen oder Sulfonat bedeutet und M einem Alkalimetall- oder Erdalkalimetall-Äquivalent, insbesondere Li, Na, K, Mg, entspricht,
gegebenenfalls in Gegenwart einer Base,
oder

b) für Verbindungen mit X = O eine Verbindung der Formel (V)

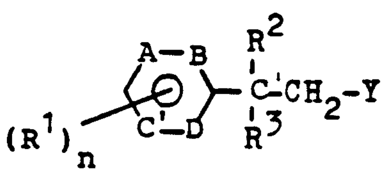 (V)

mit einer XH-aciden Verbindung des Typs (VI)

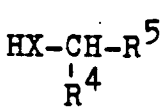 (VI)

in Gegenwart einer Base
oder mit einer metallorganischen Verbindung des Typs VII

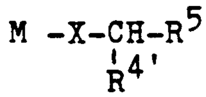 (VII),

mit $R^{4'}$ = H oder $(C_1\text{-}C_4)$Alkyl,
oder
c) für Verbindungen mit X = $CH_2$ eine Verbindung der allgemeinen Formel VIII

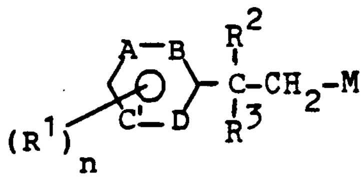 (VIII)

mit einer Verbindung des Typs (IX)

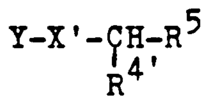 (IX),

mit X' = $CH_2$,
oder
d) eine Verbindung der Formel (X)

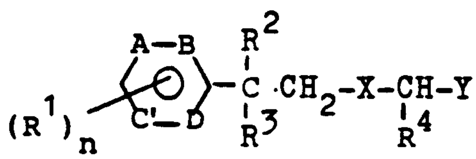 (X)

mit einem metallorganischen Reagenz des Typs (XI)

M - $R^5$ (XI)

oder
e) für Verbindungen mit $R^4$ = H und X = $CH_2$ einen Aldehyd der Formel (XII)

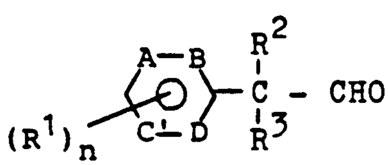 (XII)

mit einem Keton der Formel (XIII)

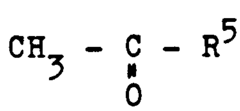 (XIII)

zu einer Zwischenstufe (XIV)

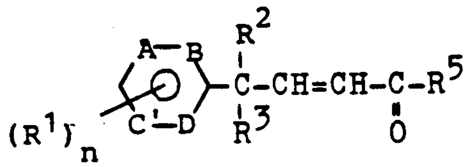

(XIV)

kondensiert und diese anschließend auf übliche Weise reduziert, oder

f) für Verbindungen mit X = $CH_2$ einen Aldehyd der Formel (XV)

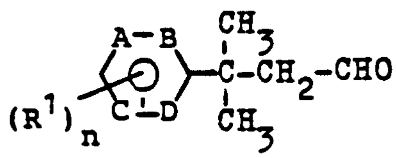

(XV)

mit einem Ylid der Formel (XVI) oder einem Phosphonester der Formel (XVII)

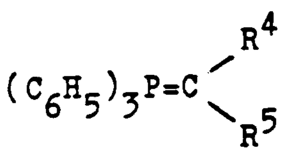

(XVI) ,

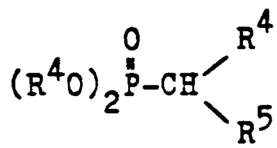

(XVII)

zu einer Zwischenstufe (XVIII)

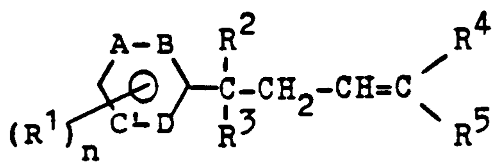

(XVIII)

kondensiert und diese anschließend mit einem Reduktionsmittel zum Endprodukt (I) mit X = $CH_2$ umsetzt.

**2.** Insektizide oder akarizide Mittel, die eine Verbindung der Formel I in einer wirksamen Menge und übliche Formulierungshilfsmittel enthalten.

**3.** Verwendung der Verbindungen von Formel I zur Bekämpfung von Insekten oder Akariden.

**4.** Verfahren zur Bekämpfung von Insekten oder Akariden, dadurch gekennzeichnet, daß man diese oder deren Lebensraum mit einer wirksamen Menge einer Verbindung der Formel I behandelt.

## Claims

## Claims for the following Contracting States : AT, BE, CH, DE, FR, GB, GR, IT, LI, NL

**1.** A compound of the formula I and its stereoisomers

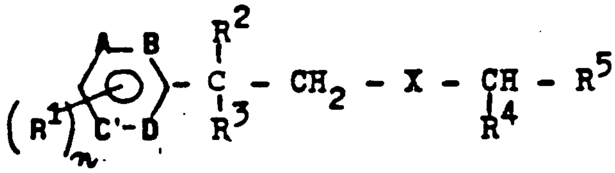 (I)

in which

A, B, C' and D independently of one another are CH or N, where at least one of the symbols A, B, C' and D must correspond to a nitrogen atom,

X is $CH_2$ or oxygen,

$R^1$ is a radical bonded to a carbon atom, from the series comprising H, $(C_1\text{-}C_4)$-alkyl, tri$(C_1\text{-}C_4)$-alkylsilyl, halogen, nitro, cyano, $(C_2\text{-}C_6)$alkenyl, $(C_2\text{-}C_6)$-alkynyl, amino, $(C_3\text{-}C_7)$cycloalkyl, phenyl, phenoxy, $(C_1\text{-}C_5)$alkoxy, $(C_2\text{-}C_4)$alkenyloxy, $(C_2\text{-}C_4)$-alkynyloxy, hydroxycarbonyl, $(C_1\text{-}C_4)$alkylthio, $(C_3\text{-}C_7)$cycloalkyloxy, $(C_1\text{-}C_6)$-alkylcarbonyl, $(C_1\text{-}C_4)$-alkoxycarbonyl, $(C_2\text{-}C_4)$alkenyloxycarbonyl, $(C_3\text{-}C_5)$-alkynyloxycarbonyl, $(C_1\text{-}C_4)$haloalkyl, $(C_1\text{-}C_4)$-alkoxy$(C_1\text{-}C_4)$alkyl, $(C_1\text{-}C_3)$haloalkoxy, $(C_1\text{-}C_3)$-haloalkylthio, halo$(C_1\text{-}C_4)$alkoxy$(C_1\text{-}C_4)$alkyl, $(C_1\text{-}C_4)$-alkylthio$(C_1\text{-}C_4)$alkyl, $(C_1\text{-}C_4)$alkoxy$(C_1\text{-}C_4)$alkoxy, halo$(C_1\text{-}C_4)$alkoxy$(C_1\text{-}C_4)$alkoxy, $(C_2\text{-}C_4)$alkenyloxy-$(C_1\text{-}C_4)$alkoxy,halo$(C_2\text{-}C_4)$alkenyloxy, $(C_1\text{-}C_4)$alkoxy$(C_1\text{-}C_4)$alkylthio, $(C_1\text{-}C_4)$-alkylthio$(C_1\text{-}C_4)$alkoxy, $(C_1\text{-}C_4)$alkylthio$(C_1\text{-}C_4)$alkylthio, halo$(C_1\text{-}C_4)$-alkoxycarbonyl, halo$(C_2\text{-}C_4)$alkenyloxycarbonyl or di$(C_1\text{-}C_6\text{-alkyl})$amino or two radicals $R^1$ when they are positioned ortho to one another together denote a methylenedioxy, ethylenedioxy or $(C_3\text{-}C_5)$alkylene radical,

$R^2$ and $R^3$ independently of one another are $(C_1\text{-}C_3)$-alkyl, $(C_2\text{-}C_8)$alkenyl or phenyl, or $R^2$ and $R^3$ are an alkylene chain which - together with the quaternary carbon atom - forms an unsubstituted or fluorine-substituted ring having three to six ring members,

$R^4$ is -H, F, $-CCl_3$, -C≡CH, $(C_1\text{-}C_4)$alkyl,

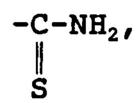

$R^5$ is a phenyl radical of the formula (A)

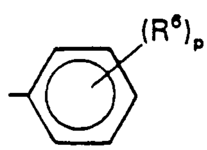 (A)

in which

$R^6$ independently of one another are H, halogen, $(C_1\text{-}C_4)$alkyl, $(C_1\text{-}C_4)$alkoxy, $(C_1\text{-}C_4)$haloalkyl, phenyl or N-pyrrolyl or a radical of the formula (B)

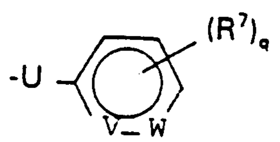 (B)

in which

$R^7$ independently of one another are H, halogen, $(C_1\text{-}C_4)$alkyl, $(C_1\text{-}C_4)$alkoxy or $(C_1\text{-}C_4)$-haloalkyl,

U is $-CH_2-$,
>C is O, -O- or -S-,
V and W are CH or N, where if V is N, W must be CH and vice versa,
p is an integer from 1 to 5 and
q is 1 or 2 or
$R^5$ is a mono- or disubstituted pyridyl group of the formula (C)

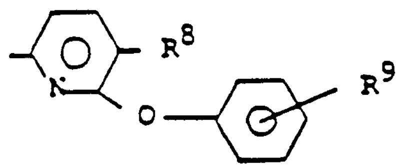
(C)

in which
$R^8$ is halogen or H and
$R^9$ is hydrogen, halogen apart from iodine, $(C_1-C_4)$alkyl, $(C_1-C_4)$alkoxy or $(C_1-C_4)$haloalkyl or
$R^5$ is a heterocycle of the formula (D)

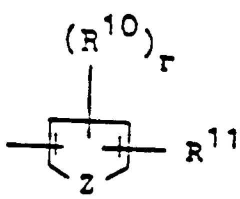
(D)

in which
Z is S,
$R^{10}$ is H, halogen, $(C_1-C_4)$alkyl, $(C_1-C_4)$alkoxy, $(C_1-C_4)$haloalkyl, CN or $NO_2$,
$R^{11}$ is optionally substituted benzyl, propargyl, allyl or phenoxy and
r is 1 or 2.

**2.** A process for the preparation of the compounds of the formula I, which comprises
a) for compounds where X = O condensing a compound of the formula (II) or (III)

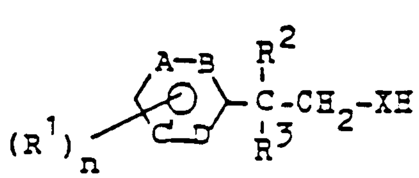
(II)
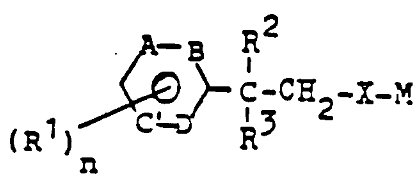
(III)

with an alkylating agent of the formula IV

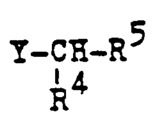
(IV)

in which Y is a nucleofugic leaving group such as, for example, halogen or sulfonate and M corresponds to an alkali metal equivalent or an alkaline earth metal equivalent, in particular Si, Na, K or Mg, if appropriate in the presence of a base, or
b) for compounds where X = O condensing a compound of the formula (V)

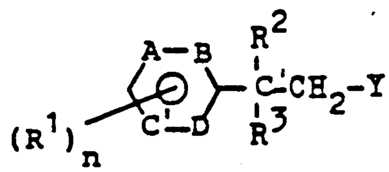

(V)

with an XH-acid compound of the type (VI)

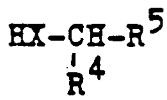

(VI)

in the presence of a base
or with an organometallic compound of the type VII

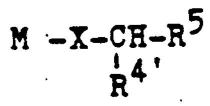

(VII)

where $R^{4'}$ = H or $(C_1\text{-}C_4)$alkyl
or
c) for compounds where X = $CH_2$ condensing a compound of the formula VIII

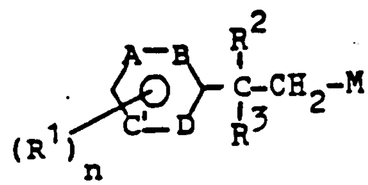

(VIII)

with a compound of the type (IX)

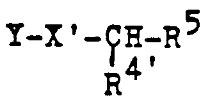

(IX)

where X' = $CH_2$,
or
d) condensing a compound of the formula (X)

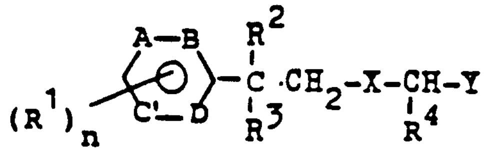

(X)

with an organometallic reagent of the type (XI)

M-$R^5$ (XI)

or

e) for compounds where $R^4$ = H and X = $CH_2$ condensing an aldehyde of the formula (XII)

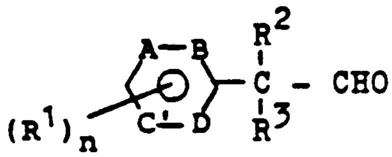

(XII)

with a ketone of the formula (XIII)

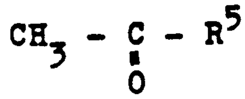

(XIII)

to form an intermediate (XIV)

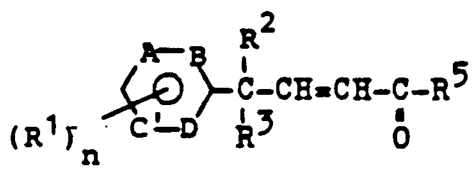

(XIV)

and reducing the latter subsequently in a customary manner,

or

f) for compounds where X = $CH_2$ condensing an aldehyde of the formula (XV)

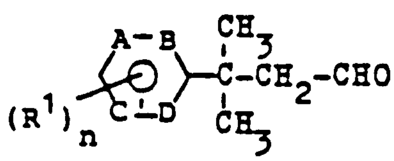

(XV)

with an ylide of the formula (XVI) or a phosphonate of the formula (XVII)

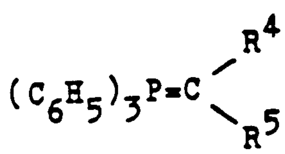

(XVI)

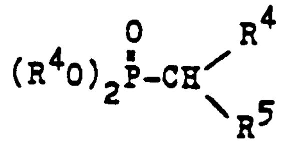

(XVII)

to form an intermediate (XVIII)

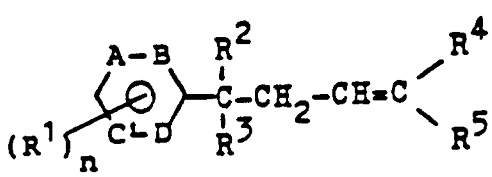

(XVIII)

and reacting the latter subsequently with a reductant to form the final product (I) where X = $CH_2$.

**3.** An insecticidal or acaricidal agent which contains a compound of the formula I in an active amount and customary formulation auxiliaries.

**4.** The use of the compounds of the formula I for combating insects or acarids.

**5.** A process for combating insects or acarids, which comprises treating them or their environment with an active amount of a compound of the formula I.

**Claims for the following Contracting State : ES**

**1.** A process for the preparation of compounds of the formula I and their stereoisomers

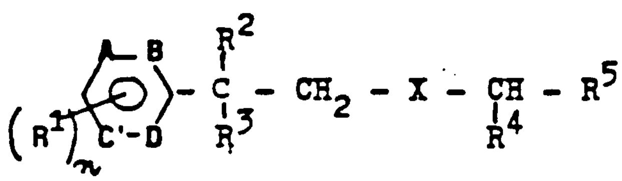

(I)

in which

A, B, C' and D independently of one another are CH or N, where at least one of the symbols A, B, C' and D must correspond to a nitrogen atom,

X is $CH_2$ or oxygen,

$R^1$ is a radical bonded to a carbon atom, from the series comprising H, ($C_1$-$C_4$)-alkyl, tri($C_1$-$C_4$)-alkylsilyl, halogen, nitro, cyano, ($C_2$-$C_6$)alkenyl, ($C_2$-$C_6$)-alkynyl, amino, ($C_3$-$C_7$)cycloalkyl, phenyl, phenoxy, ($C_1$-$C_5$)alkoxy, ($C_2$-$C_4$)alkenyloxy, ($C_2$-$C_4$)-alkynyloxy, hydroxycarbonyl, ($C_1$-$C_4$)alkylthio, ($C_3$-$C_7$)cycloalkyloxy, ($C_1$-$C_6$)-alkylcarbonyl, ($C_1$-$C_4$)-alkoxycarbonyl, ($C_2$-$C_4$)alkenyloxycarbonyl, ($C_3$-$C_5$)-alkynyloxycarbonyl, ($C_1$-$C_4$)haloalkyl, ($C_2$-$C_4$)-alkoxy($C_1$-$C_4$)alkyl, ($C_1$-$C_3$)haloalkoxy, ($C_1$-$C_3$)-haloalkylthio, halo($C_1$-$C_4$)alkoxy($C_1$-$C_4$)alkyl, ($C_1$-$C_4$)-alkylthio($C_1$-$C_4$)alkyl, ($C_1$-$C_4$)alkoxy($C_1$-$C_4$)alkoxy, halo($C_1$-$C_4$)alkoxy($C_1$-$C_4$)alkoxy, ($C_2$-$C_4$)alkenyloxy-($C_1$-$C_4$)alkoxy,halo($C_2$-$C_4$)alkenyloxy, ($C_1$-$C_4$)alkoxy($C_1$-$C_4$)alkylthio, ($C_1$-$C_4$)-alkylthio($C_1$-$C_4$)alkoxy, ($C_1$-$C_4$)alkylthio($C_1$-$C_4$)alkylthio, halo($C_1$-$C_4$)-alkoxycarbonyl, halo($C_2$-$C_4$)alkenyloxycarbonyl or di($C_1$-$C_6$-alkyl)amino or two radicals $R^1$ when they are positioned ortho to one another together denote a methylenedioxy, ethylenedioxy or ($C_3$-$C_5$)alkylene radical,

$R^2$ and $R^3$ independently of one another are ($C_1$-$C_3$)-alkyl, ($C_2$-$C_8$)alkenyl or phenyl, or $R^2$ and $R^3$ are an alkylene chain which - together with the quaternary carbon atom - forms an unsubstituted or fluorine-substituted ring having three to six ring members,

$R^4$ is -H, F, $-CCl_3$, C≡CH, ($C_1$-$C_4$)alkyl,

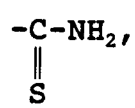

$R^5$ is a phenyl radical of the formula (A)

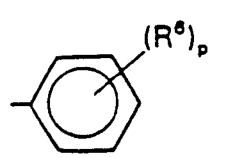

(A)

in which

$R^6$ independently of one another are H, halogen, $(C_1-C_4)$alkyl, $(C_1-C_4)$alkoxy, $(C_1-C_4)$haloalkyl, phenyl or N-pyrrolyl or a radical of the formula (B)

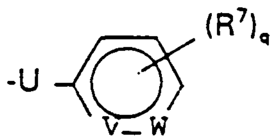

(B)

in which

$R^7$ independently of one another are H, halogen, $(C_1-C_4)$alkyl, $(C_1-C_4)$alkoxy or $(C_1-C_4)$-haloalkyl,

U is $-CH_2-$,

>C is O, -O- or -S-,

V and W are CH or N, where if V is N, W must be CH and vice versa,

p is an integer from 1 to 5 and

q is 1 or 2 or

$R^5$ is a mono- or disubstituted pyridyl group of the formula (C)

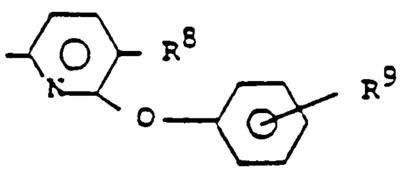

(C)

in which

$R^8$ is halogen or H and

$R^9$ is hydrogen, halogen apart from iodine, $(C_1-C_4)$alkyl, $(C_1-C_4)$alkoxy or $(C_1-C_4)$haloalkyl or

$R^5$ is a heterocycle of the formula (D)

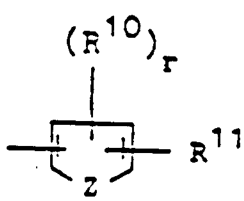

(D)

in which

Z is S,

$R^{10}$ is H, halogen, $(C_1-C_4)$alkyl, $(C_1-C_4)$alkoxy, $(C_1-C_4)$haloalkyl, CN or $NO_2$,

$R^{11}$ is optionally substituted benzyl, propargyl, allyl or phenoxy and

r is 1 or 2,

which comprises

a) for compounds where X = O condensing a compound of the formula (II) or (III)

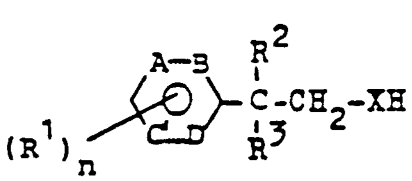

(II)

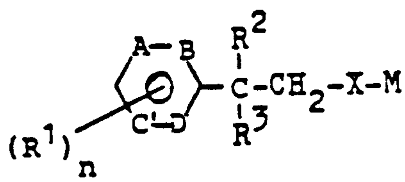

(III)

with an alkylating agent of the formula IV

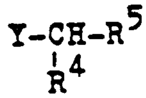 (IV)

in which Y is a nucleofugic leaving group such as, for example, halogen or sulfonate and M corresponds to an alkali metal equivalent or an alkaline earth metal equivalent, in particular Li, Na, K or Mg, if appropriate in the presence of a base, or

b) for compounds where X = O condensing a compound of the formula (V)

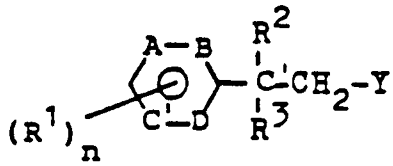 (V)

with an XH-acid compound of the type (VI)

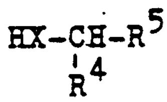 (VI)

in the presence of a base

or with an organometallic compound of the type VII

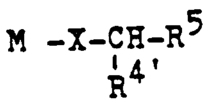 (VII)

where $R^{4'}$ = H or $(C_1\text{-}C_4)$alkyl

or

c) for compounds where X = $CH_2$ condensing a compound of the formula VIII

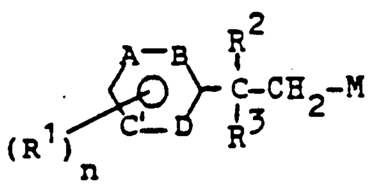 (VIII)

with a compound of the type (IX)

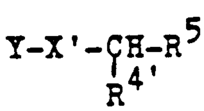 (IX)

where X' = $CH_2$,

or
d) condensing a compound of the formula (X)

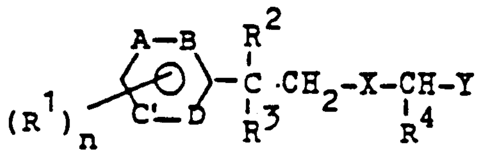

(X)

with an organometallic reagent of the type (XI)

M-$R^5$ (XI)

or
e) for compounds where $R^4$ = H and X = $CH_2$ condensing an aldehyde of the formula (XII)

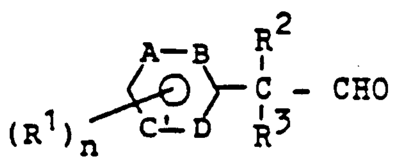

(XII)

with a ketone of the formula (XIII)

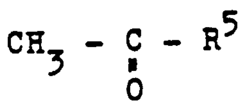

(XIII)

to form an intermediate (XIV)

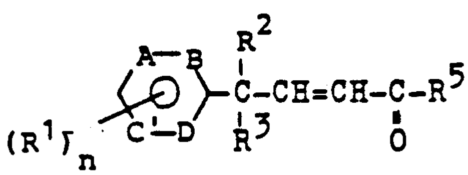

(XIV)

and reducing the latter subsequently in a customary manner,
or
f) for compounds where X = $CH_2$ condensing an aldehyde of the formula (XV)

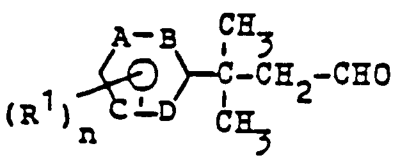

(XV)

with an ylide of the formula (XVI) or a phosphonate of the formula (XVII)

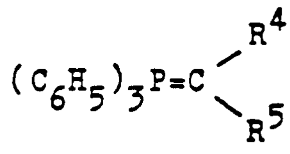

(XVI)

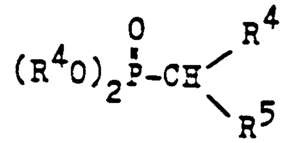

(XVII)

to form an intermediate (XVIII)

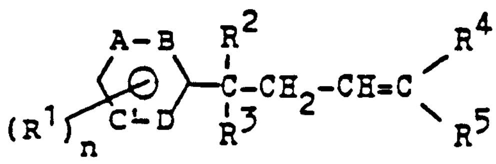

(XVIII)

and reacting the latter subsequently with a reductant to form the final product (I) where X = $CH_2$.

**2.** An insecticidal or acaricidal agent which contains a compound of the formula I in an active amount and customary formulation auxiliaries.

**3.** The use of the compounds of the formula I for combating insects or acarids.

**4.** A process for combating insects or acarids, which comprises treating them or their environment with an active amount of a compound of the formula I.

**Revendications**
**Revendications pour les Etats contractants suivants : AT, BE, CH, DE, FR, GB, GR, IT, LI, NL**

**1.** Composés de formule I ci-dessous et leurs stéréo-isomères :

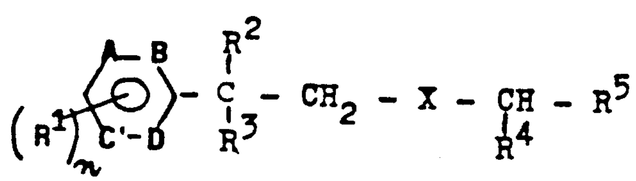

(I)

dans laquelle :

A, B, C', D désignent chacun, indépendamment les uns des autres, CH ou N, mais l'un d'eux au moins devant être un atome d'azote,

$X_1$ désigne un groupe $CH_2$ ou un atome d'oxygène,

$R^1$ un radical lié à un atome de carbone, H, un $(C_1$-$C_4)$alkyle, tri$(C_1$-$C_4)$alkylsilyle, halogène, nitro, cyano, $(C_2$-$C_6)$-alcényle, $(C_2$-$C_6)$alcynyle, amino, $(C_3$-$C_7)$cycloalkyle, phényle, phénoxy, $(C_1$-$C_5)$alcoxy, $(C_2$-$C_4)$alcényloxy, $(C_2$-$C_4)$alcynyloxy, hydroxycarbonyle, $(C_1$-$C_4)$alkylthio, $(C_3$-$C_7)$cycloalcyloxy, $(C_1$-$C_6)$-alkylcarbonyle, $(C_1$-$C_4)$alcoxycarbonyle, $(C_2$-$C_4)$alcényloxycarbonyl, $(C_3$-$C_5)$-alcynyloxycarbonyle, $(C_1$-$C_4)$halogénoalkyle, $(C_1$-$C_4)$alcoxy$(C_1$-$C_4)$alkyle, $(C_1$-$C_3)$-halogénoalcoxy, $(C_1$-$C_3)$halogénoalkylthio, halogéno$(C_1$-$C_4)$alcoxy$(C_1$-$C_4)$alkyle, $(C_1$-$C_4)$alkylthio$(C_1$-$C_4)$alkyle, $(C_1$-$C_4)$alcoxy$(C_1$-$C_4)$alcoxy, halogèno$(C_1$-$C_4)$alcoxy$(C_1$-$C_4)$-alcoxy, $(C_2$-$C_4)$alcényloxy$(C_1$-$C_4)$alcoxy, halogéno$(C_2$-$C_4)$alcényloxy, $(C_1$-$C_4)$alcoxy-$(C_1$-$C_4)$alkylthio, $(C_1$-$C_4)$alkylthio$(C_1$-$C_4)$-alcoxy, $(C_1$-$C_4)$alkylthio$(C_1$-$C_4)$alkylthio, halogéno-$(C_1$-$C_4)$alcoxycarbonyle, halogéno$(C_2$-$C_4)$alcényloxycarbonyle ou di$(C_1$-$C_6$-alkyl)amino, ou bien deux radicaux $R^1$, s'ils sont en position ortho l'un par rapport à l'autre, forment ensemble un radical méthylène-dioxy, éthylène-dioxy ou $(C_3$-$C_5)$-alkylène,

$R^2$ et $R^3$ désignent chacun, indépendamment l'un de l'autre, un $(C_1$-$C_3)$alkyle, un $(C_2$-$C_8)$-

alcényle ou un phényle, ou bien $R^2$ et $R^3$ forment ensemble une chaîne alkylène formant avec l'atome de carbone quaternaire auquel ils sont liés un cycle sans substituants ou fluoré de 3 à 6 chaînons,

$R^4$ représente -H, -F, $-CCl_3$, -C≡CH, un $(C_1-C_4)$alkyle ou un groupe

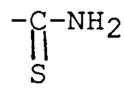 et

$R^5$ un radical phényle de formule A :

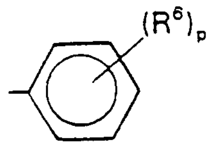

(A),

les $R^6$ désignant chacun, indépendamment les uns des autres, l'hydrogène, un halogène, un alkyle ou un alcoxy en $C_1-C_4$, un $(C_1-C_4)$halogénoalkyle ou le groupe phényle ou N-pyrrolyle,

ou bien un radical de formule B :

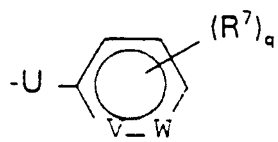

(B),

les $R^7$ désignant chacun, indépendamment l'un de l'autre, l'hydrogène, un halogène ou un alkyle ou un- alcoxy en $C_1-C_4$ ou $(C_1-C_4)$halogénoalkyle ;

U le groupe $-CH_2-$, C=O, -O- ou -S-,

V et W chacun CH ou N mais étant différents l'un de l'autre,

p étant un entier de 1 à 5,

q le nombre 1 ou 2, ou encore

$R^5$ est un groupe pyridyle avec un ou deux substituants, de formule générale C

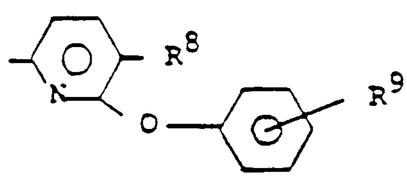

(C)

$R^8$ désignant un halogène ou l'hydrogène,

$R^9$ l'hydrogène, un halogène à l'exception de l'iode, ou bien un alkyle ou un alcoxy en $C_1-C_4$ ou un$(C_1-C_4)$halogénoalkyle, ou encore

$R^5$ est un hétérocyclique de formule générale D

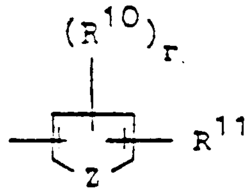

(D)

dans laquelle

Z désigne le soufre,

$R^{10}$ l'hydrogène, un halogène, un alkyle ou un alcoxy en $C_1$-$C_4$, un ($C_1$-$C_4$)halogénoalkyle.ou le groupe CN ou $NO_2$,

$R^{11}$ un groupe benzyle, propargyle, allyle ou phénoxy, éventuellement substités et

r le nombre 1 ou 2.

**2.** Procédés de préparation des composés de formule I, caractérisés en ce que :

a) pour des composés dans lesquels X est un atome d'oxygène on fait réagir un composé de formule II ou III :

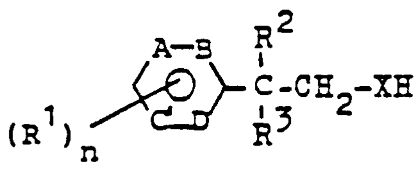

(II)

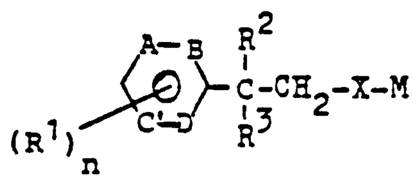

(III)

avec un agent d'alkylation de formule IV :

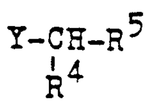

(IV),

Y désignant un radical nucléofuge qui s'élimine tel que par exemple un halogène ou un groupe sulfonate et M un équivalent de métal alcalin ou alcalino-terreux, notamment Li, Na, K ou Mg, éventuellement en présence d'une base,

ou bien

b) pour des composés dans lesquels X est aussi un atome d'oxygène on fait réagir un composé de formule V

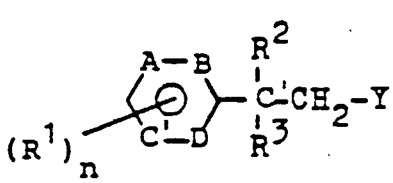

(V)

avec un composé acide XH du genre (VI)

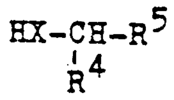

(VI)

en présence d'une base
ou avec un composé organo-métallique du genre VII

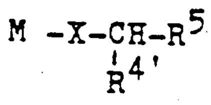

(VII),

$R^{4'}$ désignant l'hydrogène ou un (C1-C4)alkyle,
ou bien
c) pour des composés dans lesquels X est un groupe $CH_2$ on fait réagir un composé de formule générale VIII

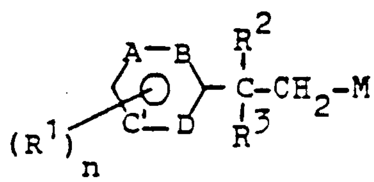

(VIII)

avec un composé du genre (IX)

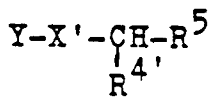

(IX) ,

X' étant le groupe $CH_2$, ou
d) un composé de formule X

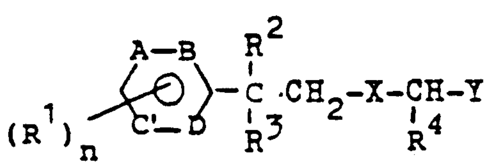

(X)

avec un réactif organo-métallique du genre XI

$M - R^5$ (XI)

ou bien
e) pour des composés dans lesquels $R^4$ est l'hydrogène et X le groupe $CH_2$ on condense un aldéhyde de formule XII

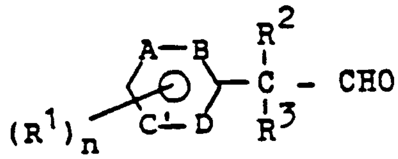

(XII)

avec une cétone de formule XIII

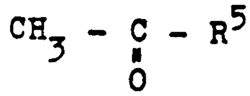

(XIII)

en un produit intermédiaire XIV

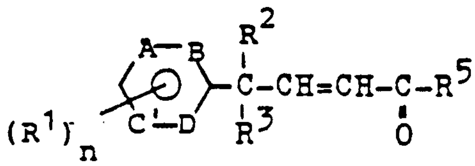

(XIV)

que l'on réduit ensuite de la manière habituelle,
ou encore
f) pour des composés dans lesquels X est le groupe $CH_2$ on condense un aldéhyde de formule XV

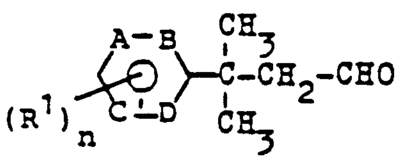

(XV)

avec un ylide de formule XVI ou un ester phosphonique de formule XVII

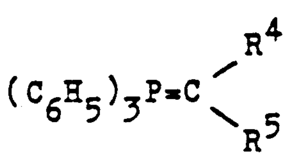

(XVI) ,

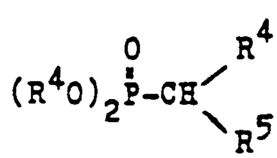

(XVII)

en un produit intermédiaire XVIII

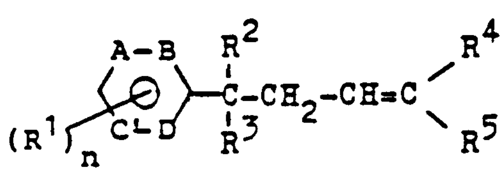

(XVIII)

que l'on transforme ensuite avec un agent réducteur en produit final (I) dans lequel X est le groupe $CH_2$.

**3.** Produits insecticides ou acaricides comprenant un composé de formule I de la revendication 1 dans une proportion appropriée avec des adjuvants de formulation courants.

**4.** L'emploi des composés de formule I de la revendication 1 pour combattre des insectes et acariens.

**5.** Procédé de lutte contre des insectes ou acariens, procédé caractérisé en ce que l'on traite ceux-ci ou leur environnement ou espace vital avec des composés de formule I de la revendication 1.

**Revendications pour l'Etat contractant suivant : ES**

**1.** Procédé de préparation de composés de formule I ci-dessous et leurs stéréo-isomères :

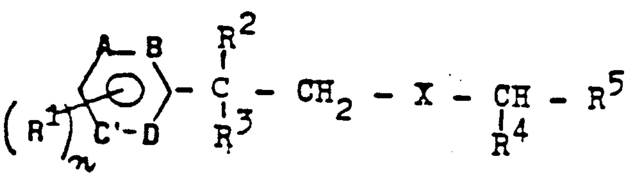

(I),

dans laquelle :

| | |
|---|---|
| A, B, C', D | désignent chacun, indépendamment les uns des autres, CH ou N, mais l'un d'eux au moins devant être un atome d'azote, |
| $X_1$ | désigne un groupe $CH_2$ ou un atome d'oxygène, |
| $R^1$ | un radical lié à un atome de carbone, H, un $(C_1-C_4)$alkyle, tri$(C_1-C_4)$alkylsilyle, halogène, nitro, cyano, $(C_2-C_6)$-alcényle, $(C_2-C_6)$alcynyle, amino, $(C_3-C_7)$cycloalkyle, phényle, phénoxy, $(C_1-C_5)$alcoxy, $(C_2-C_4)$alcényloxy, $(C_2-C_4)$alcynyloxy, hydroxycarbonyle, $(C_1-C_4)$alkylthio, $(C_3-C_7)$cycloalcyloxy, $(C_1-C_6)$-alkylcarbonyle, $(C_1-C_4)$alcoxycarbonyle, $(C_2-C_4)$alcényloxycarbonyl, $(C_3-C_5)$-alcynyloxycarbonyle, $(C_1-C_4)$halogénoalkyle, $(C_1-C_4)$alcoxy$(C_1-C_4)$alkyle, $(C_1-C_3)$-halogénoalcoxy, $(C_1-C_3)$halogénoalkylthio, halogène$(C_1-C_4)$alcoxy$(C_1-C_4)$alkyle, $(C_1-C_4)$alkylthio$(C_1-C_4)$alkyle, $(C_1-C_4)$alcoxy$(C_1-C_4)$alcoxy, halogèno$(C_1-C_4)$alcoxy$(C_1-C_4)$-alcoxy, $(C_2-C_4)$alcényloxy$(C_1-C_4)$alcoxy, halogéno$(C_2-C_4)$alcényloxy, $(C_1-C_4)$alcoxy-$(C_1-C_4)$alkylthio, $(C_1-C_4)$alkylthio$(C_1-C_4)$alcoxy, $(C_1-C_4)$alkylthio$(C_1-C_4)$alkylthio, halogéno-$(C_1-C_4)$alcoxycarbonyle, halogéno$(C_2-C_4)$alcényloxycarbonyle ou di$(C_1-C_6$-alkyl)amino, ou bien deux radicaux $R^1$, s'ils sont en position ortho l'un par rapport à l'autre, forment ensemble un radical méthylène-dioxy, éthylène-dioxy ou $(C_3-C_5)$-alkylène, |
| $R^2$ et $R^3$ | désignent chacun, indépendamment l'un de l'autre, un $(C_1-C_3)$alkyle, un $(C_2-C_8)$-alcényle ou un phényle, ou bien $R^2$ et $R^3$ forment ensemble une chaîne alkylène formant avec l'atome de carbone quaternaire auquel ils sont liés un cycle sans substituants ou fluoré de 3 à 6 chaînons, |
| $R^4$ | représente -H, -F, $-CCl_3$, $-C{\equiv}CH$, un $(C_1-C_4)$alkyle ou un groupe |

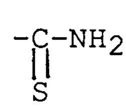 et

| | |
|---|---|
| $R^5$ | un radical phényle de formule A : |

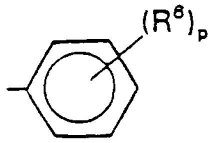

(A),

les $R^6$ désignant chacun, indépendamment les uns des autres, l'hydrogène, un halogène, un alkyle ou un alcoxy en $C_1$-$C_4$, un ($C_1$-$C_4$)halogénoalkyle ou le groupe phényle ou N-pyrrolyle,

ou bien un radical de formule B :

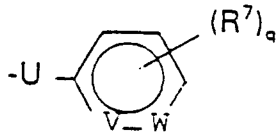

(B),

les $R^7$ désignant chacun, indépendamment l'un de l'autre, l'hydrogène, un halogène ou un alkyle ou un alcoxy en $C_1$-$C_4$ ou ($C_1$-$C_4$)halogénoalkyle ;
U le groupe $-CH_2-$, $>C=O$, -O- ou -S-,
V et W chacun CH ou N mais étant différents l'un de l'autre,
p étant un entier de 1 à 5,
q le nombre 1 ou 2, ou encore
$R^5$ est un groupe pyridyle avec un ou deux substituants, de formule générale C

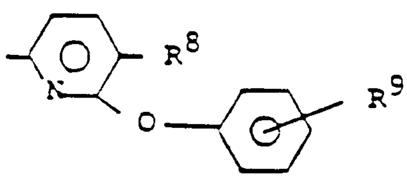

(C)

$R^8$ désignant un halogène ou l'hydrogène,
$R^9$ l'hydrogène, un halogène à l'exception de l'iode, ou bien un alkyle ou un alcoxy en $C_1$-$C_4$ ou un($C_1$-$C_4$)halogénoalkyle, ou encore
$R^5$ est un hétérocyclique de formule générale D

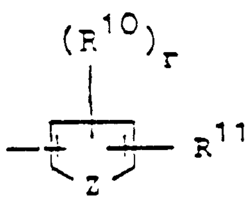

(D)

dans laquelle
Z désigne le soufre,
$R^{10}$ l'hydrogène, un halogène, un alkyle ou un alcoxy en $C_1$-$C_4$, un ($C_1$-$C_4$)halogénoalkyle.ou le groupe CN ou $NO_2$,
$R^{11}$ un groupe benzyle, propargyle, allyle ou phénoxy, éventuellement substités et
r le nombre 1 ou 2.,

procédé caractérisé en ce que :

a) pour des composés dans lesquels X est un atome d'oxygène on fait réagir un composé de

formule II ou III :

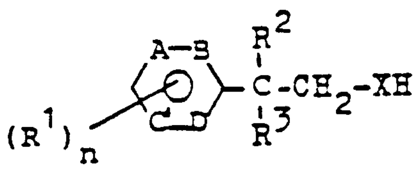 (II) 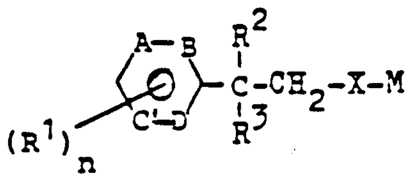 (III)

avec un agent d'alkylation de formule IV :

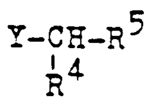 (IV),

Y désignant un radical nucléofuge qui s'élimine tel que par exemple un halogène ou un groupe sulfonate et M un équivalent de métal alcalin ou alcalino-terreux, notamment Li, Na, K ou Mg, éventuellement en présence d'une base,
ou bien
b) pour des composés dans lesquels X est aussi un atome d'oxygène on fait réagir un composé de formule V

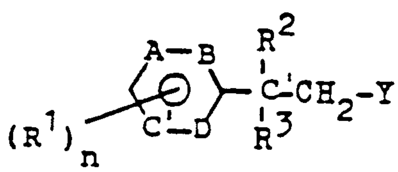 (V)

avec un composé acide XH du genre (VI)

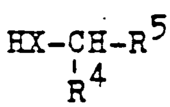 (VI)

en présence d'une base
ou avec un composé organo-métallique du genre VII

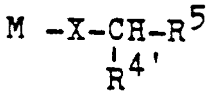 (VII),

$R^{4'}$ désignant l'hydrogène ou un (C1-C4)alkyle,
ou bien
c) pour des composés dans lesquels X est un groupe $CH_2$ on fait réagir un composé de formule générale VIII

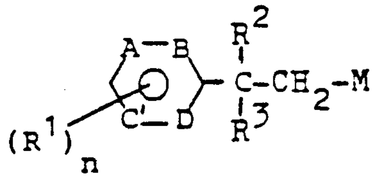

(VIII)

avec un composé du genre (IX)

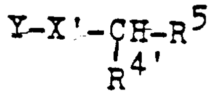

(IX),

X' étant le groupe $CH_2$, ou

d) un composé de formule X

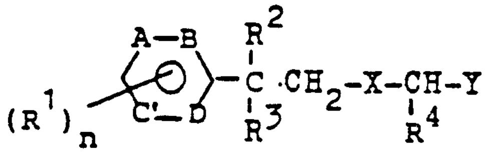

(X)

avec un réactif organo-métallique du genre XI

$M - R^5$ (XI)

ou bien

e) pour des composés dans lesquels $R^4$ est l'hydrogène et X le groupe $CH_2$ on condense un aldéhyde de formule XII

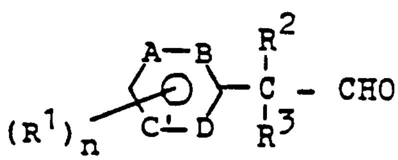

(XII)

avec une cétone de formule XIII

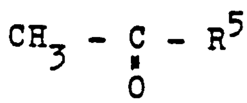

(XIII)

en un produit intermédiaire XIV

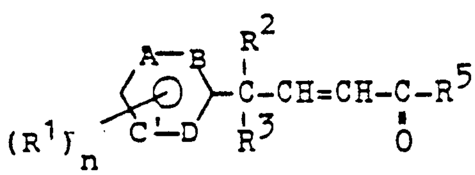

(XIV)

que l'on réduit ensuite de la manière habituelle,
ou encore
f) pour des composés dans lesquels X est le groupe $CH_2$ on condense un aldéhyde de formule XV

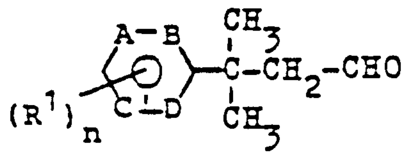

(XV)

avec un ylide de formule XVI ou un ester phosphonique de formule XVII

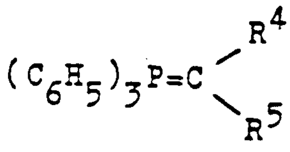

(XVI),

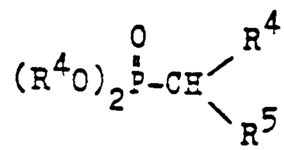

(XVII)

en un produit intermédiaire XVIII

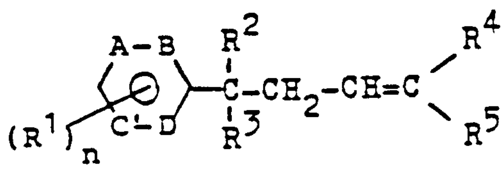

(XVIII)

que l'on transforme ensuite avec un agent réducteur en produit final (I) dans lequel X est le groupe $CH_2$.

**2.** Produits insecticides ou acaricides comprenant une proportion appropriée d'un composé de formule I de la revendication 1 avec des adjuvants de formulation courants.

**3.** L'emploi des composés de formule I de la revendication 1 pour lutter contre des insectes et acariens.

**4.** Procédé de lutte contre des insectes ou acariens, procédé caractérisé en ce que l'on traite ceux-ci ou leur environnement ou espace vital avec des composés de formule I de la revendication 1.